# EUROPÄISCHE PATENTSCHRIFT

(11) **EP 0 723 956 B1**
(45) Veröffentlichungstag und Bekanntmachung des Hinweises auf die Patenterteilung: **13.10.1999**
(21) Anmeldenummer: 96100956.0
(22) Anmeldetag: 24.01.1996
(51) Int. Cl.: C07C 311/29, C07C 381/10, C07D 295/22, C07D 295/12, C07C 311/64, C07C 317/46, C07C 323/25, C07C 279/22, C07D 233/84, C07D 233/42, C07C 335/32, C07D 235/28, C07D 213/70, C07D 215/36, C07D 233/54, C07D 233/02, C07D 213/42, C07C 317/28, A61K 31/155, A61K 31/44, A61K 31/18, A61K 31/415, A61K 31/47

(54) **Basisch-substituierte Benzoylguanidine, Verfahren zu ihrer Herstellung, ihre Verwendung als Medikament oder Diagnostikum sowie sie enthaltendes Medikament**
Basically substituted benzoylguanidines, process for their preparation, their use as medicaments or diagnostics as well as medicaments containing them
Benzoylyguanidines substitués par des groupes basiques, procédé pour leur préparation, leur utilisation comme médicaments ou agents diagnostiques ainsi que médicaments les contenant

(30) Priorität: 30.01.1995 DE 19502795; 14.02.1995 DE 19504805
(43) Veröffentlichungstag der Anmeldung: 31.07.1996
(73) Patentinhaber: HOECHST AKTIENGESELLSCHAFT, 65929 Frankfurt am Main (DE)
(72) Erfinder: Kleemann, Heinz-Werner, Dr., D-65474 Bischofshein (DE); Lang, Hans-Jochen, Dr., D-65719 Hofheim (DE); Schwark, Jan-Robert, Dr., D-65929 Frankfurt (DE); Weichert, Andreas, Dr., D-63329 Egelsbach (DE); Scholz, Wolfgang, Dr., D-65760 Eschborn (DE); Albus, Udo, Dr., D-61197 Florstadt (DE)

(56) Entgegenhaltungen:
- EP-A- 0 589 336
- EP-A- 0 602 523
- EP-A- 0 604 852
- WO-A-94/26709
- DE-A- 4 325 822

## Beschreibung

Die Erfindung betrifft Verbindungen der Formel I 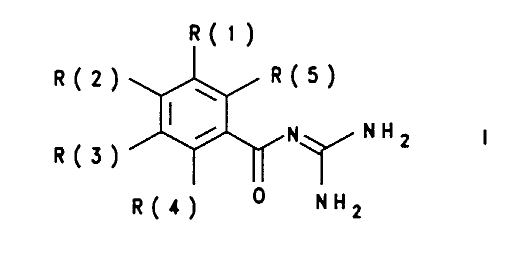 worin bedeuten:
einer der drei Substituenten R(1), R(2) und R(3)
R(6)-A-B-D-;
- R(6): ein basischer protonierbarer Rest, d.h. eine Aminogruppe -NR(7)R(8), eine Amidinogruppe R(7)R(8)N-C[=N-R(9)]- oder eine Guanidinogruppe 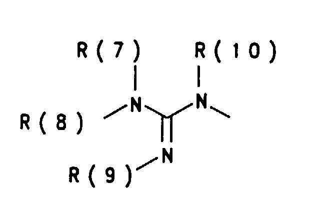
R(7), R(8), R(9) und R(10) unabhängig voneinander Wasserstoff oder Alkyl mit 1, 2, 3 oder 4 C-Atomen; oder
R(7) und R(8) gemeinsam CₐH₂ₐ;
a 4, 5, 6 oder 7;
wobei im Falle von a = 5, 6 oder 7 eine Methylengruppe der Gruppe CₐH₂ₐ durch eine Heteroatomgruppe O, SOₘ oder NR(11) ersetzt sein kann, oder
R(8) und R(9) oder R(9) und R(10) oder R(7) und R(10) eine Gruppe CₐH₂ₐ;
a 2, 3, 4 oder 5;
wobei im Falle von a = 3, 4 oder 5 eine Methylengruppe der Gruppe CₐH₂ₐ durch eine Heteroatomgruppe O, SOₘ oder NR(11) ersetzt sein kann;
m Null, 1 oder 2;
R(11) Wasserstoff oder Methyl; oder
R(6) ein basisches heteroaromatisches Ringsystem mit 1 - 9 C-Atomen;
A C_{b}H_{2b};
b 1, 2, 3, 4, 5, 6, 7, 8, 9 oder 10;
wobei in der Gruppe C_{b}H_{2b} ein oder zwei Methylengruppen durch eine der Gruppierungen ausgewählt aus der Gruppe bestehend aus -O-, -CO-, -CH[OR(20)]-, -SOₘ-, -NR(20)-, -NR(20)-CO-, -NR(20)-CO-NH-, -NR(20)-CO-NH-SO₂- 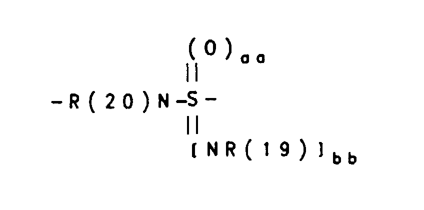 und -SOₐₐ[NR(19)]_{bb}- ersetzt sein können;
und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1; aa + bb = 2;
R(19) Wasserstoff oder Alkyl mit 1, 2, 3 oder 4 C-Atomen
R(20) Wasserstoff oder Methyl;
B einen Phenylen- oder Naphthylenrest, 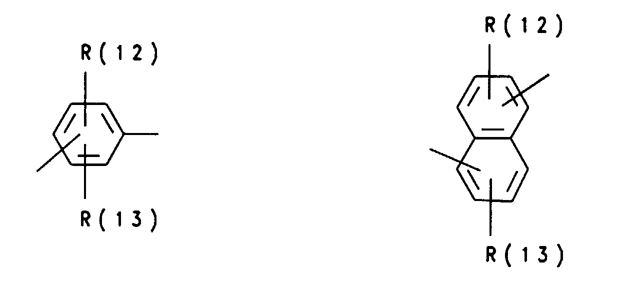
R(12) und R(13) unabhängig voneinander Wasserstoff, Methyl, F, Cl, Br, J, CF₃ oder -SO_{w}-R(14);
R(14) Methyl oder NR(15)R(16);
R(15) und R(16) unabhängig voneinander Wasserstoff oder Alkyl mit 1, 2, 3 oder 4 C-Atomen;
w Null, 1 oder 2;
D -C_{d}H_{2d}-X_{f}-;
d Null, 1, 2, 3 oder 4;
X -O-, -CO-, -CH[OR(21)]-, -SOₘ- oder -NR(21)-;
f 1;
R(21) Wasserstoff oder Methyl;
m Null, 1 oder 2; und die jeweils anderen Substituenten R(1) und R(2) und R(3) unabhängig voneinander Wasserstoff, F, Cl, Br, J, -CN, -(C₁-C₈)-Alkyl, -(C₂-C₈)-Alkenyl, -NR(35)R(36) oder R(17)-C_{g}H_{2g}-Zₕ-;
g Null, 1, 2, 3 oder 4;
h Null oder 1;
R(35) und R(36) unabhängig voneinander Wasserstoff oder Alkyl mit 1, 2, 3, 4, 5 oder 6 C-Atomen; oder
R(35) und R(36) gemeinsam 4 - 7 Methylengruppen, von denen eine CH₂-Gruppe durch Sauerstoff, -S-, -NH-, -NCH₃ oder -N-Benzyl ersetzt sein kann;
Z O-, -CO-, -SOᵥ-,-NR(18)-, -NR(18)-CO-, -NR(18)-CO-NH- oder -NR(18)-SO₂-;
R(18) Wasserstoff oder Methyl;
v Null, 1 oder 2;
R(17) Wasserstoff, Cycloalkyl mit 3, 5 oder 6 C-Atomen oder CₖF₂ₖ₊₁-; k 1, 2 oder 3,
oder
R(17) Pyrrol-1-yl, Pyrrol-2-yl oder Pyrrol-3-yl,
welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl und Methoxy; oder
- R(17): -(C₃-C₈)-Cydoalkyl oder Phenyl,
welches nicht substituiert oder substituiert ist mit 1 - 3 Substituenten ausgewählt aus der Gruppe bestehend aus F und Cl, -CF₃, Methyl, Hydroxy, Methoxy, -NR(37)R(38), CH₃SO₂- und H₂NO₂S-;
R(37) und R(38) Wasserstoff oder -CH₃;
- R(4) und R(5): unabhängig voneinander Wasserstoff, Alkyl mit 1, 2, 3 oder 4 C-Atomen, F, Cl, -OR(32), -NR(33)R(34) oder -CᵣF₂ᵣ₊₁;
R(32), R(33) und R(34) unabhängig voneinander Wasserstoff oder Alkyl mit 1, 2 oder 3 C-Atomen;
r 1, 2, 3 oder 4;
sowie deren pharmakologisch verträgliche Salze.

Bevorzugt sind Verbindungen der Formel I, worin bedeuten:
- R(1): Wasserstoff, F, Cl, -(C₁-C₄)-Alkyl, -(C₂-C₄)-Alkenyl, -NR(35)R(36) oder R(17)-C_{g}H_{2g}-Zₕ-;
R(35) und R(36) unabhängig voneinander Wasserstoff, Methyl oder Ethyl; oder
R(35) und R(36) gemeinsam 4 - 5 Methylengruppen, von denen eine CH₂-Gruppe durch Sauerstoff, -S-, -NH-, oder -NCH₃ ersetzt sein kann;
R(17) Wasserstoff, Cycloalkyl mit 5 oder 6 C-Atomen oder CₖF₂ₖ₊₁-;
k 1, 2 oder 3,
g Null, 1, 2, 3 oder 4;
h Null oder 1;
Z -O-, -CO-, -SOᵥ-,-NR(18)-, -NR(18)-CO-, -NR(18)-CO-NH- oder -NR(18)-SO₂-;
R(18) Wasserstoff oder Methyl;
v Null, 1 oder 2; oder, wenn g und h gleich null sind,
R(17) Pyrrol-1-yl, Pyrrol-2-yl oder Pyrrol-3-yl,
welches nicht substituiert oder substituiert ist mit 1 - 2 Substituenten ausgewählt aus der Gruppe bestehend aus F, Cl, (C₂-C₅)-Alkanoyl, (C₂-C₅)-Alkoxycarbonyl, Formyl, Carboxy, -CF₃, Methyl und Methoxy; oder
- R(17): -(C₃-C₈)-Cydoalkyl oder Phenyl,
welches nicht substituiert oder substituiert ist mit 1 - 2 Substituenten ausgewählt aus der Gruppe bestehend aus F und Cl, -CF₃, Methyl, Methoxy, -NR(37)R(38), CH₃SO₂- und H₂NO₂S-;
R(37) und R(38) unabhängig voneinander Wasserstoff oder -CH₃;
einer der Substituenten R(2) und R(3)
R(6)-A-B-D-;
- R(6): -NR(7)R(8), eine Amidinogruppe R(7)R(8)N-C[=N-R(9)]- oder eine Guanidinogruppe 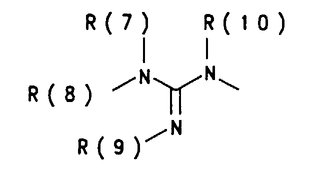
R(7), R(8), R(9) und R(10) unabhängig voneinander Wasserstoff oder Alkyl mit 1, 2, 3 oder 4 C-Atomen; oder
R(7) und R(8) gemeinsam CₐH₂ₐ;
a 4, 5, 6 oder 7;
wobei im Falle von a = 5, 6 oder 7 eine Methylengruppe der Gruppe CₐH₂ₐ durch eine Heteroatomgruppe O, SOₘ oder NR(11) ersetzt sein kann;
R(11) Wasserstoff oder Methyl; oder
R(8) und R(9) gemeinsam CₐH₂ₐ;
a 2, 3, 4 oder 5;
wobei im Falle von a = 3, 4 oder 5 eine Methylengruppe der Gruppe CₐH₂ₐ durch eine Heteroatomgruppe O, SOₘ oder NR(11) ersetzt sein kann;
m Null, 1 oder 2; oder
- R(6): Imidazolyl-, Pyridyl-, Chinolinyl oder Isochinolinyl;
- A: C_{b}H_{2b};
b 1, 2, 3, 4 oder 5,
wobei in der Gruppe C_{b}H_{2b} eine oder zwei Methylengruppen durch eine der Gruppierungen ausgewählt aus der Gruppe bestehend aus -O-, -CO-, -CH[OR(20)]-,-SOₘ-, NR(20), -NR(20)-CO-, -NR(20)-CO-NH-, -NR(20)-CO-NH-SO₂-, 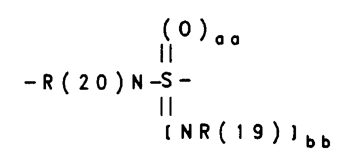 und -SOₐₐ[NR(19)]_{bb}- ersetzt sein können;
und wobei in der Gruppe C_{b}H_{2b} eine Methylengruppe durch -CH-R(99) ersetzt sein kann, wobei R(99) gemeinsam mit R(7) einen Pyrrolidin- oder Piperidinring bildet;
aa 1 oder 2;
bb 0 oder 1; aa + bb = 2;
R(19) Wasserstoff oder Alkyl mit 1, 2, 3 oder 4 C-Atomen
R(20) Wasserstoff oder Methyl;
- B: einen Phenylen- oder Naphthylenrest, 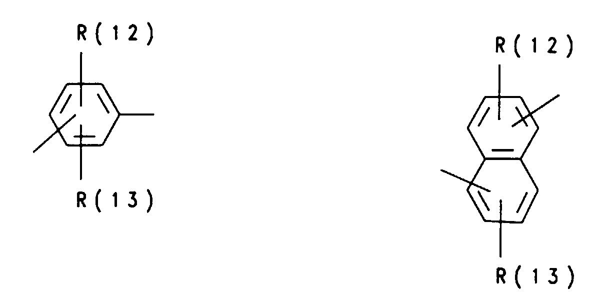
- R(12) und R(13): unabhängig voneinander Wasserstoff, Methyl, F, Cl, CF₃ oder -SO₂-R(14);
- R(14): Methyl oder NR(15)R(16);
R(15) und R(16) unabhängig voneinander Wasserstoff oder Alkyl mit 1, 2, 3 oder 4 C-Atomen ;
D -C_{d}H_{2d}-X_{f};
d Null, 1, 2, 3 oder 4;
X -O-, -CO-, -CH[OR(21)]-, -SOₘ- oder -NR(21)-;
f 1;
R(21) Wasserstoff oder Methyl;
m Null, 1 oder 2;
und der jeweils andere Rest R(2) und R(3)
Wasserstoff, Alkyl mit 1, 2, 3 oder 4 C-Atomen, F, Cl oder CF₃; R(4) und R(5)
unabhängig voneinander Wasserstoff, Alkyl mit 1, 2 oder 3 C-Atomen, F, Cl, oder -CF₃. sowie deren pharmakologisch verträgliche Salze.

Besonders bevorzugt sind Verbindungen der Formel I, worin bedeuten:
- R(1): Wasserstoff, F, Cl, Alkyl mit 1, 2, 3 oder 4 C-Atomen, -NR(35)R(36) oder R(17)-C_{g}H_{2g}-Zₕ-;
g Null, 1, 2, 3 oder 4;
h Null oder 1;
Z -O-, -CO-, -SOᵥ- ,-NR(18)-, -NR(18)-CO-, -NR(18)-CO-NH-, oder -NR(18)-SO₂-;
R(18) Wasserstoff oder Methyl;
v Null, 1 oder 2;
R(17) Wasserstoff, Cycloalkyl mit 5 oder 6 C-Atomen oder CF₃-; oder, wenn g und h gleich null sind,
R(17) Pyrrol-1-yl,
welches nicht substituiert oder substituiert ist mit 1 - 2 Substituenten ausgewählt aus der Gruppe bestehend aus F, Cl, (C₂-C₅)-Alkanoyl, (C₂-C₅)-Alkoxycarbonyl, -CF₃ und Methyl; oder
- R(17): -(C₅-C₆)-Cycloalkyl oder Phenyl,
welches nicht substituiert oder substituiert ist mit einem Substituenten ausgewählt aus der Gruppe bestehend aus F und Cl, -CF₃, Methyl, CH₃SO₂- und H₂NO₂S-;
- R(35) und R(36): unabhängig voneinander Wasserstoff, Methyl oder Ethyl;
oder
- R(35) und R(36): gemeinsam 4 - 5 Methylengruppen, von denen eine CH₂-Gruppe durch Sauerstoff, -S-, -NH-, oder -NCH₃ ersetzt sein kann;
einer der Substituenten R(2) und R(3)
R(6)-A-B-D-;
- R(6) -NR(7)R(8),: eine Amidinogruppe R(7)R(8)N-C[=N-R(9)]- oder eine Guanidinogruppe 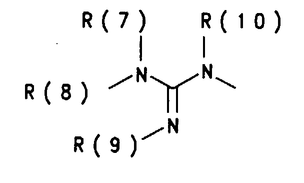
R(7) Wasserstoff oder Alkyl mit 1, 2, 3 oder 4 C-Atomen;
R(8), R(9) und R(10) unabhängig voneinander Wasserstoff, Methyl oder Ethyl; oder
R(7) und R(8) gemeinsam CₐH₂ₐ;
a 4 oder 5;
wobei im Falle von a = 5 eine Methylengruppe der Gruppe CₐH₂ₐ durch NR(11) ersetzt sein kann, R(11) Wasserstoff oder Methyl;
oder
- R(6): Imidazolyl- oder Pyridyl;
- A: C_{b}H_{2b};
b 1, 2, 3, 4 oder 5,
wobei in der Gruppe C_{b}H_{2b} ein oder zwei Methylengruppen durch eine der Gruppierungen ausgewählt aus der Gruppe bestehend aus -CO-, -CH[OR(20)]-, -NR(20)-CO-, 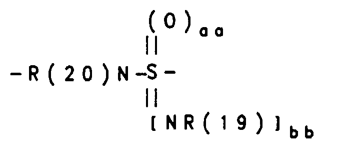 -SOₐₐ[NR(19)]_{bb} und -SO₂-ersetzt sein können;
und wobei in der Gruppe C_{2b}H_{2b} eine Methylengruppe durch -CH-R(99) ersetzt sein kann, wobei R(99) gemeinsam mit R(7) einen Pyrrolidin- oder Piperidinring bildet;
aa 1 oder 2;
bb 0 oder 1; aa + bb = 2;
R(19) Wasserstoff oder Alkyl mit 1, 2, 3 oder 4 C-Atomen
R(20) Wasserstoff oder Methyl; oder, wenn b gleich 2, 3, 4 oder 5 bedeutet
ein C-Atom in C_{b}H_{2b} durch eine Gruppierung -O-, -S-,-NR(20)-, -NR(20)-CO- oder -NR(20)-CO-NH- ersetzt sein kann;
- B: einen Phenylenrest, 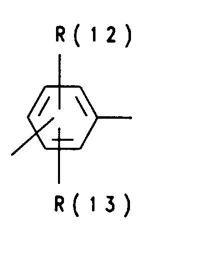
R(12) und R(13) unabhängig voneinander Wasserstoff, Methyl, F, Cl, CF₃ oder -SO₂R(14);
R(14) Methyl oder NH₂;
- D: -CH₂-, -O-, -CO-, -SOₘ- oder -NR(21)-;
m Null oder 2;
R(21) Wasserstoff oder Methyl;
und der jeweils andere Rest R(2) und R(3)
Wasserstoff;
- R(4) und R(5): unabhängig voneinander Wasserstoff, Alkyl mit 1, 2 oder 3 C-Atomen, F, Cl, oder -CF₃;
sowie deren pharmazeutisch verträgliche Salze.

Ganz besonders bevorzugt sind Verbindungen der Formel I, worin bedeuten
- R(1): Wasserstoff, F, Cl, Alkyl mit 1, 2, 3 oder 4 C-Atomen, -NR(35)R(36) oder R(17)-C_{g}H_{2g}-Zₕ-;
g Null oder 1;
h Null oder 1;
Z -O-, -CO-, -NR(18)-CO-, -NR(18)-CO-NH-, oder -NR(18)-SO₂-; R(18) Wasserstoff oder Methyl; oder, wenn g gleich 1;
Z -SO₂-;
R(17) Wasserstoff oder CF₃-;
R(35) und R(36) unabhängig voneinander Wasserstoff, Methyl oder Ethyl; oder
R(35) und R(36) gemeinsam 4 - 5 Methylengruppen, von denen eine CH₂-Gruppe durch Sauerstoff, -S-, -NH-, oder -NCH₃ ersetzt sein kann;
einer der Substituenten R(2) und R(3)
R(6)-A-B-O-;
- R(6): -NR(7)R(8) oder eine Guanidinogruppe 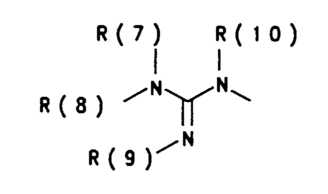
R(7) Wasserstoff oder Alkyl mit 1, 2, 3 oder 4 C-Atomen;
R(8), R(9) und R(10) unabhängig voneinander Wasserstoff, Methyl oder Ethyl; oder
R(7) und R(8) gemeinsam CₐH₂ₐ;
a 4 oder 5; wobei im Falle von a = 5 eine Methylengruppe der Gruppe CₐH₂ₐ durch -NH- oder -NCH₃- ersetzt sein kann, oder
- R(6): Imidazolyl-;
- A: C_{b}H_{2b};
b 1, 2, 3 oder 4;
wobei in der Gruppe C_{b}H_{2b} eine oder zwei Methylengruppen durch eine der Gruppierungen ausgewählt aus der Gruppe bestehend aus -CO-, 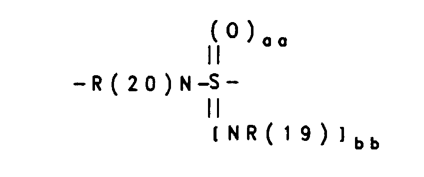 -SOₐₐ[NR(19)]_{bb}- und -SO₂-ersetzt sein können,
und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bilden kann;
oder, wenn b gleich 2, 3 oder 4 bedeutet,
eine Methylengruppe in der Gruppe C_{b}H_{2b} durch eine Gruppierung -O-, -S- ersetzt sein kann;
aa 1 oder 2;
bb 0 oder 1; aa + bb = 2;
R(19) Wasserstoff oder Alkyl mit 1, 2, 3 oder 4 C-Atomen
R(20) Wasserstoff oder Methyl;
- B: einen Phenylenrest, 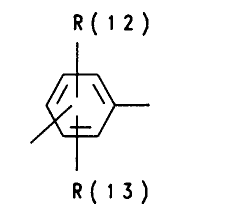
R(12) und R(13) Wasserstoff;
und der jeweils andere Rest R(2) und R(3) Wasserstoff;
- R(4) und R(5): Wasserstoff;
sowie deren pharmakologisch verträgliche Salze.

Insbesondere bevorzugt ist eine Verbindung ausgewählt aus der Gruppe bestehend aus
4-[4-N-(Dimethylaminoethyl)-methylsulfamoyl]phenoxy-3-trifluormethylbenzoylguanidin, Dihydrochlorid;
4-[4-(4-Methylpiperazinosulfonyl)phenoxy]-3-trifluormethyl-benzoylguanidin, Dihydrochlorid;
4-[4-(2-Pyrolidinethylaminosulfonyl)phenoxy]-3-trifluormethyl-benzoylguanidin, Dimaleinat;
4-[4-(2-Piperidinethylaminosulfonyl)phenoxy]-3-trifluormethyl-benzoylguanidin, Dimaleinat;
4-[4-(N-Dimethylamino-n-propyl)sulfamoyl]phenoxy-3-trifluormethyl-benzoylguanidin;
4-[4-(N-Dimethylaminoethyl)sulfamoyl]phenoxy-3-trifluormethyl-benzoylguanidin;
4-(4-lmidamidosulfonyl)phenoxy-3-trifluormethyl-benzoylguanidin;
3-Trifluormethyl-4-(4-N-methylimidamidosulfonyl)phenoxy-benzoylguanidin;
3-Methyl-4-(4-(1-methylpiperazin-4-ylsulfonyl)phenoxy)-benzoylguanidin;
4-(4-Guanidinosulfonyl)phenoxy-3-trifluormethyl-benzoylguanidin;
4-[4-(2-lmidazolylthio-acetyl)phenoxy]-3-methylsulfonyl-benzoyl-guanidin Dihydrochlorid;
4-[4-(N, N'-Dimethyl-S-isothiuronyl-acetyl)phenoxy]-3-methylsulfonyl-benzoylguanidin Dihydrochlorid;
4-[4-(2-Benzimidazolylthio-acetyl)phenoxy]-3-methylsulfonyl-benzoylguanidin Dihydrochlorid;
4-[4-(2-N-Imidazolyl-1-hydroxyethyl)phenoxy]-3-methylsulfonyl-benzoylguanidin Dihydrochlorid;
4-[4-(N,N-Dimethylglycylamino)phenoxy]-3-methylsulfonylbenzoyl-guanidin Dihydrochlorid;
4-[4-(N,N-Diethylaminoethyl)aminosulfonylphenoxy]-3-methylsulfonylbenzoylguanidin Dihydrochlorid;
4-[4-(4-lmidazolylethyl)aminosulfonyl-phenoxy]-3-methylsulfonyl-benzoyl-guanidin Dihydrochlorid;
4-[4-(3-N-Imidazolyl-1-propyl)aminosulfonyl-phenoxy]-3-methylsulfonylbenzoylguanidin Dihydrochlorid;
4-[4-(1-Methyl-2-pyrrolidinylethyl)aminosulfonyl-phenoxy]-3-methylsulfönylbenzoyl guanidin Dihydrochlorid;
4-[4-(N-Piperidinoethyl)aminosulfonyl-phenoxy]-3-methylsulfonyl-benzoyl-guanidin Dihydrochlorid;
4-[4-(2-Dimethylaminoethyl)sulfonylmethyl-phenoxy]-3-methylsulfonylbenzoylguanidin Dihydrochlorid;
4-[4-(2-Dimethylaminoethyl)sulfonylmethyl-phenoxy]-3-trifluormethylbenzoylguanidin Dihydrochlorid.

Die bezeichneten Alkylreste können sowohl geradkettig wie verzweigt vorliegen.

Unter einem basischen heteroaromatischen Ringsystem mit 1 - 9 C-Atomen werden insbesondere Reste verstanden, die sich von Cyclopentyl, Phenyl oder Naphthyl ableiten, in welchen eine oder mehrere CH-Gruppen durch N ersetzt sind. Als Heteroaryl gelten insbesondere Imidazolyl, Pyrazolyl, Oxazolyl, Isoxazolyl, Thiazolyl, Isothiazolyl, Pyridyl, Chinolyl, Isochinolyl.
Halogen bedeutet F, Cl, Br oder J.

Enthalten die Verbindungen der Formel I asymmetrische Zentren, so beschreibt Formel I sowohl die einzelnen optischen Antipoden als auch deren mögliche Enantiomerengemische.

Die Erfindung betrifft weiterhin ein Verfahren zur Herstellung einer Verbindung I, dadurch gekennzeichnet, daß man eine Verbindung der Formel II 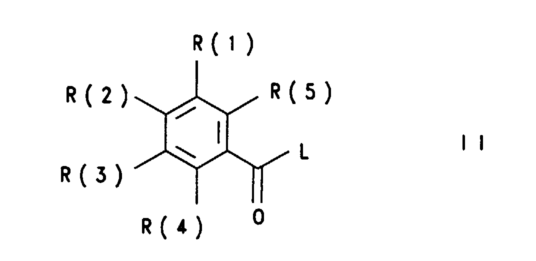 worin R(1) bis R(5) die angegebene Bedeutung besitzen und L für eine leicht nucleophil substituierbare leaving group steht, mit Guanidin umsetzt,
und daß man gegebenenfalls in ein pharmakologisch verträgliches Salz überführt.

Die aktivierten Säurederivate der Formel II, worin L eine Alkoxy, vorzugsweise eine Methoxygruppe, eine Phenoxygruppe, Phenylthio, Methylthio, 3-Pyridyloxy-, 2-Pyridylthiogruppe, einen Stickstoffheterocyclus, vorzugsweise 1-Imidazolyl, bedeutet, erhält man vorteilhaft in bekannter Weise aus den zugrundeliegenden Carbonsäurechloriden (Formel II, L = Cl), die man ihrerseits wiederum in bekannter Weise aus den zugrundeliegenden Carbonsäuren (Formel II, L = OH), beispielsweise mit Thionylchlorid, herstellen kann.

Neben den Carbonsäurechloriden der Formel II (L = Cl) lassen sich auch weitere aktivierte Säurederivate der Formel II in bekannter Weise direkt aus den zugrundeliegenden Benzoesäurederivaten (Formel II, L = OH) herstellen, beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 - 367 (1962)], die gemischten Anhydride II mit CICOOC₂H₅ oder Tosylchlorid in Gegenwart von Triethylamin in einem inerten Lösungsmittel; die Aktivierung von Benzoesäuren ist auch mit Dicyclohexylcarbodiimid (DCC) oder mit O[(Cyano(ethoxycarbonyl)methylen)amino]-1,1,3,3-tetramethyluroniumtetrafluorborat ("TOTU") möglich [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II ist unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in bekannter Weise in einem protischen oder aprotischen polaren, aber inerten organischen Lösungsmittel. Dabei haben sich bei der Umsetzung der Benzoesäuremethylester (II, L = OMe) mit Guanidin Methanol, Isopropanol oder THF bei Temperaturen von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln, wie THF, Dimethoxyethan, Dioxan oder Isopropanol gearbeitet. Aber auch Wasser kann unter Gebrauch einer Base, beispielsweise NaOH, als Lösungsmittel bei der Umsetzung von II und Guanidin verwendet werden.

Wenn L = Cl bedeutet, arbeitet man vorteilhaft unter Zusatz eines Säurefängers, z. B. in Form von überschüssigen Guanidin zur Abbindung der Halogenwasserstoffsäure.

Die zugrundeliegenden Benzoesäurederivate sind teilweise bekannt. Sie werden nach literaturbekannten Methoden dargestellt, indem man beispielsweise den fertigen Rest R(6)-A-B-C_{d}H_{2d}-X_{f}- oder eine Vorstufe davon durch Austausch von Halogen in ein Benzoesäurederivat der Formel III überführt 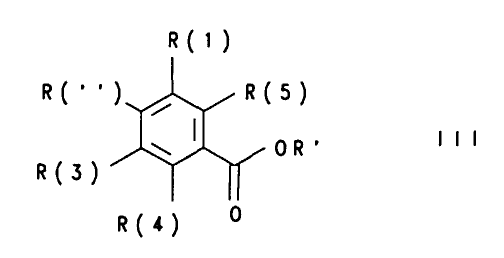 worin der Substituent R' die Bedeutung eines niederen Alkylrestes, wie zum Beispiel Methyl oder Ethyl hat und R" = Halogen bedeutet. Die Reaktionen sind in der Literatur beschrieben, beispielsweise als nucleophile Substitutionsreaktion, als radikalisch verlaufende Ullmann-Reaktion oder palladium-katalysierte Reaktionen.

Die Einführung der im Phenylteil mit Schwefel-, Sauerstoff- oder Stickstoffnucleophilen substituierten Benzolsulfonamid-Derivate gelingt durch literaturbekannte Methoden der nucleophilen Substitution am Aromaten. Als Abgangsgruppe am Benzoesäurederivat haben sich bei dieser Substitution Halogenide und Trifluormethansulfonate bewährt. Man arbeitet vorteilhaft in einem dipolar aprotischen Lösungsmittel, wie DMF oder TMU, bei einer Temperatur von 0°C bis zum Siedepunkt des Lösungsmittels, bevorzugt von 80°C bis zum Siedepunkt des Lösungsmittels. Als Säurefänger dient vorteilhaft ein Alkali- oder Erdalkalisalz mit einem Anion hoher Basizität und geringer Nucleophilie, zum Beispiel K₂CO₃ oder CsCO₃.

Die als Vorstufen verwendeten Verbindungen R(6)-A-B-C_{d}H_{2d}-X_{f}- sind größtenteils bekannt und z.T. auch als Reagenzien käuflich. Ihre Herstellung erfolgt nach literaturbekannten Methoden, die dem Fachmann bekannt sind.

Die Einführung einiger Substituenten in 4- und 5-Stellung gelingt durch literaturbekannte Methoden des Palladium-vermittelten cross-couplings von Arylhalogeniden mit beispielsweise Organostannanen, Organoboronsäuren oder Organoboranen oder Organokupfer- bzw. -zink-verbindungen.

Benzoylguanidine I sind im allgemeinen schwache Basen und können Säure unter Bildung von Salzen binden. Als Säureadditionssalze kommen Salze aller pharmakologisch verträglichen Säuren infrage, beispielsweise Halogenide, insbesondere Hydrochloride, Lactate, Sulfate, Citrate, Tartrate, Ascorbate, Acetate, Phosphate, Methylsulfonate, p-Toluolsulfonate.

Die Verbindungen I sind substituierte Acylguanidine. Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 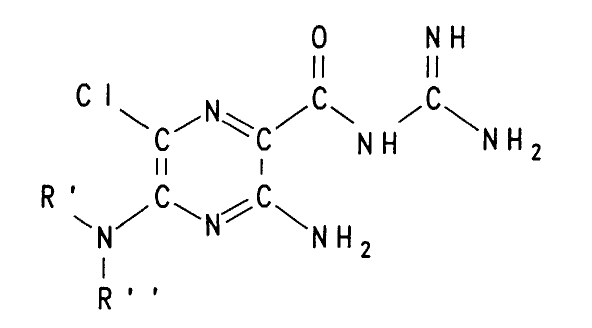
Amilorid: R',R"= H
Dimethylamilorid: R',R" = CH₃
Ethylisopropylamilorid: R' = C₂H₅, R" = CH(CH₃)₂

Darüber hinaus sind Untersuchungen bekannt geworden, die auf antiarrhythmische Eigenschaften von Amilorid hinweisen (Circulation 79, 1257 bis 1263 (1989)). Einer breiten Anwendung als Antiarrhythmikum steht jedoch entgegen, da dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung nur ein Wasserstoff-Atom tragen, und in denen keiner der Substituenten die Bedeutung von R(5)-A-B-D-hat. In der Europäischen Offenlegungsschrift 0 556 674 A (HOE 92/F 034) werden 3,5-substituierte Benzoylguanidine vorgeschlagen, in welchen aber der Substituent R(2) nicht die nach der vorliegenden Erfindung beanspruchte Bedeutung von R(5)-A-B-D- hat.

In der US-Patentschrift 3 780 027 werden Acylguanidine beansprucht, die strukturell den Verbindungen der Formel I ähnlich sind und sich von im Handel befindlichen Schleifendiuretika, wie Bumetanid, ableiten. Entsprechend wird für diese Verbindungen eine starke salidiuretische Wirksamkeit berichtet.

Aus der WO 94 26 709 sind Verbindungen der Formel 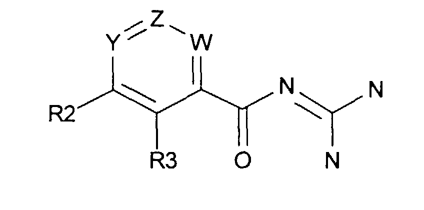 bekannt, aus welcher Vielfalt der angegebenen Möglichkeiten sich auch Verbindungen konstruieren lassen, welche den erfindungsgemäßen ähnlich sind, nämlich mit:
- Y: C-R(1);
- R(1): aryl, phenyl(lower alkoxy);
- Z: C-R(4)
- W: C-R(12);
oder mit einer unsubstituierten Phenoxy-Gruppe als R2.

Aber selbst bei dieser sehr gewollten Auswahl aus dem Stand der Technik resultierten stets nur Biphenyl-, Phenoxy- oder Benzyloxy-Verbindungen, welche von den Ansprüchen generell ausgeschlossen sind, nach denen sich immer ein Abstandhalter [-O-, -CO-, -CH[OR(21)]- oder -NR(21)-, aber keine Benzyloxy-Gruppe] zwischen den beiden Phenylkernen befindet.

In keinem Falle ist nach WO 94 26 709 eine stark basische Substitution vorgesehen oder nahegelegt, wie sie erfindungsgemäß durch den Substituenten R(6) stets vorhanden ist.

Es war überraschend, daß die erfindungsgemäßen Verbindungen keine unerwünschten und nachteiligen salidiuretischen, jedoch sehr gute antiarrhythmische Eigenschaften gegen solche Arrhythmien aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüber hinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüber hinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen der Formel I für die präventive Therapie zur Verhinderung der Genese des Bluthochdrucks, beispielweise der essentiellen Hypertonie, geeignet.

Neben einer sehr starken inhibitorischen Wirkung auf den Na⁺/H⁺-Austauscher weisen die Verbindungen nach der Erfindung gegenüber den bekannten Verbindungen eine signifikant verbesserte Wasserlöslichkeit auf. Daher sind sie wesentlich besser für i.V.-Applikationen geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

### Liste der Abkürzungen:

- AIBN: α,α-Azo-bis-isobutyronitril
- Bn: Benzyl
- Brine: gesättigte wäßrige NaCl-Lösung
- CH₂Cl₂: Dichlormethan
- DCI: Desorption-Chemical Ionisation
- DIP: Diisopropylether
- DMA: Dimethylacetamid
- DME: Dimethoxyethan
- DMF: N,N-Dimethylformamid
- EE: Ethylacetat (EtOAc)
- El: electron impact
- eq: Äquivalent
- ES: Elektrospray-lonisation
- Et: Ethyl
- FAB: Fast Atom Bombardment
- HEP: n-Heptan
- HOAc: Essigsäure
- Me: Methyl
- MeOH: Methanol
- mp: Schmelzpunkt
- MTB: Methyltertiärbutylether
- NBS: N-Bromsuccinimid
- NMP: N-Methylpyrrolidon
- RT: Raumtemperatur
- THF: Tetrahydrofuran
- TMU: N,N,N',N'-Tetramethylharnstoff
- ZNS: Zentralnervensystem

### Experimenteller Teil

### Allgemeine Vorschrift zur Herstellung von Benzoyl-guanidinen (I)

### Variante A: aus Benzoesäuren (II, L = OH)

0,01 M des Benzoesäurederivates der Formel II löst bzw. suspendiert man in 60 ml wasserfreiem THF und versetzt sodann mit 1,78 g (0,011 M) Carbonyldiimidazol. Nach dem Rühren über 2 Stunden bei RT werden 2,95 g (0,05 M) Guanidin in die Reaktionslösung eingetragen. Nach dem Rühren über Nacht destilliert man das THF unter vermindertem Druck (Rotationsverdampfer) ab, versetzt mit Wasser, stellt mit 2 N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

### Allgemeine Vorschrift zur Herstellung von Benzoyl-guanidinen (I)

### Variante B: aus Benzoesäure-alkylestern (II, L = O-Alkyl)

5 mmol des Benzoesäure-alkylesters der Formel II sowie 25 mmol Guanidin (freie Base) werden in 15 ml Isopropanol gelöst oder in 15 ml THF suspendiert und bis zum vollständigen Umsatz (Dünnschichtkontrolle) unter Rückfluß gekocht (typische Reaktionszeit 2 bis 5 h). Das Lösungsmittel wird unter vermindertem Druck (Rotationsverdampfer) abdestilliert, in 300 ml EE aufgenommen und 3 x mit je 50 ml NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1: 4-(4-Aminosulfonyl)phenoxy-3-trifluormethyl-benzoylguanidin

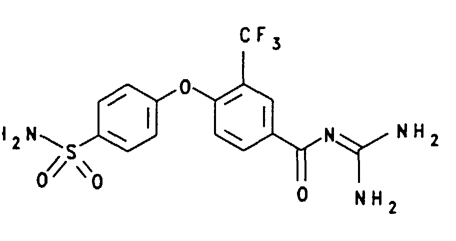

a) 4-Fluor-3-trifluormethyl-benzoesäuremethylester
   5 g 4-Fluor-3-trifluormethyl-benzoesäure und 9 ml SOCl₂ werden in 50 ml MeOH 8 h bei 60°C gerührt. Anschließend werden die flüchtigen Bestandteile im Vakuum entfernt und man erhält 5,1 g eines farblosen Öls, das ohne Reinigung weiter eingesetzt wird.
   Rf (EE/MeOH 10:1) = 0.74 MS (DCI) 223 (M+H)⁺
b) 4-(4-Aminosulfonyl)phenoxy-3-trifluormethyl-benzoesäuremethylester
   890 mg Fluorid a), 690 mg 4-Hydroxybenzolsulfonamid und 1.1 g K₂CO₃ werden in 5 ml DMF 2 h bei 120°C gerührt. Man läßt auf RT abkühlen, gibt 100 ml Brine hinzu und extrahiert 3 x mit je 50 ml EE. Über Na₂SO₄ wird getrocknet und das Solvens im Vakuum entfernt. Man erhält 1.2 g eines farblosen Öls.
   Rf (MTB) = 0.45 MS (DCI) 376 (M+H)⁺
c) 4-(4-Aminosulfonyl)phenoxy-3-trifluormethyl-benzoylguanidin
   550 mg Methylester 1 b) werden nach Variante B guanyliert. Man erhält 170 mg eines amorphen Pulvers.
   Rf (EE/MeOH 10:1) = 0.50 MS (ES) 403 (M+H)⁺ Wird in das Hydrochlorid überführt.
   mp > 270 °C

### Beispiel 2: 4-[4-(N-t-Butylimido-N'-t-butyl)sulfamoyl]-3-trifluormethyl-benzoylguanidin, Dihydrochlorid

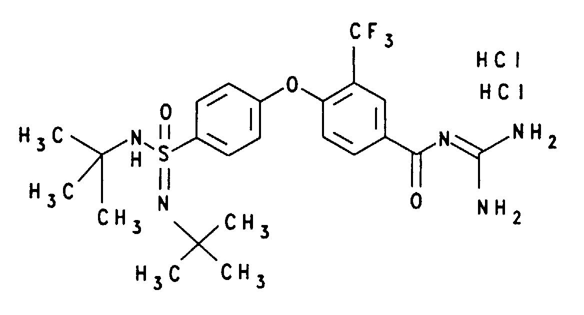

a) 4-Fluor-benzolsulfonsäure-N-N'-bis-t-butyl-imidamid
   900 ml t-Butylamin werden bei -30°C mit 10.3 ml Brom versetzt. Man läßt auf -5°C erwärmen und addiert 6.6 ml 4-Fluorthiophenol. Das Gemisch wird auf RT erwärmt und 4 h bei dieser Temperatur nachgerührt. Anschließend wird auf 600 g Eis gegossen, 500 ml EE zugegeben und 3 x mit je 100 ml gesättigter wäßriger Na₂SO₃-Lösung gewaschen. Die organische Phase wird nun im Vakuum eingeengt, erneut mit 500 ml EE aufgenommen und 3 x mit je 200 ml 0.6 M wäßriger KH₂PO₄-Lösung gewaschen. Dann wird die organische Phase 1 h lang mit 100 ml 2 N wäßriger HCI-Lösung gerührt und anschließend die EE-Phase abgetrennt. Die wäßrige Phase wird mit Na₂CO₃ auf pH=9 eingestellt und 3 x mit je 200 ml EE extrahiert. Über Na₂SO₄ wird getrocknet und das Lösungsmittel im Vakuum entfernt. Man erhält 6.4 g eines farblosen Öls.
   R_{f} (DIP) = 0.46 MS (DCI): 287 (M+H)⁺
b) 4-Hyd roxy-benzolsulfonsäure-N-N'-bis-t-butyl-imidamid
   2.9 g 4-Fluor-benzolsulfonsäure-N-N'-bis-t-butyl-imidamid und 3.4 g CsOH (Monohydrat) werden in 25 ml TMU 8 h bei 160-170°C gerührt. Man läßt anschließend auf RT abkühlen, versetzt mit 100 ml Wasser und 50 ml gesättigter wäßriger NaHCO₃-Lösung und extrahiert 3 x mit je 100 ml EE. Über Na₂SO₄ wird getrocknet und das Solvens im Vakuum entfernt. Chromatographie an Kieselgel mit EE/HEP 1:1 liefert 700 mg eines farblosen Öls.
   R_{f} (MTB/DIP 1.1) = 0.27 MS (El): 285 (M + H)⁺
c) 4-[4-(N-t-Butylimido-N'-t-butyl)sulfamoyl]phenoxy-3-trifluormethylbenzoesäuremethylester
   600 mg 4-Hydroxy-benzolsulfonsäure-N-N'-bis-t-butyl-imidamid, 468 mg 4-Fluor-3-trifluormethyl-benzoesäuremethylester und 2.1 g Cs₂CO₃ werden in 10 ml TMU 1.5 h bei 160°C gerührt. Man läßt auf RT abkühlen, gibt 100 ml einer gesättigten wäßrigen NaHCO₃-Lösung zu und extrahiert 3 x mit je 100 ml EE. Über Na₂SO₄ wird getrocknet und das Solvens im Vakuum entfernt. Chromatographie an Kieselgel mit DIP liefert 400 mg eines farblosen Öls.
   R_{f} (DIP) = 0.28 MS (ES) : 487 (M + H)⁺
d) 4-[4-(N-t-Butylimido-N'-t-butyl)sulfamoyl]phenoxy-3-trifluormethyl-benzoylguanidin 300 mg 4-[4-(N-t-Butylimido-N'-t-butyl)sulfamoyl]-3-trifluormethylbenzoesäuremethylester und 182 mg Guanidin werden in 10 ml Isopropanol nach der allgemeinen Vorschrift zur Herstellung von Benzoyl-guanidinen, Variante B umgesetzt. Man erhält 120 mg eines farblosen Öls.
   R_{f} (EE) = 0.24 MS (FAB) : 587 (M + H)⁺
   mp (Dihydrochlorid) = 165 - 168°C.

### Beispiel 3: 4-[4-N-(Dimethylaminoethyl)-methylsulfamoyl]phenoxy-3-trifluormethylbenzoylguanidin, Dihydrochlorid

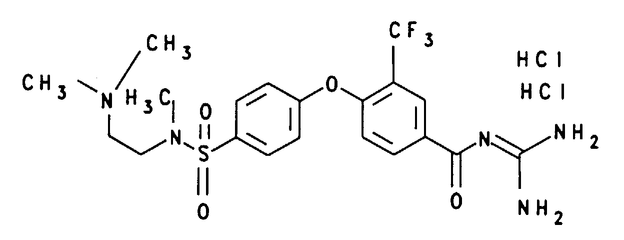

a) 4-Phenoxy-3-trifluormethyl-benzoesäuremethylester
   15 g 4-Chlor-3-trifluormethyl-benzoesäuremethylester, 5.9 g Phenol und 17.4 g K₂CO₃ werden in 100 ml DMF 14 h bei 110°C gerührt. Man läßt auf RT abkühlen, verdünnt mit 1 IEE, wäscht 2 x mit je 200 ml Wasser, 2 x mit je 200 ml 0.1 N wäßriger NaOH-Lösung und 2 x mit je 300 ml gesättigter wäßriger NaCl-Lösung. Über Na₂SO₄ wird getrocknet und das Lösungsmittel im Vakuum entfernt. Chromatographie an Kieselgel mit EE/HEP 1:8 liefert 11 g eines farblosen Öls.
   R_{f} (EE/HEP 1:8) = 0.24 MS (DCI): 297 (M + H)⁺
b) 4-Phenoxy-3-trifluormethyl-benzoesäure
   11 g 4-Phenoxy-3-trifluormethyl-benzoesäuremethylester werden in 200 ml MeOH gelöst und mit 41 ml 1 N wäßriger NaOH-Lösung versetzt und 24 h bei RT gerührt. Anschließend wird das MeOH im Vakuum entfernt, mit 1 l Wasser verdünnt, mit wäßriger HCI-Lösung auf pH = 2 gestellt und der Niederschlag abfiltriert. 48 h wird an der Luft getrocknet und man erhält 9.2 g eines amorphen Feststoffs.
   R_{f} (EE) = 0.10 MS (DCI): 283 (M + H)⁺
c) 4-(4-Chlorsulfonyl)phenoxy-3-trifluormethyl-benzoesäure
   1 g 4-Phenoxy-3-trifluormethyl-benzoesäure wird in 15 ml CHCl₃ gelöst und 710 µl Chlorsulfonsäure zugetropft. 3 h wird bei RT gerührt und anschließend das Lösungsmittel im Vakuum entfernt. Dann wird 50 g Eis und 50 ml Wasser zugegeben, 10 Minuten gerührt und der Niederschlag abfiltriert. Man erhält 0.96 g eines amorphen Feststoffs.
   R_{f} (DIP 2% HOAc) = 0.38 MS (El) : 381 (M + H)⁺
d) 4-[4-N-(Dimethylaminoethyl)-methylsulfamoyl]phenoxy-3-trifluormethylbenzoesäure
   475 mg 4-(4-Chlorsulfonyl)phenoxy-3-trifluormethyl-benzoesäure werden in 10 ml Aceton gelöst und mit 160 µl Trimethylethylendiamin und 350 µl Triethylamin versetzt. Das Gemisch wird 2 h bei RT gerührt, anschließend mit 100 ml Wasser verdünnt und das Aceton im Vakuum entfernt. Mit 0.1 N wäßriger HCI-Lösung wird auf pH=6-7 eingestellt und 6 x mit je 100 ml EE extrahiert. Über Na₂SO₄ wird getrocknet und das Lösungsmittel im Vakuum entfernt. Man erhält 330 mg eines amorphen Feststoffs.
   R_{f} (CH₂Cl₂/MeOH/HOAc/H₂O 8:4:1:1) = 0.42 MS (EI) : 447 (M + H)⁺
e) 4-[4-N-(Dimethylaminoethyl)-methylsulfamoyl]phenoxy-3-trifluormethylbenzoesäuremethylester
   330 mg 4-[4-N-(Dimethylaminoethyl)-methylsulfamoyl]phenoxy-3-trifluormethylbenzoesäure und 1 ml SOCl₂ werden in 10 ml MeOH 8 h lang unter Rückfluß erhitzt. Die flüchtigen Bestandteile des Gemisches werden im Vakuum entfernt; der Rückstand wird mit je 100 ml gesättigter wäßriger Na₂CO₃-Lösung und 100 ml EE aufgenommen, und dann wird 3 x mit je 100 ml EE extrahiert. Über Na₂SO₄ wird getrocknet und das Lösungsmittel im Vakuum entfernt. Chromatographie an Kieselgel mit EE/MeOH 2:1 liefert 150 mg eines farblosen Harzes.
   R_{f} (EE/MeOH 1:1) = 0.30 MS (El) :461 (M + H)⁺
f) 4-[4-N-(Dimethylaminoethyl)-methylsulfamoyl]phenoxy-3-trifluormethylbenzoylguanidin
   140 mg 4-[4-N-(Dimethylaminoethyl)-methylsulfamoyl]phenoxy-3-trifluormethylbenzoesäuremethylester und 90 mg Guanidin werden in 3 ml Isopropanol nach der allgemeinen Vorschrift zur Herstellung von Benzoyl-guanidinen, Variante B umgesetzt. Man erhält 130 mg eines amorphen Feststoffs.
   R_{f} (EE/MeOH 1:1) = 0.12 MS (EI) : 488 (M + H)⁺
   mp (Dihydrochlorid) = 203°C

Die Titelverbindungen der Beispiele 4 - 8 wurden analog Beispiel 3 synthetisiert:

### Beispiel 4: 4-[4-(4-Methylpiperazinosulfonyl)phenoxy]-3-trifluormethylbenzoylguanidin, Dihydrochlorid

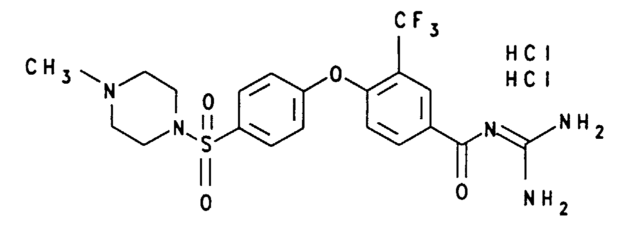

R_{f} (EE/MeOH 1:1) = 0.15 MS (El) : 486 (M + H)⁺
mp (Dihydrochlorid) > 250°C

### Beispiel 5: 4-[4-(2-Pyrolidinethylaminosulfonyl)phenoxy]-3-trifluormethylbenzoylguanidin, Dimaleinat

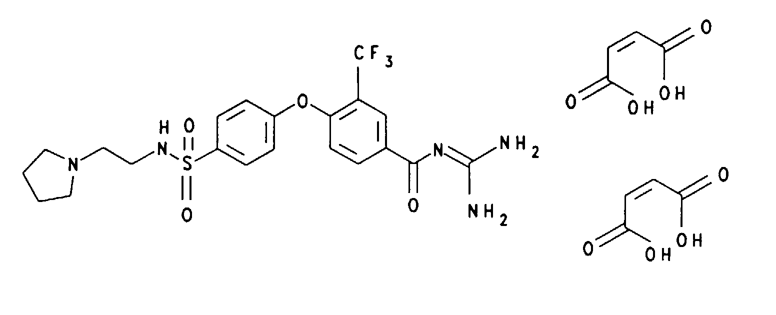

R_{f}(CH₂Cl₂/MeOH/HOAc/H₂O 8:4:1:1) = 0.37 MS (FAB) : 500 (M + H)⁺

### Beispiel 6: 4-[4-(2-Piperidinethylaminosulfonyl)phenoxy]-3-trifluormethylbenzoylguanidin, Dimaleinat

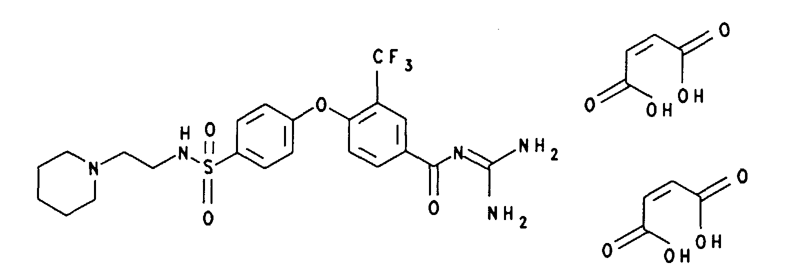

R_{f}(CH₂Cl₂/MeOH/HOAc/H₂O 8:4:1:1) = 0.40 MS (FAB) : 514 (M + H)⁺

### Beispiel 7: 4-[4-(N-Dimethylamino-n-propyl)sulfamoyl]phenoxy-3-trifluormethylbenzoylguanidin

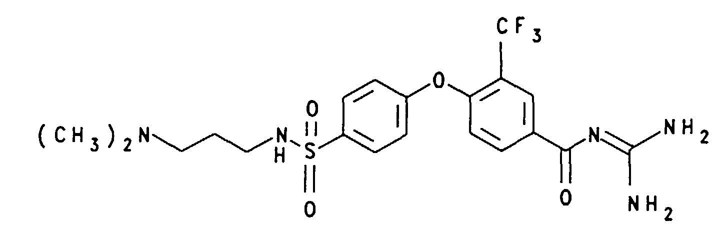

R_{f} (EE/MeOH 1:1) = 0.06 MS (EI) : 488 (M + H)⁺

### Beispiel 8: 4-[4-(N-Dimethylaminoethyl)sulfamoyl]phenoxy-3-trifluormethylbenzoylguanidin

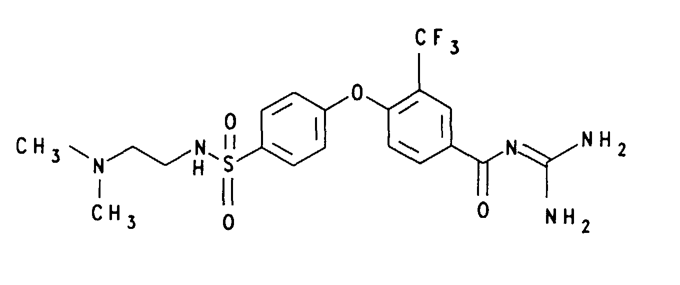

R_{f} (EE/MeOH 1:1) = 0.17 MS (EI) 474 (M + H)⁺

### Beispiel 9: 4-(4-lmidamidosulfonyl)phenoxy-3-trifluormethyl-benzoylguanidin

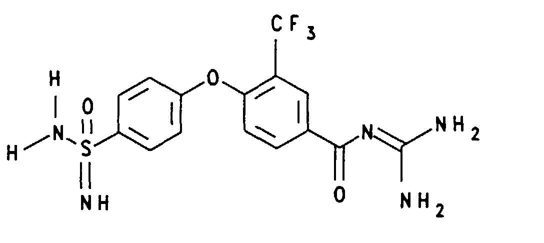

a) 4-[4-(N-t-Butylimido-N'-t-butyl)sulfamoyl]phenoxy-3-trifluormethyl-benzoesäure
   7.9 g 4-[4-(N-t-Butylimido-N'-t-butyl)sulfamoyl]phenoxy-3-trifluormethylbenzoesäuremethylester (Beispiel 2 c) werden in 100 ml MeOH gelöst und 40 ml einer 2 N wäßrigen NaOH-Lösung zugegeben. 3 h wird unter Rückfluß gekocht, das MeOH im Vakuum entfernt und der Rückstand in einem Gemisch aus 100 ml Wasser und 100 ml EE aufgenommen. 500 ml gesättigte wäßrige NaH₂PO₄-Lösung wird zugegeben und 3 mal mit je 200 ml EE extrahiert. Über Na₂SO₄ wird getrocknet und das Solvens im Vakuum entfernt. Man erhält 7.2 g weißer Kristalle.
   R_{f} (MTB) = 0.25 MS (ES) : 473 (M + H)⁺
   mp = 200 °C
b) 4-(4-Imidosulfamoyl)phenoxy-3-trifluormethyl-benzoesäure
   6.6 g 4-[4-(N-t-Butylimido-N'-t-butyl)sulfamoyl]phenoxy-3-trifluormethyl-benzoesäure werden in 140 ml wasserfreiem CH₂Cl₂ gelöst, 3.7 ml Trifluormethansulfonsäure zugegeben und 24 h bei RT gerührt. Das Gemisch wird in 1 l einer 0.66 M wäßrigen KH₂PO₄-Lösung eingerührt, die Methylenchlorid-Phase abgetrennt und 3 mal mit je 300 ml EE extrahiert. Die vereinigten organischen Phasen werden über Na₂SO₄ getrocknet und die Solventien im Vakuum entfernt. Man erhält 6.7 g eines zähen Öls, das ohne weitere Reinigung eingesetzt wird.
   R_{f} (EE/MeOH 5:1) = 0.21 MS (ES): 361 (M + H)⁺
c) 4-(4-lmidosulfamoyl)phenoxy-3-trifluormethyl-benzoesäuremethylester und
d) 4-(4-N-Methyl-imidosulfamoyl)phenoxy-3-trifluormethyl-benzoesäuremethylester 6.7 g 4-(4-lmidosulfamoyl)phenoxy-3-trifluormethyl-benzoesäure werden in 100 ml MeOH gelöst, 20 ml einer 2 N Lösung von Trimethylsilyldiazomethan in HEP zugetropft und 6 h bei RT gerührt. Anschließend werden 200 ml einer 20% wäßrigen Essigsäurelösung zugegeben und 30 Minuten nachgerührt. Dann werden 200 ml EE zugegeben und 9 mal mit je 100 ml einer 1 N wäßrigen HCI-Lösung extrahiert. Die wäßrige Phase wird anschließend mit Na₂CO₃ auf pH = 10 eingestellt und 3 mal mit je 200 ml EE extrahiert. Über Na₂SO₄ wird getrocknet und das Solvens im Vakuum entfernt. Der Rückstand wird an Kieselgel mit EE/HEP 1:1 chromatographiert und man erhält 1.2 g Produkt c) neben 890 mg Produkt d), in beiden Fällen Öle.
   c) R_{f} (EE) = 0.32 MS (ES) : 375 (M + H)⁺
   d) R_{f} (EE) = 0.38 MS (ES) : 389 (M + H)⁺
e) 4-(4-Imidamidosulfonyl)phenoxy-3-trifluormethyl-benzoylguanidin
   220 mg 4-(4-Imidosulfamoyl)phenoxy-3-trifluormethyl-benzoesäuremethylester und 174 mg Guanidin werden in 10 ml THF nach der allgemeinen Vorschrift zur Herstellung von Benzoyl-guanidinen, Variante B umgesetzt. Man erhält nach Chromatographie an Kieselgel mit EE/MeOH 5:1 80 mg farbloser Kristalle.
   R_{f} (EE/MeOH 5:1) = 0.22 MS (ES) : 402 (M + H)⁺
   mp = 156°C

### Beispiel 10: 3-Trifluormethyl-4-(4-N-methylimidamidosulfonyl)phenoxybenzoylguanidin

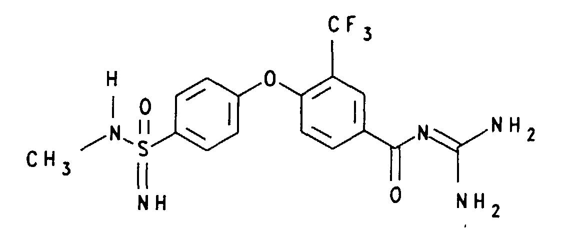

490 mg 4-(4-N-Methyl-imidosulfamoyl)phenoxy-3-trifluormethylbenzoesäuremethylester (Beispiel 9 d) und 373 mg Guanidin werden in 20 ml THF nach der allgemeinen Vorschrift zur Herstellung von Benzoyl-guanidinen, Variante B umgesetzt. Man erhält nach Chromatographie an Kieselgel mit EE/MeOH 5:1 370 mg farbloser Kristalle.
R_{f} (EE/MeOH 5:1) = 0.33 MS (ES) : :416 (M + H)⁺ mp (Dihydrochlorid) = 233 °C

Die Titelverbindung des Beispiels 11 wurde analog Beispiel 3 aus 3-Methyl-4-phenoxy-benzoesäuremethylester synthetisiert:

### Beispiel 11: 3-Methyl-4-(4-(1-methylpiperazin-4-ylsulfonyl)phenoxy)-benzoylguanidin

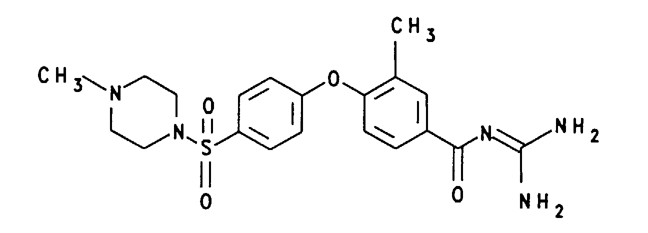

a) 3-Methyl-4-phenoxy-benzoesäuremethylester
   3.4 g 4-Fluor-3-methyl-benzoesäuremethylester, 2.4 g Phenol und 19.5 g Cs₂CO₃ werden in 100 ml NMP 20 Minuten lang bei 160 °C gerührt. Das Gemisch wird in 400 ml einer gesättigten wäßrigen NaHCO₃-Lösung gegossen, mit 400 ml Wasser verdünnt und 3 mal mit je 200 ml MTB extrahiert. Über Na₂SO₄ wird getrocknet und das Solvens im Vakuum entfernt. Chromatographie des Rückstands an Kieselgel mit EE/HEP 1:4 liefert 2.0 g eines farblosen Öls.
   R_{f} (EE/HEP 1:4) = 0.33 MS (El) : 243 (M + H)⁺

Die Titelverbindung des Beispiels 12 wird analog Beispiel 9 synthetisiert:

### Beispiel 12: 3-Methylsulfonyl-4-(4-imidamidosulfonyl)phenoxy-benzoylguanidin

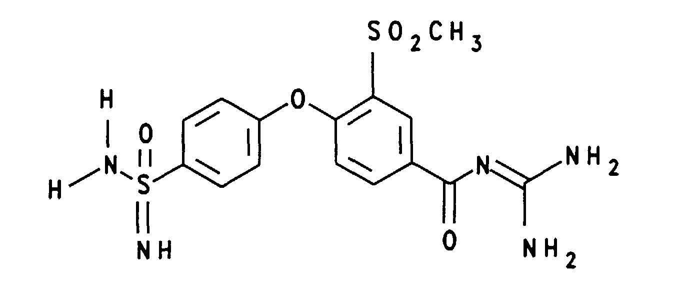

R_{f} (EE/MeOH 5:1) = 0.24 MS (ES) : 412 (M + H)⁺

### Beispiel 13: 4-(4-Guanidinosulfonyl)phenoxy-3-trifluormethyl-benzoylguanidin

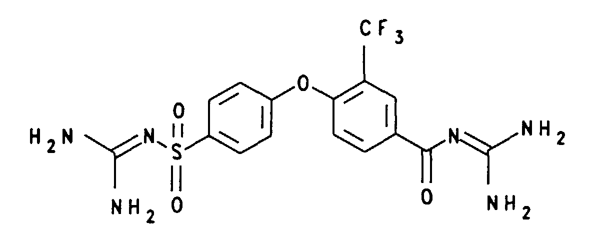

a) 4-(4-Hydroxysulfonyl)phenoxy-3-trifluormethyl-benzoesäure
   13.5 g 4-Phenoxy-3-trifluormethyl-benzoesäure (Beispiel 3 b) werden in 150 ml CHCl₃ gelöst, bei RT 3.3 ml Chlorsulfonsäure zugetropft und 3 h nachgerührt. Das Solvens wird im Vakuum entfernt, 300 g Eis und 100 ml Wasser zugegeben und 10 Minuten gerührt. Anschließend werden 400 ml einer auf 0 °C gekühlten, gesättigten wäßrigen NaCl-Lösung zugegeben, weitere 15 Minuten bei 0 °C gerührt und der Niederschlag abgesaugt. Bei 40 °C wird im Vakuum getrocknet und man erhält 16.5 g eines weißen Feststoffs, der ohne Reinigung weiter eingesetzt wird.
b) 4-(4-Chlorsulfonyl)phenoxy-3-trifluormethyl-benzoylchlorid
   14.0 g 4-(4-Hydroxysulfonyl)phenoxy-3-trifluormethyl-benzoesäure und 1 ml DMF werden in 250 ml SOCl₂ gelöst und 8 h unter Rückfluß erhitzt. Etwa die Hälfte des überschüssigen SOCl₂ wird anschließend im Vakuum entfernt und die Lösung auf 1 kg Eis getropft. 3 mal wird mit je 500 ml CH₂Cl₂ extrahiert, über MgSO₄ getrocknet und das Solvens im Vakuum entfernt. Man erhält 18.0 g eines Öls, das ohne weitere Reinigung eingesetzt wird.
   R_{f} (DIP/2%HOAc) = 0.51
c) 4-[4-(3-Pyridyloxy)sulfonyl]phenoxy-3-trifluormethyl-benzoesäure-3-hydroxypyridylester
   18.0 g 4-(4-Chlorsulfonyl)phenoxy-3-trifluormethyl-benzoylchlorid werden in 150 ml Aceton gelöst, mit 3.7 g 3-Hydroxypyridin und 11.0 g K₂CO₃ versetzt und 3 h bei RT gerührt. Das Reaktionsgemisch wird auf 500 ml Wasser gegossen und 3 mal mit je 300 ml EE extrahiert. Über Na₂SO₄ wird getrocknet, das Solvens im Vakuum entfernt und an Kieselgel mit MTB/2%HOAc chromatographiert. Man erhält 8.0 g eines gelblichen Öls.
   R_{f} (MTB/2%HOAc) = 0.29 MS (FAB): 517 (M + H)⁺
d) 4-(4-Guanidinosulfonyl)phenoxy-3-trifluormethyl-benzoylguanidin
   3.0 g 4-[4-(3-Pyridyloxy)sulfonyl]phenoxy-3-trifluormethyl-benzoesäure-3-hydroxypyridylester und 3.4 g Guanidin werden in 10 ml i-Propanol gelöst und 3 h unter Rückfluß erhitzt. Das Solvens wird im Vakuum entfernt, mit 400 ml Wasser aufgenommen mit wäßriger HCI-Lösung auf pH = 8 eingestellt und 2 h bei RT gerührt. Der Niederschlag wird abfiltriert und an Kieselgel mit EE/MeOH 5:1 chromatographiert. Man erhält 272 mg eines amorphen Feststoffs.
   R_{f} (EE/MeOH 5:1) = 0.33 MS (ES) : 445 (M + H)⁺

### Beispiel 14: 3-Methylsulfonyl-[4-(2-dimethylaminoethyl)phenoxy]benzoyl-guanidin Bis-methansulfonat

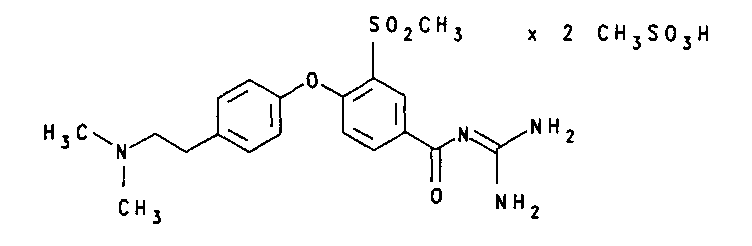

14 a) 5-Carboxy-2-fluorbenzolsulfinsäure
   15,6 g (0,124 M) Natriumsulfit werden bei 70°C in 120 ml Wasser gelöst. Unter Beibehaltung der Temperatur gibt man gleichzeitig und portionsweise 23,8 g (0,1 M) 4-Fluor-3-chlorsulfonylbenzoesäure und 10 n NaOH so zu, daß der pH zwischen 9 und 10 gehalten wird (exotherme Reaktion). Man rührt weitere 3 Stunden bei 70°C, läßt weiter 15 Minuten mit A-Kohle rühren und filtriert sodann. Das Filtrat wird unter Außenkühlung mit konzentrierter Salzsäure auf pH 0 - 1 gestellt und die kristalline 5-Carboxy-2-fluorbenzolsulfinsäure abfiltriert. Farblose Kristalle,
   Fp.: 167 - 170°C.
14 b) 5-Carboxy-2-fluorbenzolsulfinsäure Dinatriumsalz
   erhält man durch Eintragen von 17,2 g (0,084 M) Carboxy-2-fluorbenzolsulfinsäure in eine gerührte Lösung von 6,72 g (0,168 M) NaOH in einem Gemisch aus 150 ml Methanol und 30 ml Wasser: Nach Filtration von Schwebestoffen destilliert man das Lösungsmittel ab und bringt den Rückstand unter Aceton zur Kristallisation. Farblose kristalline Substanz, Fp.: >320°C.
14 c) 4-Fluor-3-methylsulfonylbenzoesäuremethylester
   Zu einer Suspension von 15 g (0,06 M) 5-Carboxy-2-fluorbenzolsulfinsäure Dinatriumsalz in 80 ml trockenem DMF gibt man 30 g (0,21 M) Methyliodid, rührt über 6 Stunden bei 60°C, destilliert das Lösungsmittel ab und versetzt den Rückstand mit Wasser. Man rührt 30 Min. unter Eiskühlung und filtriert den
   Niederschlag ab.
   Farblose kristalline Substanz,
   Fp.: 102-105°C.
14 d) 3-Methylsulfonyl-[4-(2-dimethylaminoethyl)phenoxy]-benzoesäuremethylester
   4,64 g (0,02 M) 4-Fluor-3-methylsulfonylbenzoesäuremethylester werden in eine Mischung aus 60 ml Dimethylacetamid, 3,6 g (0,022 M) N,N-Dimethyl-2-(4-hydroxyphenyl)ethylamin und 9,08 g (0,066 M) gepulvertes wasserfreies K₂CO₃ gegeben und die Suspension für 4 Stunden bei 90°C gerührt. Nach Abdestillieren des Lösungsmittels und Versetzen des Rückstandes mit Wasser extrahiert man mehrmals mit Essigsäureethylester, engt die vereinigten organischen Phasen unter vermindertem Druck ein und erhält die Substanz als gelbe ölige Flüssigkeit.
14 e) Darstellung von 3-Methylsulfonyl-4-[4-(2-dimethylaminoethyl)phenoxy]benzoesäure Hydrochlorid
   1,88 g (0,005 M) 3-Methylsulfonyl-4-[4-(2-dimethylaminoethyl)phenoxy]benzoesäuremethylester werden in 40 ml halbkonzentrierter Salzsäure über 5 Stunden unter Rückfluß gekocht, die wäßrige Salzsäure abdestilliert und der Rückstand unter Aceton zur Kristallisation gebracht.
   Farblose kristalline Substanz, Fp.: 246 - 248°C.
14 f) 3-Methylsulfonyl-4-[4-(2-dimethylaminoethyl)phenoxy]benzoylguanidin erhält man analog der in Variante A angegebenen Vorschrift aus 3-Methylsulfonyl-4-[4-(2-dimethylaminoethyl)phenoxy]benzoesäure bei einem pH zwischen 7 und 8. Farblose Kristalle, Fp.: 214 - 218°C.

3-Methylsulfonyl-4-[4-(2-dimethylaminoethyl)phenoxy]benzoylguanidin Bis-methansulfonat
erhält man analog der in Variante A angegebenen Vorschrift aus 3-Methylsulfonyl-4-[4-(2-dimethylaminoethyl)phenoxy]benzoylguanidin durch Behandeln mit 2,5 eq Methansulfonsäure in Ethanol.
Farbloser Feststoff,
Fp.: 102°C.

### Beispiel 15: 4-[4-(2-Dimethylaminoethyl)thiomethyl-phenoxy]-3-methylsulfonylbenzoyl-guanidin Dihydrochlorid

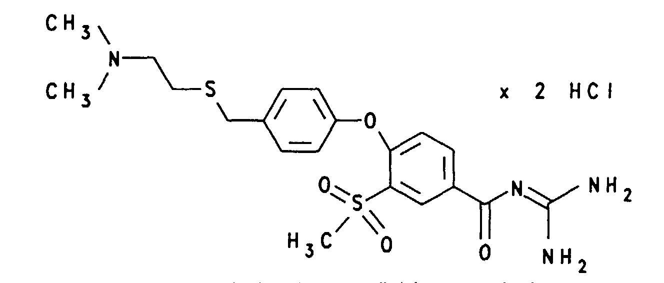

15 a) Darstellung von 4-(2-Dimethylaminoethyl)thiomethyl-phenol
   Eine Mischung aus 200 mg p-Toluolsulfonsäure, 14,1 g (0,1 M) 2-Dimethylaminoethylmercaptan Hydrochlorid und 12,4 g (0,1 M) 4-Hydroxybenzylalkohol in 250 ml Toluol wird über ca. 5 Stunden am Wasserabscheider nach Kutscher und Steudel unter Rückfluß gekocht, das Lösungsmittel vertrieben und der Rückstand nach Auflösung in Methanol filtriert. Nach erneutem Einengen bringt man den Rückstand unter Aceton zur Kristallisation, filtriert das kristalline Produkt ab und trocknet die leicht hygroskopische Masse über NaOH unter Luftabschluß.
   Fp.: 134 - 140°C.
15 b) 4-[4-(2-Dimethylaminoethyl)thiomethyl-phenoxy]-3-methylsulfonylbenzoesäuremethylester
   Eine Mischung aus 3,48 g (0,015 M) 4-(2-Dimethylaminoethyl)thiomethyl-phenol, 40 ml wasserfreiem Tetramethylharnstoff, 6,8 g (0,049 M) wasserfreiem gepulvertem K₂CO₃ und 3,48 g (0,015 M) 4-Fluor-3-methylsulfonylbenzoesäure-methylester wird 6 Stunden bei 90 - 100°C gerührt, das Lösungsmittel abdestilliert und der Rückstand mit Wasser. Nach Extraktion mit Essigester, Trocknen und Einengen der vereinigten organischen Phasen erhält man das gewünschte Produkt als Öl.
15 c) 4-[4-(2-Dimethylaminoethyl)thiomethyl-phenoxy]-3-methylsulfonyl-benzoesäure Hydrochlorid
   erhält man analog der in Beispiel 14 d) beschriebenen Vorschrift durch Hydrolyse von 4-Fluor-3-methylsulfonylbenzoesäure-methylester in 20%iger HCI. Farblose bis hellgelbe kristalline Substanz,
   Fp.: 179 - 185°C.
15 d) 4-[4-(2-Dimethylaminoethyl)thiomethyl-phenoxy]-3-methylsulfonylbenzoylguanidin Dihydrochlorid
   erhält man analog der in Beispiel 14 e) beschriebenen Vorschrift aus 4-[4-(2-Dimethylaminoethyl)thiomethyl-phenoxy]-3-methylsulfonylbenzoesäure Hydrochlorid. Hygroskopische Substanz, Fp.: 230°C.

### Beispiel 16: 4-[4-(2-Dimethylaminoethylthio)phenoxy]-3-methylsulfonylbenzoylguanidin Dihydrochlorid

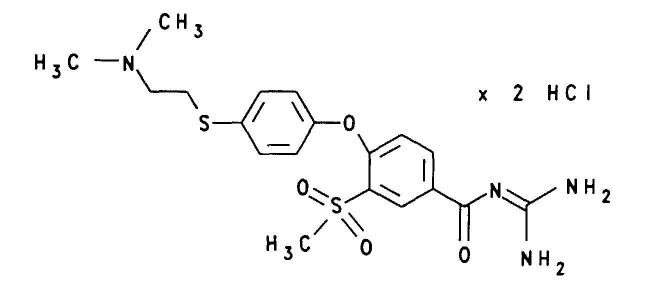

16 a) N,N-Dimethyl-2-(4-hydroxyphenylthio)ethylamin
   Zu einer Lösung aus 12,6 g (0,1 M) 4-Mercaptophenol in 100 ml wasserfreiem DMF gibt man unter Schutzgasatmosphäre (Argon) 17,28 g (0,11 M) 2-Dimethylaminoethylchlorid Hydrochlorid und anschließend 19,38 g (0,15 M) Ethyldiisopropylamin und erhitzt die Reaktionsmischung für 12 Stunden unter magnetischer Rührung auf 110°C. Nach Abdestillieren des Lösungsmittels versetzt man den Rückstand mit Wasser, stellt mit 2 N NaOH auf pH 8-9, extrahiert den amorphen Niederschlag mit mehrfach Ethylacetat und trocknet die vereinten organischen Phasen über Natriumsulfat. Das Lösungsmittel wird abdestilliert und der Rückstand an Kieselgel säulenchromatografisch mit einem Gemisch bestehend aus 8 Teilen Ethylacetat und 1 Teil Methanol eluiert.
   Farblose Kristalle,
   Fp. 108 - 110°C.
16 b) 4-[4-(2-Dimethylaminoethylthio)phenoxy]-3-methylsulfonylbenzoesäuremethylester
   erhält man als gelbes Öl analog der in 14 d) beschriebenen Vorschrift durch Umsetzung von N,N-Dimethyl-2-(4-hydroxyphenylthio)ethylamin mit 4-Fluor-3-methylsulfonylbenzoesäure-methylester, wobei DMF anstelle von Dimethylacetamid als Reaktionsmedium verwendet wurde.
   Gelbe ölige Flüssigkeit.
16 c) 4-[4-(2-Dimethylaminoethylthio)phenoxy]-3-methylsulfonylbenzoesäure
   6,8 g 4-[4-(2-Dimethylaminoethylthio)phenoxy]-3-methylsulfonylbenzoesäuremethylester werden in 10 ml Eisessig und 70 ml halbkonzentrierter HCI 5 Stunden unter Rückflußbedingungen gekocht. Nach dem Abdestillieren des Lösungsmittels erhält man die gewünschte Substanz als amorphen Feststoff ohne definierten Schmelzpunkt.
16 d) 4-[4-(2-Dimethylaminoethylthio)phenoxy]-3-methylsulfonylbenzoylguanidin Dihydrochlorid
   erhält man analog der als Variante A angegeben Vorschrift.
   Farbloser Feststoff,
   Zersetzungspunkt: 160°C unter Aufschäumen.

### Beispiel 17: 4-[4-(2-Dimethylaminoethylsulfonyl)phenoxy]-3-methylsulfonylbenzoylguanidin Dihydrochlorid

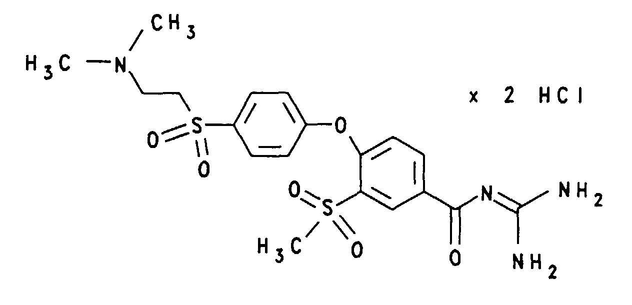

17 a) 4-[4-(2-Dimethylaminoethylsulfonyl)phenoxy]-3-methylsulfonyl-benzoesäure Zu einer Lösung von 3,8 g (0,008 M) 4-[4-(2-Dimethylaminoethylthio)phenoxy]-3-methylsulfonylbenzoesäure in 50 ml Eisessig gibt man portionsweise bei 5-10°C 6,9 g (0,028 M) 3-Chlorperbenzoesäure und rührt 12 Stunden bei RT. Nach Zugabe von Wasser filtriert man den Niederschlag der 3-Chlorbenzoesäure ab und extrahiert aus dem Filtrat weitere Verunreinigungen mit Essigester. Die wäßrige Phase wird eingeengt und der amorphe Rückstand unter Ethylacetat zur Kristallisation gebracht. Farblose Kristalle,
   Fp.: 167 - 171°C
17 b) 4-[4-(2-Dimethylaminoethylsulfonyl)phenoxy]-3-methylsulfonylbenzoyl-guanidin Dihydrochlorid
   erhält man analog der in Variante A angegebenen Vorschrift aus 4-[4-(2-Dimethylaminoethylsulfonyl)phenoxy]-3-methylsulfonylbenzoesäure in TMU als Reaktionsmedium. Das Dihydrochlorid wird unter Methanol zur Kristallisation gebracht.
   Farblose Kristalle,
   Fp.: 233 - 240°C (Zersetzung).

### Beispiel 18: 4-[(4-Guanidinocarbonyl)phenoxy]-3-methylsulfonylbenzoylguanidin Dihydrochlorid

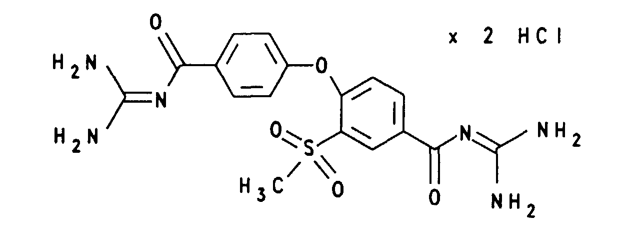

18 a) von 4-(4-Carboxyphenoxy)-3-methylsulfonylbenzoesäure
   Durch Umsetzung von 4-Hydroxybenzoesäureethylester mit 4-Fluor-3-methylsulfonylbenzoesäuremethylester erhält man analog der unter 14 d) angegebenen Vorschrift den 4-(4-Ethoxycarbonylphenoxy)benzoesäure-methylester als farbloses bis hellgelbes Öl, das ohne weitere Reinigungsmaßnahmen analog der in Vorschrift 16 c) angegebenen Weise zur 4-(4-Carboxyphenoxy)-3-methylsulfonylbenzoesäure hydrolysiert wird.
   Farblose Kristalle,
   Fp.: 272 - 275°C.
18 b) 4-[(4-Guanidinocarbonyl)phenoxy]-3-methylsulfonylbenzoylguanidin Dihydrochlorid
   erhält man analog der in Variante A beschriebenen Vorschrift durch Umsetzung von 0,74 g (0,0022 M) (4-Carboxyphenoxy)-3-methylsulfonylbenzoesäure mit 0,78 g (0,0048 M) Carbonyldiimidazol und 1,55 g (0,026 M) Guanidin in DMA.
   Farblose Kristalle,
   Fp.: 252°C (Zersetzung).

### Beispiel 19: 4-[4-(2-Dimethylaminoethyl)thiomethyl-phenoxy]-3-trifluormethylbenzoylguanidin Dihydrochlorid

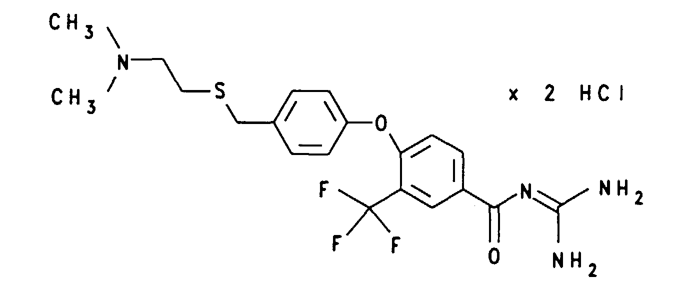

19 a) 4-[4-(2-Dimethylaminoethyl)thiomethyl-phenoxy]-3-trifluormethyl-benzoesäuremethylester
   erhält man analog der in Beispiel 15 b) beschriebenen Vorschrift aus 4-(2-Dimethylaminoethyl)thiomethyl-phenol und 4-Fluor-3-trifluormethylbenzoesäuremethylester in DMU als Reaktionsmedium als amorphes, öliges Produkt.
19 b) 4-[4-(2-Dimethylaminoethyl)thiomethyl-phenoxy]-3-trifluormethyl-benzoesäure erhält man analog der in Beispiel 14 d) beschriebenen Vorschrift durch saure Hydrolyse von 4-[4-(2-Dimethylaminoethyl)thiomethyl-phenoxy]-3-trifluormethylbenzoesäure-methylester.
   Farblose, hygroskopische Kristalle,
   Fp.: 158 - 168°C (Zersetzung).
19 c) 4-[4-(2-Dimethylaminoethyl)thiomethyl-phenoxy]-3-trifluormethylbenzoylguanidin Dihydrochlorid
   erhält man analog der in Variante A beschriebenen Vorschrift aus 4-[4-(2-Dimethylaminoethyl)thiomethyl-phenoxy]-3-trifluormethylbenzoesäure in TMU als Reaktionsmedium. Amorpher, stark hygroskopischer Feststoff,
   Zersetzung bei 80 - 85°C.

### Beispiel 20: 4-[4-(2-Dimethylaminoethylthio)phenoxy]-3-trifluormethylbenzoylguanidin Dihydrochlorid

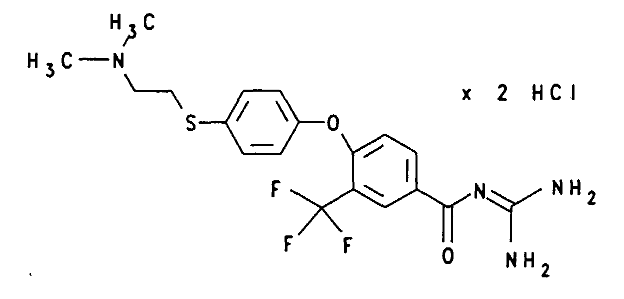

20 a) 4-[4-(2-Dimethylaminoethylthio)phenoxy]-3-trifluormethyl-benzoesäuremethylester
   erhält man analog der in Beispiel 15 b) beschriebenen Vorschrift aus 4-(2-Dimethylaminoethylthio)phenol und 4-Fluor-3-trifluormethylbenzoesäuremethylester in DMU als Reaktionsmedium als amorphes, öliges Produkt.
20 b) 4-[4-(2-Dimethylaminoethylthio)phenoxy]-3-trifluormethyl-benzoesäure erhält man analog der in Beispiel 14 d) beschriebenen Vorschrift durch saure Hydrolyse von 4-[4-(2-Dimethylam inoethylthio)phenoxy]-3-trifluormethylbenzoesäuremethylester. Die gewünschte 4-[4-(2-Dimethylaminoethylthio)phenoxy]-3-trifluormethyl-benzoesäure kommt unter Aceton zur Kristallisation.
   Farblose Kristalle,
   Fp. 174 - 182°C (Zersetzung)
20 c) 4-[4-(2-Dimethylaminoethylthio)phenoxy]-3-trifluormethylbenzoylguanidin Dihydrochlorid
   erhält man analog der in Variante A beschriebenen Vorschrift aus 4-[4-(2-Dimethylaminoethylthio)phenoxy]-3-trifluormethyl-benzoesäure in THF als Reaktionsmedium.
   Amorpher, hygroskopischer kristalliner Feststoff,
   Fp.: 240°C.

### Beispiel 21: 3-Trifluormethyl-4-[4-(2-dimethylaminoethyl)p henoxy]benzoyl-guanidin Dihydrochlorid

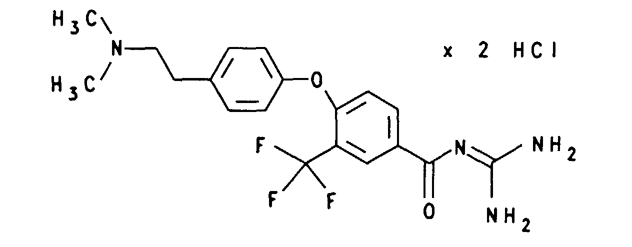

21 a) 4-Chlor-3-trifluormethyl-benzoesäure
   Aus 100 g 5-Brom-2-chlor-benzotrifluorid und 10,2 g Magnesium in 600 ml Diethylether wird zunächst die Grignard-Verbindung hergestellt. Anschließend wird in diese Lösung bei RT ein Strom von 60 g wasserfreiem CO₂ eingeleitet. Man tropft 500 ml gesättigte wäßrige NaHSO₄-Lösung zu, trennt die Phasen und extrahiert noch mit 2 x 100 ml Diethylether. Die organische Phase wird 3 x mit je 300 ml 1 n NaOH extrahiert, anschließend die wäßrige Phase 3 x mit je 100 ml Diethylether gewaschen. Die wäßrige Phase wird nun mit HCI auf pH 2 gebracht und mit Wasser auf 4 1 verdünnt. Das Produkt wird abgesaugt und im Vakuum getrocknet.
   75 g weißes Pulver.
   Rf(MTB 2% HOAc) = 0.68
21 b) 4-Chlor-3-trifluormethyl-benzoesäure-methylester
   75 g 4-Chlor-3-trifluormethyl-benzoesäure werden in 500 ml MeOH gelöst und 75 ml SOCl₂ zugetropft. 5 h wird unter Rückfluß gekocht, anschließend die flüchtigen Bestandteile im Vakuum entfernt. Mit 1 IEE wird aufgenommen und mit 500 ml gesättigter wäßriger Na₂CO₃-Lösung gewaschen. Über Na₂SO₄ wird getrocknet, das Solvens im Vakuum entfernt und im Vakuum destilliert.
   Sdp. 94°C (2 Torr)
   Rf (DIP) = 0.49 MS (DCI): 239 (M + H)⁺
21 c) 4-[4-(2-Dimethylamino)ethyl]phenoxy-3-trifluormethyl-benzoesäure-methylester
   1.9 g 4-Chlor-3-trifluormethyl-benzoesäure-methylester, 1.3 g 4-(2-Dimethylamino)ethyl-phenol, 7.8 g Cs₂CO₃ werden in 50 ml Tetramethylharnstoff 5 h lang bei 140°C gerührt. Man läßt abkühlen, dann werden 300 ml gesättigte wäßrige NaHCO₃-Lösung und 150 ml Wasser zugegeben, und es wird 3 x mit je 100 ml EE extrahiert. Über Na₂SO₄ wird getrocknet, anschließend die Lösemittel im Vakuum entfernt. Chromatographie mit Aceton/Wasser 10:1 liefert 2.0 g eines farblosen Öls.
   Rf (Aceton/Wasser 10:1) = 0.15
   MS (DCI): 368 (M + H)⁺
21 d) 4-[4-(2-Dimethylamino)ethyl]phenoxy-3-trifluormethyl-benzoylguanidin
   2.0 g 4-[4-(2-Dimethylamino)ethyl]phenoxy-3-trifluormethyl-benzoesäure-methylester werden mit 1.6 g Guanidin in 60 ml Isopropanol nach Variante B guanyliert. Das Rohprodukt wird in das Dihydrochlorid überführt und aus 1,2-Dimethoxyethan/Wasser 9 : 1 umkristallisiert.
   mp (freie Base):164°C
   mp (Dihydrochlorid) : 236°C MS (DCI): 395 (M + H)⁺
   Wasserlöslichkeit des Dihydrochlorids 49 mg/ml (pH = 5.3)

### Beispiel 22: 4-[4-(2-N,N-Dimethylamino)ethyl]phenoxy-3-sulfamoyl-benzoyl-guanidin Bismethansulfonat

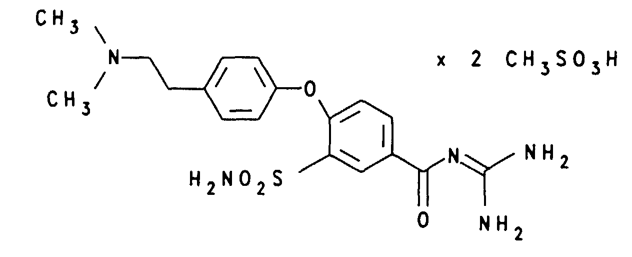

erhält man analog der in Variante A beschriebenen Vorschrift aus 4-[4-(2-Dimethylaminoethyl)phenoxy]-3-sulfamoylbenzoesäure in Dimethylacetamid oder N-Methylpyrrolidon als Reaktionsmedium. Amorpher, hygroskopischer
kristalliner Feststoff,
Fp.: 183°C.
a) 4-[4-(2-Dimethylaminoethyl)phenoxy]-3-sulfamoylbenzoesäure-methylester
   erhält man durch Erhitzen von 0,011 Mol N,N-Dimethyl-2-(4-hydroxyphenyl)ethylamin, 0,033 Mol Kaliumcarbonat (gemahlen) und 0,01 Mol 4-Fluor-3-sulfamoylbenzoesäuremethylester in 40 ml Dimethylacetamid über 5 Stunden auf 90-100°C. Nach Abdestillieren des Lösungsmittels wird der Rückstand mit Wasser verrührt, das braune amorphe Produkt mit Essigester behandelt, vom kristallinen Niederschlag abfiltriert und aus dem Filtrat das Lösungsmittel abdestilliert. Amorphes öliges Produkt.
b) 4-[4-(2-Dimethylaminoethyl)phenoxy]-3-sulfamoylbenzoesäure
   erhält man durch Hydrolyse von 0,0066 Mol des entsprechenden Methylesters (a) in 60 ml siedender halbkonzentrierter Salzsäure über 5 Stunden. Nach dem Verdampfen der wäßrigen Salzsäure im Vakuum behandelt man den Rückstand mit Aceton und filtriert den Niederschlag ab. Man destilliert das Lösungsmittel ab und erhält die gewünschte Benzoesäure. Erweichungspunkt ab 60°C.

### Beispiel 23: 3-Chlor-4-[4-(2-dimethylaminoethyl)phenoxy]-5-methylsulfonylbenzoylguanidin Bismethansulfonat

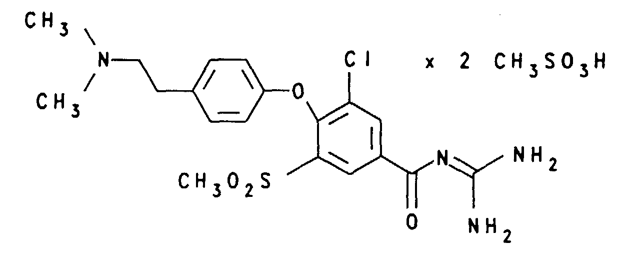

erhält man analog der in Variante A beschriebenen Vorschrift aus 3-Chlor-4-[4-(2-dimethylaminoethyl)phenoxy]-5-methylsulfonylbenzoesäure in Dimethylacetamid oder N-Methylpyrrolidon als Reaktionsmedium. Hygroskopischer kristalliner
Feststoff,
Fp.: 200°C.
a) 3-Chlor-4-[4-(2-dimethylaminoethyl)phenoxy]-5-methylsulfonylbenzoesäuremethylester
   erhält man analog Beispiel 22 a) durch Umsetzung von 3,4-Dichlor-5-methylsulfonylbenzoesäuremethylester mit N, N-Dimethyl-2-(4-hydroxyphenyl)ethylamin. Amorpher, öliger Stoff.
b) 3-Chlor-4-[4-(2-dimethylaminoethyl)phenoxy]-5-methylsulfonylbenzoesäure erhält man analog Beispiel 22 a). Kristalliner Feststoff,
   Zers. : 238 - 244°C.

### Beispiel 24: 4-[(4-Guanidinocarbonyl)phenoxy]-3-methylsulfonylbenzoylguanidin

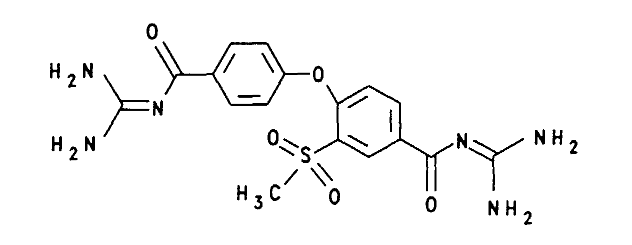

erhält man als salzfreie Base von Beispiel 18 durch Behandlung von 4-[(4-Guanidinocarbonyl)phenoxy]-3-methylsulfonylbenzoylguanidin Dihydrochlorid mit Triethylamin in DMF.
Farbloser kristalliner Feststoff, Fp.: 237°C

### Beispiel 25: 4-[4-(2-N-lmidazolyl-1-oxo-ethyl)phenoxy]-3-methylsulfonylbenzoylguanidin Dihydrochlorid

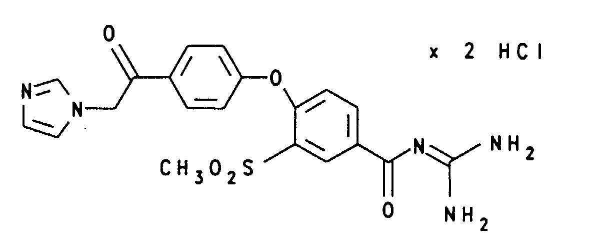

erhält man analog der in Variante A beschriebenen Vorschrift aus 4-[4-(2-N-Imidazolyl-1-oxo-ethyl)phenoxy]-3-methylsulfonylbenzoesäure in trockenem Dimethylacetamid oder N-Methylpyrrolidon als Reaktionsmedium. Farbloser kristalliner Feststoff, Zers.: 255°C.
a) 4-(4-Chloracetylphenoxy)-3-methylsulfonylbenzoesäure
   0,02 Mol 3-Methylsulfonyl-4-phenoxybenzoesäure werden portionsweise in eine Mischung aus 0,08 Mol Chloracetylchlorid, 16 g wasserfreies Aluminiumchlorid und 150 ml 1,2-Dichlorethan bei 0-5°C eingetragen, rührt 2 Stunden bei Raumtemperatur und rührt anschließend 2 Stunden bei 40-45°C. Nach Stehenlassen über Nacht gießt man das Reaktionsgemisch unter Rührung in Eiswasser, filtriert den kristallinen Niederschlag ab, wäscht mit Wasser und trocknet. Gelbe Kristalle, Fp.: 184-187°C.
b) 4-[4-(2-N-Imidazolyl-1-oxo-ethyl)phenoxy]-3-methylsulfonyl-benzoesäure
   Eine Lösung von 0,005 Mol 4-(4-Chloracetylphenoxy)-3-methylsulfonyl-benzoesäure (25,a) in 25 ml wasserfreiem DMF wird mit 0,0225 Mol Imidazol für 4 Stunden bei 60°C gerührt und sodann das Lösungsmittel abdestilliert. Nach Behandeln des amorphen Rückstandes mit Wasser und Einstellung eines pH 2-3 mit 2N Salzsäure wird eine Stunde gerührt und der kristalline Niederschlag abfiltriert. Kristalle, Zers. 235-241°C.

### Beispiel 26: 4-[4-(2-lmidazolylthio-acetyl)phenoxy]-3-methylsulfonyl-benzoylguanidin Dihydrochlorid

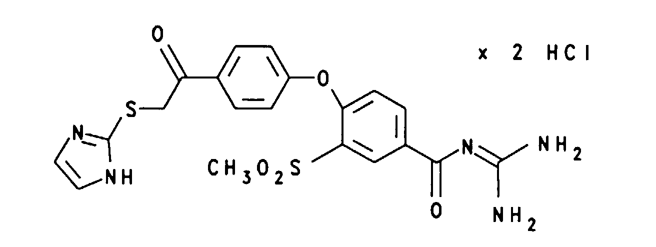

erhält man analog der in Variante A beschriebenen Vorschrift aus 4-[4-(2-Imidazolylthio-acetyl)phenoxy]-3-methylsulfonylbenzoesäure in einer Mischung aus 50 ml THF und 10 ml Dimethylacetamid als Reaktionsmedium. Farbloser kristalliner Feststoff,
Fp.: 220°C.
a) 4-[4-(2-lmidazolylth io-acetyl)phenoxy]-3-methylsulfonyl-benzoesäure erhält man durch Umsetzung von 4-(4-Chloracetylphenoxy)-3-methylsulfonylbenzoesäure (Beispiel 25 a) und 1 Mol 2-Mercaptoimidazol in 20 ml Aceton und kurzzeitigem Erwärmen zum Sieden. Man rührt ca. 5 Tage bei Raumtemperatur und filtriert den kristallinen Niederschlag ab.
   Zers.: 202 - 205°C.

### Beispiel 27: 4-[4-(4,5-Dihydro-2-imidazolylthio-acetyl)phenoxy]-3-methylsulfonylbenzoylguanidin Dihydrochlorid

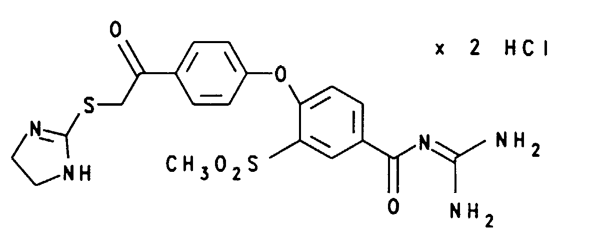

erhält man analog der in Variante A beschriebenen Vorschrift aus 4-[4-(4,5-Dihydro-2-imidazolylthio-acetyl)phenoxy]-3-methylsulfonylbenzoesäure in einer Mischung aus 50 ml THF und 10 ml Dimethylacetamid als Reaktionsmedium. Farbloser kristalliner Feststoff, Fp.: 210°C.
a) Darstellung von 4-[4-(4,5-Dihydro-2-imidazolylthio-acetyl)phenoxy]-3-methylsulfonylbenzoesäure erfolgt analog Vorschrift 26 a) durch Reaktion von 4-(4-Chloracetylphenoxy)-3-methylsulfonylbenzoesäure (Beispiel 25 a) mit 2-Mercapto-4,5-dihydroimidazol. Zers.: 305-310°C.

### Beispiel 28: 4-[4-(N,N'-Dimethyl-S-isothiuronyl-acetyl)phenoxy]-3-methylsulfonylbenzoylguanidin Dihydrochlorid

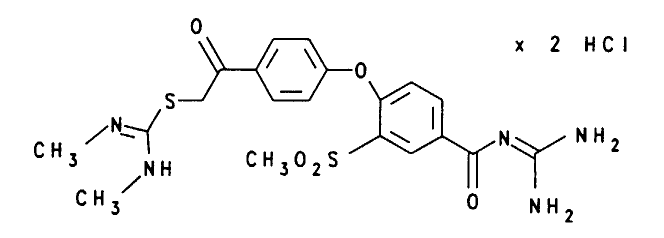

erhält man analog der in Variante A beschriebenen Vorschrift aus 4-[4-(N,N'-Dimethyl-S-isothiuronyl-acetyl)phenoxy]-3-methylsulfonylbenzoesäure in einer Mischung aus 50 ml THF und 10 ml Dimethylacetamid als Reaktionsmedium.
Farbloser kristalliner Feststoff,
Fp.: 150°C.
a) 4-[4-(N, N'-Dimethyl-S-isothiuronyl-acetyl)phenoxy]-3-methylsulfonyl-benzoesäure erhält man analog Vorschrift 26 a) durch Reaktion von 4-(4-Chloracetylphenoxy)-3-methylsulfonylbenzoesäure (Beispiel 25 a) mit N,N'-Dimethylthioharnstoff bei Raumtemperatur. Farblose Kristalle.
   Zers.: 185-190°C.

### Beispiel 29: 4-[4-(2-Benzimidazolylthio-acetyl)phenoxy]-3-methylsulfonylbenzoylguanidin Dihydrochlorid

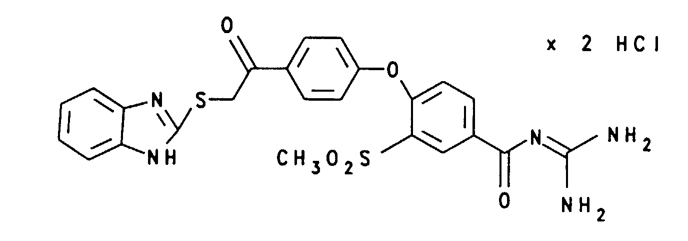

erhält man analog der in Variante A beschriebenen Vorschrift aus 4-[4-(2-Benzimidazolylthio-acetyl)phenoxy]-3-methylsulfonylbenzoesäure in TH F als Reaktionsmedium. Farbloser kristalliner Feststoff,
Fp.: 228°C.
a) 4-[4-(2-Benzimidazolylthio-acetyl)phenoxy]-3-methylsulfonylbenzoesäure erhält man Vorschrift 26 a) durch Reaktion von 4-(4-Chloracetylphenoxy)-3-methylsulfonylbenzoesäure (Beispiel 25 a) mit 2-Mercaptobenzimidazol in DMF.
   Fp.: 182°C.

### Beispiel 30: 4-[4-(2-Pyridylthio-acetyl)phenoxy]-3-methylsulfonyl-benzoyl-guanidin Dihydrochlorid

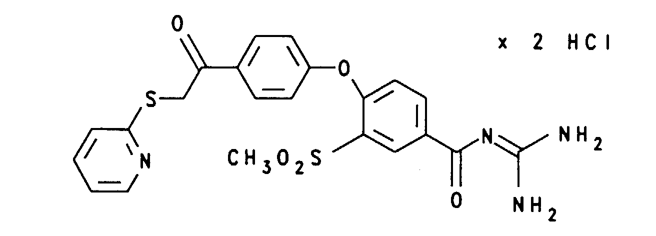

erhält man analog der in Variante A beschriebenen Vorschrift aus 4-[4-(2-Pyridylthioacetyl)phenoxy]-3-methylsulfonylbenzoesäure in TH F als Reaktionsmedium.
Farbloser kristalliner Feststoff,
Fp.: 203°C.
a) 4-[4-(2-Pyridylthio-acetyl)phenoxy]-3-methylsulfonylbenzoesäure erhält man analog Vorschrift 26 a) durch Reaktion von 4-(4-Chloracetylphenoxy)-3-methylsulfonylbenzoesäure (Beispiel 25 a) mit 2-Mercaptopyridin.
   Fp.: 194-196°C.

### Beispiel 31: 4-[4-(2-Chinolylthio-acetyl)phenoxy]-3-methylsulfonyl-benzoyl-guanidin Dihydrochlorid

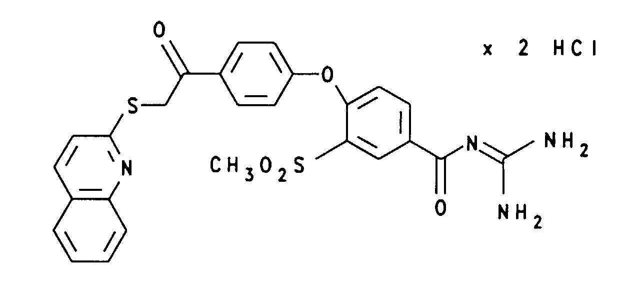

erhält man analog der in Variante A beschriebenen Vorschrift aus 4-[4-(2-Chinolylthio-acetyl)phenoxy]-3-methylsulfonylbenzoesäure in THF als Reaktionsmedium. Farbloser kristalliner Feststoff, Fp.: 192°C.
a) Darstellung von 4-[4-(2-Chinolylthio-acetyl)phenoxy]-3-methylsulfonylbenzoesäure erfolgt analog Vorschrift 26 a) durch Reaktion von 4-(4-Chloracetylphenoxy)-3-methylsulfonylbenzoesäure (Beispiel 25 a) mit 2-Mercaptochinolin in DMF. Fp. 210-214°C.

### Beispiel 32: 4-[4-(2-N-lmidazolyl-1-hydroxyethyl)phenoxy]-3-methylsulfonylbenzoylguanidin Dihydrochlorid

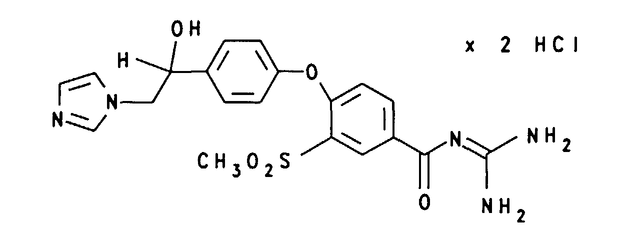

erhält man analog der in Variante A beschriebenen Vorschrift aus 4-[4-(2-N-Imidazolyl-1-hydroxyethyl)phenoxy]-3-methylsulfonylbenzoesäure in Dimethylacetamid oder N-Methylpyrrolidin als Reaktionsmedium. Farbloser kristalliner Feststoff,
Zersetzungspunkt: 260°C.
a) 4-[4-(2-N-lmidazolyl-1-hydroxyethyl)phenoxy]-3-methylsulfonylbenzoesäure erhält man durch Reduktion von 0,0034 Mol 4-[4-(2-N-lmidazolyl-1-oxoethyl)phenoxy]-3-methylsulfonyl-benzoesäure mit 0,0068 Mol Natriumboranat in 40 ml Ethanol. Nach Abdestillieren des Lösungsmittels, Versetzen des Rückstandes mit Wasser und Einstellung von pH 4 mit 2N HCI rührt man einige Stunden bei Raumtemperatur und filtriert die Kristalle ab.
   Fp. 240-248 °C.

### Beispiel 33: 3-Methylsulfonyl-4-(4-sulfamoylphenoxy)benzoylguanidin Hydrochlorid

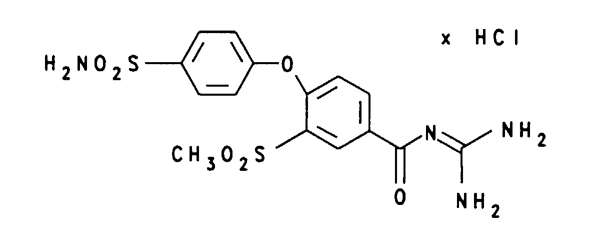

erhält man analog der in Variante A beschriebenen Vorschrift aus 3-Methylsulfonyl-4-(4-sulfamoylphenoxy)benzoesäure in Dimethylacetamid als Reaktionsmedium. Farbloser kristalliner Feststoff,
Zersetzungspunkt: 267°C.
a) 3-Methylsulfonyl-4-(4-chlorsulfonylphenoxy)benzoesäure erhält man durch Umsetzung von 0,03 Mol 4-Phenoxy-3-methylsulfonylbenzoesäure mit 0,15 Mol Chlorsulfonsäure in 60 ml Methylenchlorid bei 0 - 5°C und weiterem Rühren für 4 Stunden bei Raumtemperatur. Nach Behandlung mit Eiswasser trennt man die Methylenchloridphase ab, wäscht mit Wasser, trocknet über Magnesiumsulfat und destilliert das Lösungsmittel ab. Kristalle,
   Fp.: 167 - 170°C.
b) 3-Methylsulfonyl-4-(4-sulfamoylphenoxy)benzoesäure
   erhält man durch Reaktion von 3-Methylsulfonyl-4-(4-chlorsulfonylphenoxy)benzoesäure mit wäßrig konzentrierter Ammoniaklösung für 24 Stunden bei Raumtemperatur, abdestillieren des Ammoniaks und Ansäuern der wäßrigen Lösung mit 2N HCI auf pH 1 - 2. Abfiltrieren der Kristalle, mit Wasser waschen und trocknen. Farblose Kristalline Substanz,
   Fp 240 - 245 °C.

### Beispiel 34: 4-[4-(1-Hydroxy-2-(2-pyridylthio)ethyl)phenoxy]-3-methylsulfonylbenzoyl-guanidin Dihydrochlorid

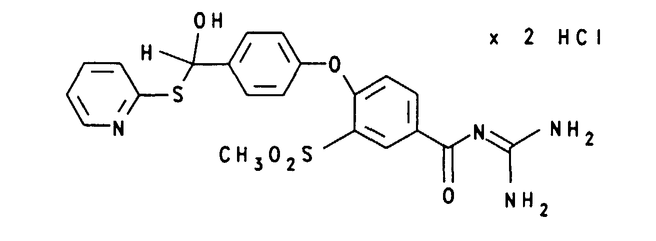

erhält man analog der in Variante A beschriebenen Vorschrift aus 4-[4-(1-Hydroxy-2-(2-pyridylthio)ethyl)phenoxy]-3-methylsulfonylbenzoesäure in THF als Reaktionsmedium. Farbloser kristalliner Feststoff,
Fp.: 116°C.
a) 4-[4-(1-Hydroxy-2-(2-pyridylthio)ethyl)phenoxy]-3-methylsulfonylbenzoesäure erhält man analog Beispiel 32 a). Farblose kristalline Substanz.
   Fp.: 169-174°C.

### Beispiel 35: 4-[4-(1-Hydroxy-2-(2-benzimidazolylthio)ethyl)phenoxy]-3-methylsulfonylbenzoyl-guanidin Dihydrochlorid

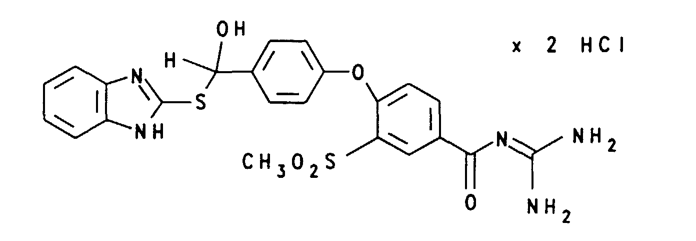

erhält man analog der in Variante A beschriebenen Vorschrift aus 4-[4-(1-Hydroxy-2-(2-benzimidazolylthio)ethyl)phenoxy]-3-methylsulfonylbenzoesäure in Dimethylacetamid als Reaktionsmedium. Farbloser kristalliner Feststoff,
Fp.: 213°C.
a) 4-[4-(1-Hydroxy-2-(2-benzimidazolylthio)ethyl)phenoxy]-3-methylsulfonylbenzoesäure
   erhält man analog Beispiel 32 a). Farblose kristalline Substanz.
   Fp.: 186-188°C.

### Beispiel 36: 4-[4-(1-Hydroxy-2-(2-chinolylthio)ethyl)phenoxy]-3-methylsulfonylbenzoyl-guanidin Dihydrochlorid

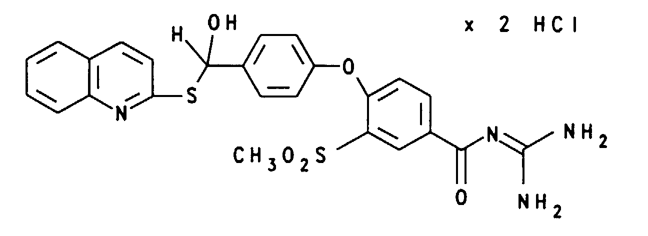

erhält man analog der in Variante A beschriebenen Vorschrift aus 4-[4-(1-Hydroxy-2-(2-chinolylthio)ethyl)phenoxy]-3-methylsulfonylbenzoesäure in THF als Reaktionsmedium. Farbloser kristalliner Feststoff,
Fp.: 180°C.
a) 4-[4-(1-Hydroxy-2-(2-chinolylthio)ethyl)phenoxy]-3-methylsufonyl-benzoesäure erhält man analog Beispiel 32 a) aus 4-[4-(2-Chinolylthio-acetyl)phenoxy]-3-methylsulfonyl-benzoesäure (Beispiel 31 a).
   Farblose kristalline Substanz.

### Beispiel 37: 4-[4-(N, N-Dimethylglycylamino)phenoxy]-3-methylsulfonylbenzoylguanidin Dihydrochlorid

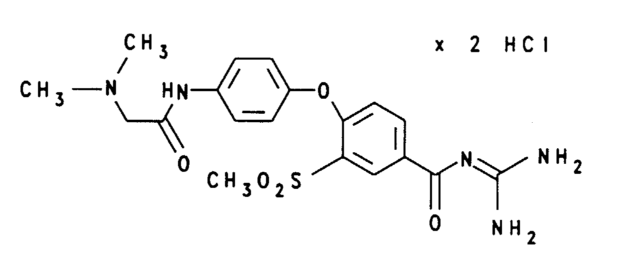

erhält man analog der in Variante A beschriebenen Vorschrift aus 4-[4-(N,N-Dimethylglycylamino)phenoxy]-3-methylsulfonylbenzoesäure in THF als Reaktionsmedium. Farbloser kristalliner Feststoff,
Fp.: 223°C.
a) Darstellung von 3-Methylsulfonyl-4-(4-nitrophenoxy)benzoesäure erfolgt durch Umsetzung von 0,02 Mol 3-Methylsulfonyl-4-phenoxybenzoesäure mit 0,96 ml 100%ige Salpetersäure in einer Mischung von 8 ml Acetanhydrid und 4 ml Eisessig bei -10°C. Nach 1,5 Stunden Rührung bei dieser Temperatur rührt man 24 Stunden bei Raumtemperatur und weitere 6 Stunden bei 40°C. Man gießt auf Eiswasser, und bringt das amorphe ölige Produkt durch Rühren unter Wasser zur Kristallisation. Gelbe Kristalline Substanz,
   Fp.: 140-150°C.
b) 4-(4-Aminophenoxy)-3-methylsulfonylbenzoesäure
   erhält man durch katalytische Hydrierung von 3-Methylsulfonyl-4-(4-nitrophenoxy) benzoesäure (Beispiel 37 a) mit Raney Nickel in Methanol unter Normaldruck bis zur vollständigen Wasserstoffaufnahme. Man erhält nach Verdampfen des Lösungsmittels ein einheitliches amorphes Öl.
c) N,N-Dimethylglycin-imidazolid Hydrochlorid
   erhält man durch Umsetzung von N,N-Dimethylglycin Hydrochlorid mit Carbonyldiimidazol in wasserfreiem Dimethylacetamid, aus der sich das Produkt abscheidet. Das Gemisch wird ohne weitere Aufarbeitungsschritte weiter umgesetzt.
d) 4-[4-(N, N-Dimethylglycylamino)phenoxy]-3-methylsulfonylbenzoesäure
   erhält man durch Umsetzung von N,N-Dimethylglycin-imidazolid Hydrochlorid (Beispiel 37, c) mit 4-(4-Aminophenoxy)-3-methylsulfonyl benzoesäure (Beispiel 37, b) durch Rühren über 5 Stunden in Dimethylacetamid bei Raumtemperatur. Man destilliert das Lösungsmittel ab, stellt mit HCI auf pH 1-2 und extrahiert mit Ethylacetat. Die wäßrige Phase wird abdestilliert und der Rückstand mit Methanol behandelt. Nach Abfiltrieren des unlöslichen Anteils erhält man die gewünschte Verbindung als farbloses Öl.

### Beispiel 38: 4-[4-(N,N-Diethylaminoethyl)aminosulfonylphenoxy]-3-methylsulfonylbenzoyl-guanidin Dihydrochlorid

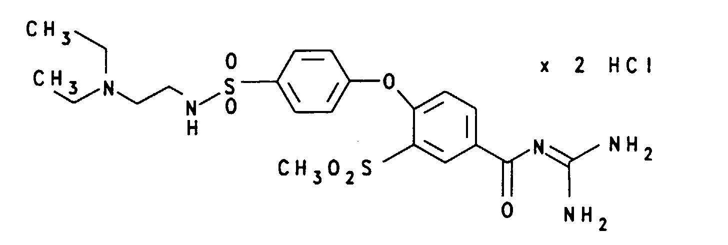

erhält man analog der in Variante A beschriebenen Vorschrift aus 4-[4-(N,N-Diethylaminoethyl)aminosulfonylphenoxy]-3-methylsulfonylbenzoesäure in wasserfreiem Dimethylacetamid als Reaktionsmedium. Farbloser kristalliner Feststoff,
Fp.: 206°C.
a) 4-[4-(N,N-Diethylaminoethyl)aminosulfonylphenoxy]-3-methylsulfonylbenzoesäure
   erhält man durch Reaktion von 0,05 Mol 3-Methylsulfonyl-4-(4-chlorsulfonylphenoxy)benzoesäure (Beispiel 33 a) mit 0,06 Mol N,N-Diethylaminoethylamin in Gegenwart von überschüssigem Triethylamin (ca. 0,015 Mol) in Methanol als Reaktionsmedium. Nach Abdestillieren des Lösungsmittelgemisches versetzt man mit wenig Wasser und stellt auf pH 4 - 5. Abfiltrieren des kristallinen Niederschlags.
   Fp.: 263 - 268°C.

### Beispiel 39: 4-[4-(1-Methyl-4-piperazino)sulfonylphenoxy]-3-methylsulfonyl-benzoylguanidin Dihydrochlorid

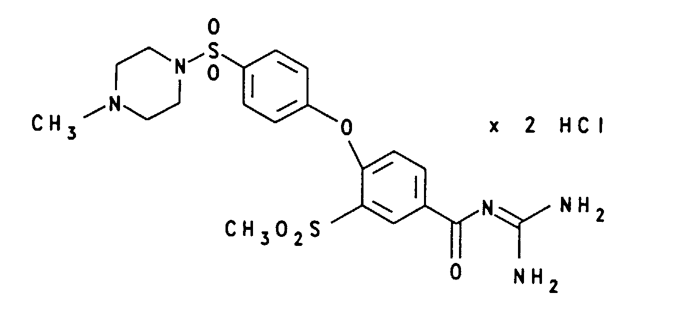

erhält man analog der in Variante A beschriebenen Vorschrift aus 4-[4-(1-Methyl-4-piperazino)sulfonylphenoxy]-3-methylsulfonylbenzoesäure in THF als Reaktionsmedium. Farbloser kristalliner Feststoff,
Zers.: 234-244°C.
a) 4-[4-(1-Methyl-4-piperazino)sulfonylphenoxy]-3-methylsulfonylbenzoesäure erhält man durch Umsetzung von Methylsulfonyl-4-(4-chlorsulfonylphenoxy)benzoesäure (Beispiel 33 a) mit N-Methylpiperazin analog Beispiel 38 a). Farblose Kristalle,
   Zers.: 287 - 292°C.

### Beispiel 40: 4-[4-(4-Imidazolylethyl)aminosulfonyl-phenoxy]-3-methylsulfonylbenzoyl-guanidin Dihydrochlorid

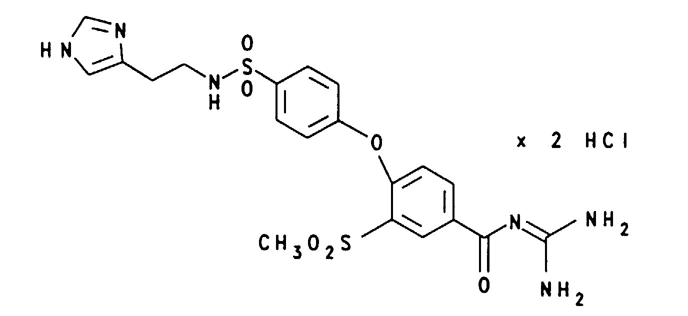

erhält man analog der in Variante A beschriebenen Vorschrift aus 4-[4-(4-lmidazolylethyl)aminosulfonylphenoxy]-3-methylsulfonylbenzoesäure in Dimethylacetamid als Reaktionsmedium. Farbloser kristalliner Feststoff,
Fp.: 264°C.
a) 4-[4-(4-Imidazolylethyl)aminosulfonylphenoxy]-3-methylsulfonylbenzoesäure erhält man analog Beispiel 38 a) durch Umsetzung von Methylsulfonyl-4-(4-chlorsulfonylphenoxy)benzoesäure (Beispiel 33 a) mit Histamin. Farbloser, amorpher Feststoff.
   Zers. 265°C.

### Beispiel 41: 4-[4-(3-N-lmidazolyl-1-propyl)aminosulfonyl-phenoxy]-3-methylsulfonylbenzoyl-guanidin Dihydrochlorid

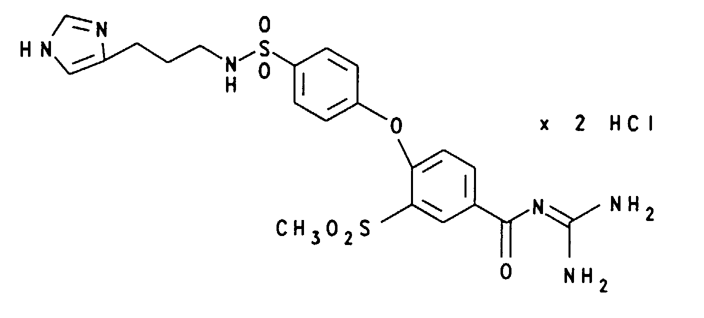

erhält man analog der in Variante A beschriebenen Vorschrift aus 4-[4-(3-N-Imidazolyl-1-propyl)aminosulfonylphenoxy]-3-methylsulfonylbenzoesäure in THF als Reaktionsmedium. Farbloser kristalliner Feststoff,
Zers.: 257°C.
a) 4-[4-(3-N-lmidazolyl-1-propyl)aminosulfonylphenoxy]-3-methylsulfonylbenzoesäure
   erhält man analog Beispiel 38 a) durch Umsetzung von Methylsulfonyl-4-(4-chlorsulfonylphenoxy)benzoesäure (Beispiel 33 a) mit 1-(3-Aminopropyl)imidazol. Farbloser amorpher Feststoff,
   Fp.: 250°C.

### Beispiel 42: 4-[4-(1-Methyl-2-pyrrolidinylethyl)aminosulfonyl-phenoxy]-3-methylsulfonylbenzoyl-guanidin Dihydrochlorid

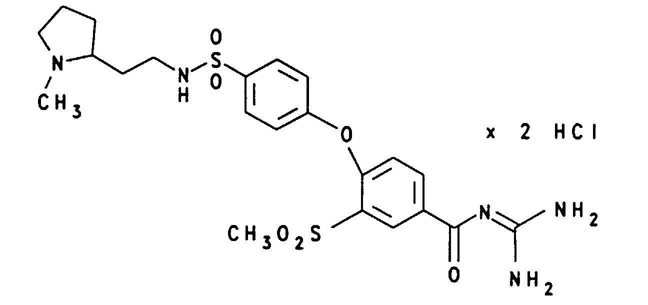

erhält man analog der in Variante A beschriebenen Vorschrift aus 4-[4-(1-Methyl-2-pyrrolidinylethyl)aminosulfonylphenoxy]-3-methylsulfonylbenzoesäure in Dimethylacetamid als Reaktionsmedium. Farbloser kristalliner Feststoff,
Fp.: 254°C.
a) 4-[4-(1-Methyl-2-pyrrolidinylethyl)aminosulfonylphenoxy]-3-methylsulfonylbenzoesäure
   erhält man analog Beispiel 38 a) durch Umsetzung von Methylsulfonyl-4-(4-chlorsulfonylphenoxy)benzoesäure (Beispiel 33 a) mit 2-(2-Aminoethyl)-1-methylpyrrolidin.
   Fp. 242 - 247°C.

### Beispiel 43: 4-[4-(N-Morpholinoethyl)aminosulfonyl-phenoxy]-3-methylsulfonylbenzoyl-guanidin Dihydrochlorid

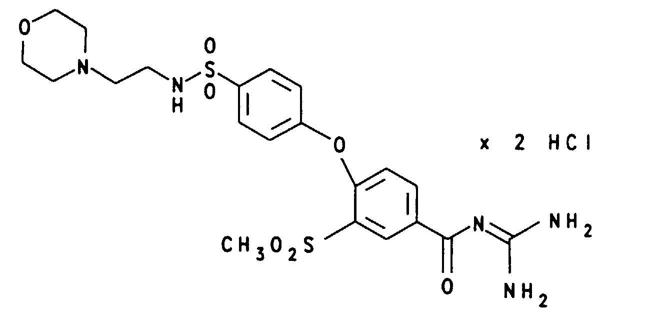

erhält man analog der in Variante A beschriebenen Vorschrift aus 4-[4-(N-Morpholinoethyl)aminosulfonylphenoxy]-3-methylsulfonylbenzoesäure in Dimethylacetamid als Reaktionsmedium. Farbloser kristalliner Feststoff,
Fp.: 272°C.
a) 4-[4-(N-Morpholinoethyl)aminosulfonylphenoxy]-3-methylsulfonylbenzoesäure erhält man analog Beispiel 38 a) durch Umsetzung von Methylsulfonyl-4-(4-chlorsulfonylphenoxy)benzoesäure (Beispiel 33 a) mit N-(2-Aminoethyl)morpholin. Farblose kristalline Verbindung,
   Fp.: 220-224°C.

### Beispiel 44: 4-[4-(N-Piperidinoethyl)aminosulfonyl-phenoxy]-3-methylsulfonylbenzoyl-guanidin Dihydrochlorid

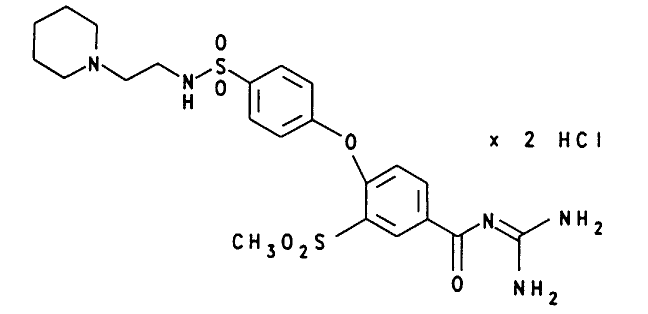

erhält man analog der in Variante A beschriebenen Vorschrift aus 4-[4-(N-Piperidinoethyl)aminosulfonylphenoxy]-3-methylsulfonylbenzoesäure in wasserfreiem Dimethylacetamid als Reaktionsmedium. Farbloser kristalliner Feststoff,
Fp.: 266°C.
a) 4-[4-(N-Piperidinoethyl)aminosulfonylphenoxy]-3-methylsulfonylbenzoesäure erhält man analog Beispiel 38 a) durch Umsetzung von Methylsulfonyl-4-(4-chlorsulfonylphenoxy)benzoesäure (Beispiel 33 a) mit N-(2-Aminoethyl)piperidin. Farblose kristalline Substanz,
   Fp.: 271-273°C.

### Beispiel 45: 4-[4-(2-Pyridylethyl)aminosulfonyl-phenoxy]-3-methylsulfonyl-benzoylguanidin Dihydrochlorid

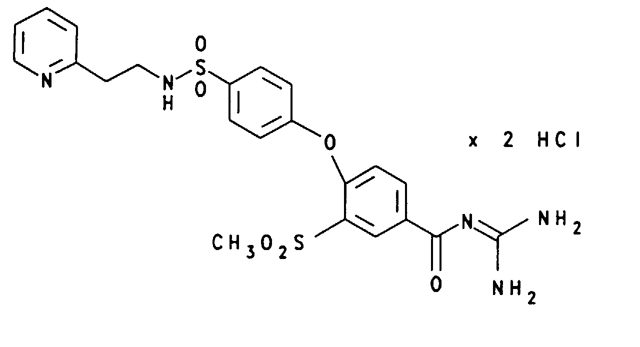

erhält man analog der in Variante A beschriebenen Vorschrift aus 4-[4-(2-Pyridylethyl)aminosulfonylphenoxy]-3-methylsulfonylbenzoesäure in wasserfreiem Dimethylacetamid als Reaktionsmedium. Farbloser kristalliner Feststoff, Fp.: 257°C.
a) 4-[4-(2-Pyridylethyl)aminosulfonylphenoxy]-3-methylsulfonylbenzoesäure erhält man analog Beispiel 38 a) durch Umsetzung von Methylsulfonyl-4-(4-chlorsulfonylphenoxy)benzoesäure (Beispiel 33 a) mit 2-(Aminoethyl)pyridin. Farblose Kristalle.
   Fp. 268-270°C.

### Beispiel 46: 4-[4-(2-Dimethylaminoethyl)sulfonylmethyl-phenoxy]-3-methylsulfonylbenzoyl-guanidin Dihydrochlorid

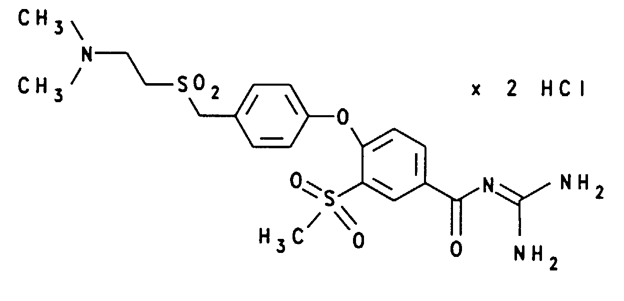

erhält man durch Persäureoxidation analog der in Beispiel "4" beschriebenen Vorschrift aus 4-[4-(2-Dimethylaminoethyl)thiomethyl-phenoxy]-3-methylsulfonylbenzoyl-guanidin Dihydrochlorid (Beispiel 15). Farblose Kristalle.
Fp. 245°C

### Beispiel 47: 4-[4-(2-Dimethylaminoethyl)sulfonylmethyl-phenoxy]-3-trifluormethylbenzoyl-guanidin Dihydrochlorid

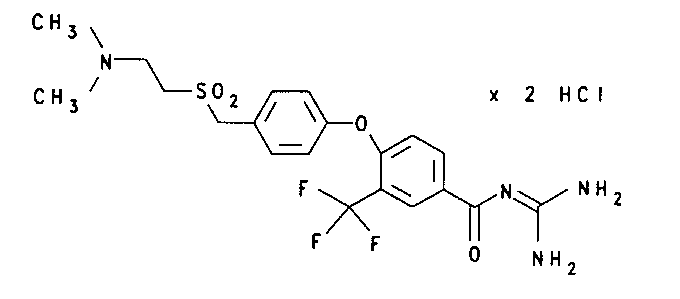

erhält man durch Persäureoxidation analog der in Beispiel 17 beschriebenen Vorschrift aus 4-[4-(2-Dimethylaminoethyl)thiomethyl-phenoxy]-3-trifluormethylbenzoyl-guanidin Dihydrochlorid (Beispiel 19).
Farblose Kristalle. Fp. 140°C.

### Pharmakologische Daten:

### Inhibition des Na⁺/H⁺-Exchangers von Kaninchenerythrocyten

Weiße Neuseeland-Kaninchen (Ivanovas) erhielten eine Standard-Diät mit 2 % Cholesterin für sechs Wochen, um den Na⁺/H⁺-Austausch zu aktivieren und so den Na⁺-Influx in die Erythrocyten via Na⁺/H⁺-Austausch flammenphotometrisch bestimmen zu können. Das Blut wurde den Ohrarterien entnommen und durch 25 IE Kalium-Heparin ungerinnbar gemacht. Ein Teil jeder Probe wurde zur Doppelbestimmung des Hämatokrits durch Zentrifugieren benutzt. Aliquots von jeweils 100 µl dienten zur Messung des Na⁺-Ausgangsgehalts der Erythrocyten.

Um den Amilorid-sensitiven Natrium-lnflux zu bestimmen, wurden 100 µl jeder Blutprobe in jeweils 5 ml eines hyperosmolaren Salz-Sucrose-Mediums (mmol/l: 140 NaCl, 3 KCI, 150 Sucrose, 0,1 Ouabain, 20 Tris-hydroxymethyl-aminomethan) bei pH 7,4 und 37°C inkubiert. Die Erythrocyten wurden danach dreimal mit eiskalter MgCl₂-Ouabain-Lösung (mmol/l: 112 MgCl₂, 0,1 Ouabain) gewaschen und in 2,0 ml destilliertem Wasser hämolysiert. Der intrazelluläre Natriumgehalt wurde flammenphotometrisch bestimmt.

Der Na⁺-Nettoinflux wurde aus der Differenz zwischen Natrium-Ausgangswerten und dem Natriumgehalt der Erythrocyten nach Inkubation errechnet. Der Amiloridhemmbare Natrium-lnflux ergab sich aus der Differenz des Natriumgehalts der Erythrocyten nach Inkubation mit und ohne Amilorid 3 x 10-4 mol/l. Auf diese Weise wurde auch bei den erfindungsgemäßen Verbindungen verfahren.

### Ergebnisse

### Inhibition des Na⁺/H⁺-Exchangers:

| Beispiel | IC50 (µmol/l) |
|---|---|
| 1 | 0.8 |
| 2 | 0.32 |
| 3 | 0.05 |
| 4 | 0.082 |
| 5 | 0.3 |
| 6 | 0.1 |
| 7 | 0.06 |
| 8 | 0.005 |
| 9 | 0.1 |
| 14 | 0.05 |
| 15 | 0.01 |
| 16 | 0.16 |
| 17 | 1 |
| 18 | 0.2 |
| 19 | 0.008 |
| 20 | 0.04 |
| 21 | 0.014 |
| 28 | 0.12 |
| 32 | 0.44 |
| 33 | 0.20 |
| 46 | 0.09 |
| 47 | 0.07 |

## Patentansprüche

1. Verbindungen der Formel I 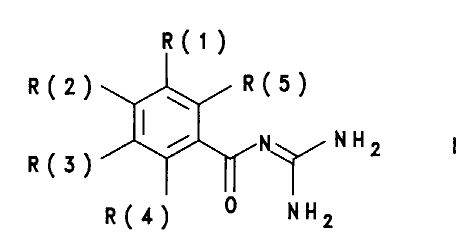 worin bedeuten:
einer der drei Substituenten R(1), R(2) und R(3)
R(6)-A-B-D-;
R(6) ein basischer protonierbarer Rest, d.h. eine Aminogruppe -NR(7)R(8), eine Amidinogruppe R(7)R(8)N-C[=N-R(9)]- oder eine Guanidinogruppe 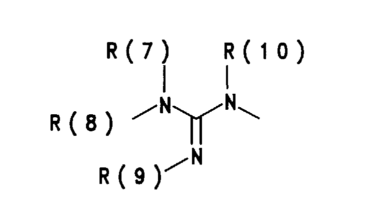
R(7), R(8), R(9) und R(10) unabhängig voneinander Wasserstoff oder Alkyl mit 1, 2, 3 oder 4 C-Atomen;
oder
R(7) und R(8) gemeinsam CₐH₂ₐ;
a 4, 5, 6 oder 7;
wobei im Falle von a = 5, 6 oder 7 eine Methylengruppe der Gruppe CₐH₂ₐ durch eine Heteroatomgruppe O, SOₘ oder NR(11) ersetzt sein kann,
oder
R(8) und R(9) oder R(9) und R(10) oder R(7) und R(10) eine Gruppe CₐH₂ₐ;
a 2, 3, 4 oder 5;
wobei im Falle von a = 3, 4 oder 5 eine Methylengruppe der Gruppe CₐH₂ₐ durch eine Heteroatomgruppe O, SOₘ oder NR(11) ersetzt sein kann;
m Null, 1 oder 2;
R(11) Wasserstoff oder Methyl;
oder
R(6) ein basisches heteroaromatisches Ringsystem mit 1 - 9 C-Atomen;
A C_{b}H_{2b};
b 1, 2, 3, 4, 5, 6, 7, 8, 9 oder 10;
wobei in der Gruppe C_{b}H_{2b} ein oder zwei Methylengruppen durch eine der Gruppierungen ausgewählt aus der Gruppe bestehend aus -O-,-CO-, -CH[OR(20)]-, -SOₘ-, -NR(20)-, -NR(20)-CO-, -NR(20)-CO-NH-, -NR(20)-CO-NH-SO₂- 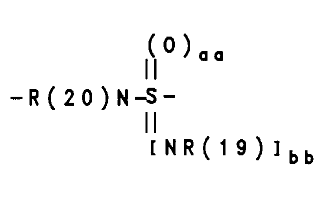 und -SOₐₐ[NR(19)]_{bb}- ersetzt sein können;
und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa + bb = 2;
R(19) Wasserstoff oder Alkyl mit 1, 2, 3 oder 4 C-Atomen
R(20) Wasserstoff oder Methyl;
B einen Phenylen- oder Naphthylenrest, 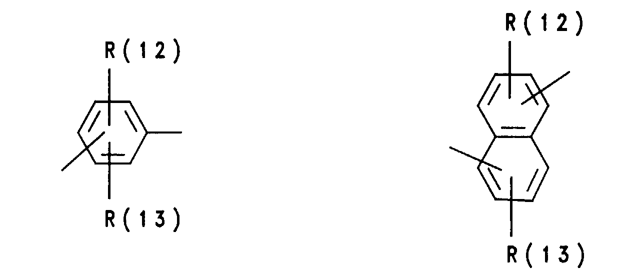
R(12) und R(13) unabhängig voneinander Wasserstoff, Methyl, F, Cl, Br, J, CF₃ oder -SO_{w}-R(14);
R(14) Methyl oder NR(15)R(16);
R(15) und R(16) unabhängig voneinander Wasserstoff oder Alkyl mit 1, 2, 3 oder 4 C-Atomen;
w Null, 1 oder 2;
D -C_{d}H_{2d}-X_{f}-;
d Null, 1, 2, 3 oder 4;
X -O-, -CO-, -CH[OR(21)]-, -SOₘ- oder -NR(21)-;
f 1;
R(21) Wasserstoff oder Methyl;
m Null, 1 oder 2;
und die jeweils anderen Substituenten R(1) und R(2) und R(3)
unabhängig voneinander Wasserstoff, F, Cl, Br, J, -CN, -(C₁-C₈)-Alkyl, -(C₂-C₈)-Alkenyl, -NR(35)R(36) oder R(17)-C_{g}H_{2g}-Zₕ-;
g Null, 1, 2, 3 oder 4;
h Null oder 1;
R(35) und R(36) unabhängig voneinander Wasserstoff oder Alkyl mit 1, 2, 3, 4, 5 oder 6 C-Atomen;
oder
R(35) und R(36) gemeinsam 4 - 7 Methylengruppen, von denen eine CH₂-Gruppe durch Sauerstoff, -S-, -NH-, -NCH₃ oder -N-Benzyl ersetzt sein kann;
Z O-, -CO-, -SOᵥ-,-NR(18)-, -NR(18)-CO-, -NR(18)-CO-NH- oder -NR(18)-SO₂-;
R(18) Wasserstoff oder Methyl;
v Null, 1 oder 2;
R(17) Wasserstoff, Cycloalkyl mit 3, 5 oder 6 C-Atomen oder CₖF₂ₖ₊₁-;
k 1, 2 oder 3,
oder
R(17) Pyrrol-1-yl, Pyrrol-2-yl oder Pyrrol-3-yl,
welches nicht substituiert oder substituiert ist mit 1 - 4 Substituenten ausgewählt aus der Gruppe bestehend aus F, Cl, Br, I, -CN, (C₂-C₈)-Alkanoyl, (C₂-C₈)-Alkoxycarbonyl, Formyl, Carboxy, -CF₃, Methyl und Methoxy;
oder
R(17) -(C₃-C₈)-Cycloalkyl oder Phenyl,
welches nicht substituiert oder substituiert ist mit 1 - 3 Substituenten ausgewählt aus der Gruppe bestehend aus F und Cl, -CF₃, Methyl, Hydroxy, Methoxy, -NR(37)R(38), CH₃SO₂- und H₂NO₂S-;
R(37) und R(38) Wasserstoff oder -CH₃;
R(4) und R(5) unabhängig voneinander Wasserstoff, Alkyl mit 1, 2, 3 oder 4 C-Atomen, F, Cl, -OR(32),-NR(33)R(34) oder -CᵣF₂ᵣ₊₁;
R(32), R(33) und R(34) unabhängig voneinander Wasserstoff oder Alkyl mit 1, 2 oder 3 C-Atomen;
r 1, 2, 3 oder 4;
sowie deren pharmakologisch verträgliche Salze.

2. Verbindungen der Formel I nach Anspruch 1, dadurch gekennzeichnet, daß darin bedeuten:
R(1) Wasserstoff, F, Cl, -(C₁-C₄)-Alkyl, -(C₂-C₄)-Alkenyl, -NR(35)R(36) oder R(17)-C_{g}H_{2g}-Zₕ-;
R(35) und R(36) unabhängig voneinander Wasserstoff, Methyl oder Ethyl;
oder
R(35) und R(36) gemeinsam 4 - 5 Methylengruppen, von denen eine CH₂-Gruppe durch Sauerstoff, -S-, -NH-, oder -NCH₃ ersetzt sein kann;
R(17) Wasserstoff, Cycloalkyl mit 5 oder 6 C-Atomen oder CₖF₂ₖ₊₁-;
k 1, 2 oder 3,
g Null, 1, 2, 3 oder 4;
h Null oder 1;
Z -O-, -CO-, -SOᵥ-,-NR(18)-, -NR(18)-CO-, -NR(18)-CO-NH- oder -NR(18)-SO₂-; R(18) Wasserstoff oder Methyl;
v Null, 1 oder 2;
oder, wenn g und h gleich null sind,
R(17) Pyrrol-1-yl, Pyrrol-2-yl oder Pyrrol-3-yl,
welches nicht substituiert oder substituiert ist mit 1 - 2 Substituenten ausgewählt aus der Gruppe bestehend aus F, Cl, (C₂-C₅)-Alkanoyl, (C₂-C₅)-Alkoxycarbonyl, Formyl, Carboxy, -CF₃, Methyl und Methoxy;
oder
R(17) -(C₃-C₈)-Cycloalkyl oder Phenyl,
welches nicht substituiert oder substituiert ist mit 1 - 2 Substituenten ausgewählt aus der Gruppe bestehend aus F und Cl, -CF₃, Methyl, Methoxy, -NR(37)R(38), CH₃SO₂- und H₂NO₂S-;
R(37) und R(38) unabhängig voneinander Wasserstoff oder -CH₃;
einer der Substituenten R(2) und R(3)
R(6)-A-B-D-;
R(6) -NR(7)R(8), eine Amidinogruppe R(7)R(8)N-C[=N-R(9)]- oder eine Guanidinogruppe 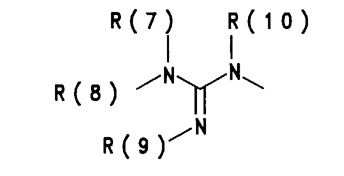
R(7), R(8), R(9) und R(10) unabhängig voneinander Wasserstoff oder Alkyl mit 1, 2, 3 oder 4 C-Atomen;
oder
R(7) und R(8) gemeinsam CₐH₂ₐ;
a 4, 5, 6 oder 7;
wobei im Falle von a = 5, 6 oder 7 eine Methylengruppe der Gruppe CₐH₂ₐ durch eine Heteroatomgruppe O, SOₘ oder
NR(11) ersetzt sein kann;
R(11) Wasserstoff oder Methyl;
oder
R(8) und R(9) gemeinsam CₐH₂ₐ;
a 2, 3, 4 oder 5;
wobei im Falle von a = 3, 4 oder 5 eine Methylengruppe der Gruppe CₐH₂ₐ durch eine Heteroatomgruppe O, SOₘ oder NR(11) ersetzt sein kann;
m Null, 1 oder 2;
oder
R(6) Imidazolyl-, Pyridyl-, Chinolinyl oder Isochinolinyl;
A C_{b}H_{2b};
b 1, 2, 3, 4 oder 5,
wobei in der Gruppe C_{b}H_{2b} eine oder zwei Methylengruppen durch eine der Gruppierungen ausgewählt aus der Gruppe bestehend aus -O-,-CO-, -CH[OR(20)]-, -SOₘ-, NR(20), -NR(20)-CO-, -NR(20)-CO-NH-, -NR(20)-CO-NH-SO₂-, und 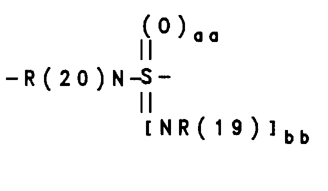 -Soₐₐ[NR(19)]_{bb}- ersetzt sein können;
und wobei in der Gruppe C_{b}H_{2b} eine Methylengruppe durch -CH-R(99) ersetzt sein kann, wobei R(99) gemeinsam mit R(7) einen Pyrrolidin- oder Piperidinring bildet;
aa 1 oder 2;
bb 0 oder 1;
aa + bb = 2;
R(19) Wasserstoff oder Alkyl mit 1, 2, 3 oder 4 C-Atomen
R(20) Wasserstoff oder Methyl; einen Phenylen- oder Napthtylenrest,
B einen Phenylen- oder 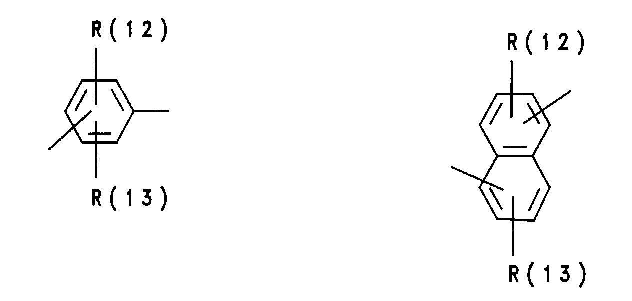
R(12) und R(13) unabhängig voneinander Wasserstoff, Methyl, F, Cl, CF₃ oder -SO₂-R(14);
R(14) Methyl oder NR(15)R(16);
R(15) und R(16) unabhängig voneinander Wasserstoff oder Alkyl mit 1, 2, 3 oder 4 C-Atomen;
D -C_{d}H_{2d}-X_{f}-;
d Null, 1, 2, 3 oder 4;
X -O-, -CO-, -CH[OR(21)]-, -SOₘ- oder -NR(21)-;
f 1;
R(21) Wasserstoff oder Methyl;
m Null, 1 oder 2;
und der jeweils andere Rest R(2) und R(3)
Wasserstoff, Alkyl mit 1, 2, 3 oder 4 C-Atomen, F, Cl oder CF₃;
R(4) und R(5) unabhängig voneinander Wasserstoff, Alkyl mit 1, 2 oder 3 C-Atomen, F, Cl, oder -CF₃.

3. Verbindungen der Formel I nach Ansprüchen 1 oder 2, dadurch gekennzeichnet, daß darin bedeuten:
R(1) Wasserstoff, F, Cl, Alkyl mit 1, 2, 3 oder 4 C-Atomen, -NR(35)R(36) oder R(17)-C_{g}H_{2g}-Zₕ-;
g Null, 1, 2, 3 oder 4;
h Null oder 1;
Z -O-, -CO-, -SOᵥ- ,-NR(18)-, -NR(18)-CO-, -NR(18)-CO-NH-, oder -NR(18)-SO₂-;
R(18) Wasserstoff oder Methyl;
v Null, 1 oder 2;
R(17) Wasserstoff, Cycloalkyl mit 5 oder 6 C-Atomen oder CF₃-;
oder, wenn g und h gleich null sind,
R(17) Pyrrol-1-yl,
welches nicht substituiert oder substituiert ist mit 1 - 2 Substituenten ausgewählt aus der Gruppe bestehend aus F, Cl, (C₂-C₅)-Alkanoyl, (C₂-C₅)-Alkoxycarbonyl, -CF₃ und Methyl;
oder
R(17) -(C₅-C₆)-Cycloalkyl oder Phenyl,
welches nicht substituiert oder substituiert ist mit einem Substituenten ausgewählt aus der Gruppe bestehend aus F und Cl, -CF₃, Methyl, CH₃SO₂- und H₂NO₂S-;
R(35) und R(36) unabhängig voneinander Wasserstoff, Methyl oder Ethyl;
oder
R(35) und R(36) gemeinsam 4 - 5 Methylengruppen, von denen eine CH₂-Gruppe durch Sauerstoff, -S-, -NH-, oder -NCH₃ ersetzt sein kann;
einer der Substituenten R(2) und R(3)
R(6)-A-B-D-;
R(6) -NR(7)R(8), eine Amidinogruppe R(7)R(8)N-C[=N-R(9)]- oder eine Guanidinogruppe 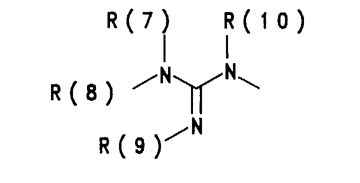
R(7) Wasserstoff oder Alkyl mit 1, 2, 3 oder 4 C-Atomen;
R(8), R(9) und R(10) unabhängig voneinander Wasserstoff, Methyl oder Ethyl;
oder
R(7) und R(8) gemeinsam CₐH₂ₐ;
a 4 oder 5;
wobei im Falle von a = 5 eine Methylengruppe der Gruppe CₐH₂ₐ durch NR(11) ersetzt sein kann,
R(11) Wasserstoff oder Methyl;
oder
R(6) Imidazolyl- oder Pyridyl;
A C_{b}H_{2b};
b 1, 2, 3, 4 oder 5,
wobei in der Gruppe C_{b}H_{2b} ein oder zwei Methylengruppen durch eine der Gruppierungen ausgewählt aus der Gruppe bestehend aus -CO-, -CH[OR(20)]-, -NR(20)-CO-, 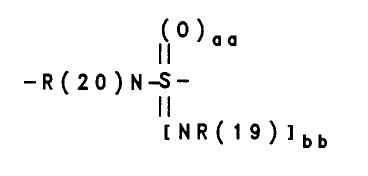 -Soₐₐ[NR(19)]_{bb} und -SO₂- ersetzt sein können;
und wobei in der Gruppe C_{2b}H_{2b} eine Methylengruppe durch -CH-R(99) ersetzt sein kann, wobei R(99) gemeinsam mit R(7) einen Pyrrolidin- oder Piperidinring bildet;
aa 1 oder 2;
bb 0 oder 1;
aa + bb = 2;
R(19) Wasserstoff oder Alkyl mit 1, 2, 3 oder 4 C-Atomen
R(20) Wasserstoff oder Methyl;
oder, wenn b gleich 2, 3, 4 oder 5 bedeutet
ein C-Atom in C_{b}H_{2b} durch eine Gruppierung -O-, -S-,-NR(20)-, -NR(20)-CO- oder -NR(20)-CO-NH- ersetzt sein kann;
B einen Phenylenrest, 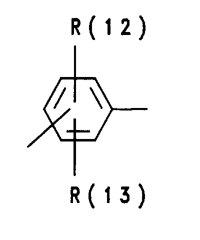
R(12) und R(13) unabhängig voneinander Wasserstoff, Methyl, F, Cl, CF₃ oder -SO₂R(14);
R(14) Methyl oder NH₂;
D -CH₂-, -O-, -CO-, -SOₘ- oder -NR(21)-;
m Null oder 2;
R(21) Wasserstoff oder Methyl;
und der jeweils andere Rest R(2) und R(3)
Wasserstoff;
R(4) und R(5) unabhängig voneinander Wasserstoff, Alkyl mit 1, 2 oder 3 C-Atomen, F, Cl, oder -CF₃.

4. Verbindungen der Formel I nach mindestens einem der Ansprücheuch 1 - 3, dadurch gekennzeichnet, daß darin bedeuten:
R(1) Wasserstoff, F, Cl, Alkyl mit 1, 2, 3 oder 4 C-Atomen, -NR(35)R(36) oder R(17)-C_{g}H_{2g}-Zₕ-;
g Null oder 1;
h Null oder 1;
Z -O-, -CO-, -NR(18)-CO-, -NR(18)-CO-NH-, oder -NR(18)-SO₂-;
R(18) Wasserstoff oder Methyl;
oder, wenn g gleich 1;
Z -SO₂-;
R(17) Wasserstoff oder CF₃-;
R(35) und R(36) unabhängig voneinander Wasserstoff, Methyl oder Ethyl;
oder
R(35) und R(36) gemeinsam 4 - 5 Methylengruppen, von denen eine CH₂-Gruppe durch Sauerstoff, -S-, -NH-, oder -NCH₃ ersetzt sein kann;
einer der Substituenten R(2) und R(3)
R(6) -A-B-O-;
R(6) -NR(7)R(8) oder eine Guanidinogruppe 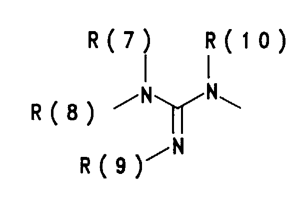
R(7) Wasserstoff oder Alkyl mit 1, 2, 3 oder 4 C-Atomen;
R(8), R(9) und R(10) unabhängig voneinander Wasserstoff, Methyl oder Ethyl;
oder
R(7) und R(8) gemeinsam CₐH₂ₐ;
a 4 oder 5;
wobei im Falle von a = 5 eine Methylengruppe der Gruppe CₐH₂ₐ durch-NH- oder -NCH₃- ersetzt sein kann,
oder
R(6) Imidazolyl-;
A C_{b}H_{2b};
b 1, 2, 3 oder 4;
wobei in der Gruppe C_{b}H_{2b} eine oder zwei Methylengruppen durch eine der Gruppierungen ausgewählt aus der Gruppe bestehend aus -CO-, 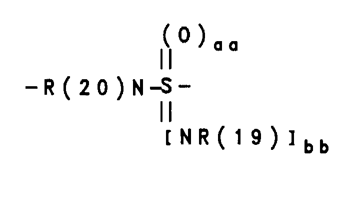 -SOₐₐ[NR(19)]_{bb}- und -SO₂- ersetzt sein können,
und wobei in der Gruppe C_{b}H_{2b} eine Methylengruppe durch -CH-R(99) ersetzt sein kann, wobei R(99) gemeinsam mit R(7) einen Pyrrolidin- oder Piperidin-Ring bilden kann;
oder, wenn b gleich 2, 3 oder 4 bedeutet,
eine Methylengruppe in der Gruppe C_{b}H_{2b} durch eine Gruppierung -O-,-S- ersetzt sein kann;
aa 1 oder 2;
bb 0 oder 1;
aa + bb = 2;
R(19) Wasserstoff oder Alkyl mit 1, 2, 3 oder 4 C-Atomen
R(20) Wasserstoff oder Methyl;
B einen Phenylenrest, 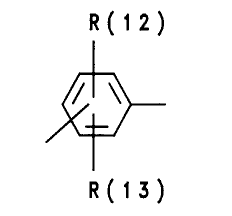
R(12) und R(13) Wasserstoff;
und der jeweils andere Rest R(2) und R(3)
Wasserstoff;
R(4) und R(5) Wasserstoff.

5. Verbindung der Formel I nach Anspruch 1, dadurch gekennzeichnet, daß sie ausgewählt ist aus der Gruppe bestehend aus 4-[4-N-(Dimethylaminoethyl)-methylsulfamoyl]phenoxy-3-trifluormethylbenzoylguanidin, Dihydrochlorid; 4-[4-(4-Methylpiperazinosulfonyl)phenoxy]-3-trifluormethyl-benzoylguanidin, Dihydrochlorid; 4-[4-(2-Pyrolidinethylaminosulfonyl)phenoxy]-3-trifluormethyl-benzoylguanidin, Dimaleinat; 4-[4-(2-Piperidinethylaminosulfonyl)phenoxy]-3-trifluormethyl-benzoylguanidin, Dimaleinat; 4-[4-(N-Dimethylamino-n-propyl)sulfamoyl]phenoxy-3-trifluormethyl-benzoylguanidin; 4-[4-(N-Dimethylaminoethyl)sulfamoyl]phenoxy-3-trifluormethyl-benzoylguanidin; 4-(4-Imidamidosulfonyl)phenoxy-3-trifluormethyl-benzoylguanidin; 3-Trifluormethyl-4-(4-N-methylimidamidosulfonyl)phenoxy-benzoylguanidin; 3-Methyl4-(4-(1-methylpiperazin-4-ylsulfonyl)phenoxy)-benzoylguanidin; 4-(4-Guanidinosulfonyl)phenoxy-3-trifluormethyl-benzoylguanidin; 4-[4-(2-lmidazolylthio-acetyl)phenoxy]-3-methylsulfonyl-benzoyl-guanidin Dihydrochlorid; 4-[4-(N, N'-Dimethyl-S-isothiuronyl-acetyl)phenoxy]-3-methylsulfonyl-benzoylguanidin Dihydrochlorid; 4-[4-(2-Benzimidazolylthio-acetyl)phenoxy]-3-methylsulfonyl-benzoylguanidin Dihydrochlorid; 4-[4-(2-N-lmidazolyl-1-hydroxyethyl)phenoxy]-3-methylsulfonyl-benzoylguanidin Dihydrochlorid; 4-[4-(N,N-Dimethylglycylamino)phenoxy]-3-methylsulfonylbenzoyl-guanidin Dihydrochlorid; 4-[4-(N,N-Diethylaminoethyl)aminosulfonylphenoxy]-3-methylsulfonylbenzoyl-guanidin Dihydrochlorid; 4-[4-(4-lmidazolylethyl)aminosulfonyl-phenoxy]-3-methylsulfonyl-benzoyl-guanidin Dihydrochlorid; 4-[4-(3-N-Imidazolyl-1-propyl)aminosulfonyl-phenoxy]-3-methylsulfonylbenzoyl-guanidin Dihydrochlorid; 4-[4-(1-Methyl-2-pyrrolidinylethyl)aminosulfonyl-phenoxy]-3-methylsulfonylbenzoyl-guanidin Dihydrochlorid; 4-[4-(N-Piperidinoethyl)aminosulfonyl-phenoxy]-3-methylsulfonyl-benzoyl-guanidin Dihydrochlorid; 4-[4-(2-Dimethylaminoethyl)sulfonylmethyl-phenoxy]-3-methylsulfonylbenzoyl-guanidin Dihydrochlorid; 4-[4-(2-Dimethylaminoethyl)sulfonylmethyl-phenoxy]-3-trifluormethylbenzoyl-guanidin Dihydrochlorid.

6. Verfahren zur Herstellung einer Verbindung I nach Anspruch 1, dadurch gekennzeichnet, daß man eine Verbindung der Formel II 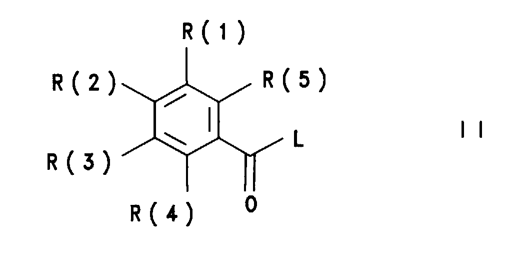 worin R(1) bis R(5) die angegebene Bedeutung besitzen und L für eine leicht nucleophil substituierbare leaving group steht,
mit Guanidin umsetzt,
und daß man gegebenenfalls in ein pharmakologisch verträgliches Salz überführt.

7. Verwendung einer Verbindung I nach Anspruch 1 zur Herstellung eines Medikaments zu Behandlung von Arrhythmien.

8. Verwendung einer Verbindung I nach Anspruch 1 zur Herstellung eines Medikaments zur Behandlung oder Prophylaxe des Herzinfarkts.

9. Verwendung einer Verbindung I nach Anspruch 1 zur Herstellung eines Medikaments zur Behandlung oder Prophylaxe der Angina Pectoris.

10. Verwendung einer Verbindung I nach Anspruch 1 zur Herstellung eines Medikaments zur Behandlung oder Prophylaxe von ischämischen Zuständen des Herzens.

11. Verwendung einer Verbindung I nach Anspruch 1 zur Herstellung eines Medikaments zur Behandlung oder Prophylaxe von ischämischen Zuständen des peripheren und zentralen Nervensystems und des Schlaganfalls.

12. Verwendung einer Verbindung I nach Anspruch 1 zur Herstellung eines Medikaments zur Behandlung oder Prophylaxe von ischämischen Zuständen peripherer Organe und Gliedmaßen.

13. Verwendung einer Verbindung I nach Anspruch 1 zur Herstellung eines Medikaments zur Behandlung von Schockzuständen.

14. Verwendung einer Verbindung I nach Anspruch 1 zur Herstellung eines Medikaments zum Einsatz bei chirurgischen Operationen und Organtransplantationen.

15. Verwendung einer Verbindung I nach Anspruch 1 zur Herstellung eines Medikaments zur Konservierung und Lagerung von Transplantaten für chirurgische Maßnahmen.

16. Verwendung einer Verbindung I nach Anspruch I zur Herstellung eines Medikaments zur Behandlung von Krankheiten, bei denen die Zellproliferation eine primäre oder sekundäre Ursache darstellt.

17. Arzneimittel, enthaltend eine wirksame Menge einer Verbindung I nach einem oder mehreren der Ansprüche 1 bis 4.

## Claims

1. A compound of the formula I 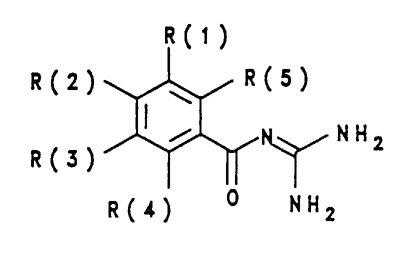 in which:
one of the three substituents R(1), R(2) and R(3) is R(6)-A-B-D-;
R(6) is a basic protonatable radical, i.e. an amino group -NR(7) R(8), an amidino group R(7)R(8)N-C[=N-R(9)]- or a guanidino group 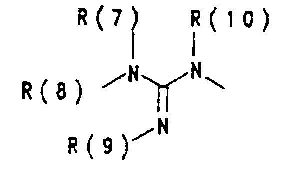
R(7), R(8), R(9) and R(10) are, independently of each other, hydrogen or alkyl having 1, 2, 3 or 4 carbon atoms;
or
R(7) and R(8) are, together, CₐH₂ₐ;
a is 4, 5, 6 or 7;
where, when a = 5, 6 or 7, a methylene group of the group CₐH₂ₐ can be replaced by a heteroatom group 0, SOₘ or NR(11),
or
R(8) and R(9) or R(9) and R(10) or R(7) and R(10) are a group CₐH₂ₐ;
a is 2, 3, 4 or 5;
where, when a = 3, 4 or 5, a methylene group of the group CₐH₂ₐ can be replaced by a heteroatom group 0, SOₘ or NR(11);
m is zero, 1 or 2;
R(11) is hydrogen or methyl;
or
R(6) is a basic heteroaromatic ring system having 1 - 9 carbon atoms;
A is C_{b}H_{2b};
b is 1, 2, 3, 4, 5, 6, 7, 8, 9 or 10;
where, in the group C_{b}H_{2b}, one or two methylene groups can be replaced by one of the groupings selected from the group consisting of -O-, -CO-, -CH[OR(20)]-, -SOₘ-, -NR(20)-, -NR(20)-CO-, -NR(20)-CO-NH-, -NR(20)-CO-NH-SO₂- 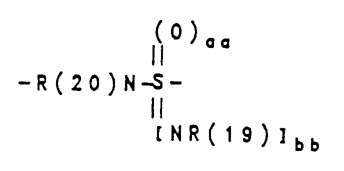 and -SOₐₐ[NR (19)]_{bb}-;
and where, in the group C_{b}H_{2b}, a methylene group can be replaced by -CH-R(99), where R(99), together with
R(7), forms a pyrrolidine or piperidine ring;
aa is 1 or 2;
bb is 0 or 1;
aa + bb = 2;
R(19) is hydrogen or alkyl having 1, 2, 3 or 4 carbon atoms
R(20) is hydrogen or methyl;
B is a phenylene or naphthylene radical 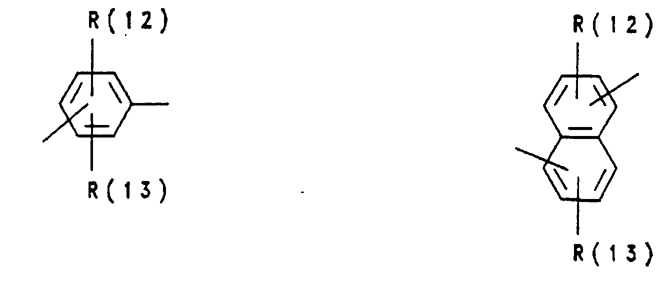
R(12) and R(13) are, independently of each other, hydrogen, methyl, F, Cl, Br, I, CF₃ or -SO_{w}-R(14);
R(14) is methyl or NR(15)R(16);
R(15) and R(16) are, independently of each other, hydrogen or alkyl having 1, 2, 3 or 4 carbon atoms;
w is zero, 1 or 2;
D is -C_{d}H_{2d}-X_{f}-;
d is zero, 1, 2, 3 or 4;
X is -O-, -CO-, -CH[OR(21)]-, -SOₘ- or -NR(21)-;
f is 1;
R(21) is hydrogen or methyl;
m is zero, 1 or 2;
and in each case the other substituents R(1) and R(2) and R(3) are,
independently of each other, hydrogen, F, Cl, Br, I, -CN,- (C₁-C₈) -alkyl,-(C₂-C₈) -alkenyl, -NR(35)R(36) or R(17)-C_{g}H_{2g}-Zₕ-;
g is zero, 1, 2, 3 or 4;
h is zero or 1;
R(35) and R(36) are, independently of each other, hydrogen or alkyl having 1, 2, 3, 4, 5 or 6 carbon atoms;
or
R(35) and R(36) are, together, 4 - 7 methylene groups of which one CH₂ group can be replaced by oxygen, -S-, -NH-, -NCH₃ or -N-benzyl;
Z is -O-, -CO-, -SOᵥ-, -NR(18)-, -NR(18)-CO-, -NR(18)-CO-NH- or -NR(18)-SO₂-;
R(18) is hydrogen or methyl;
v is zero, 1 or 2;
R(17) is hydrogen, cycloalkyl having 3, 5 or 6 carbon atoms, or CₖF₂ₖ₊₁-;
k is 1, 2 or 3,
or
R(17) is pyrrol-1-yl, pyrrol-2-yl or pyrrol-3-yl, which is not substituted or is substituted by 1 - 4 substituents selected from the group consisting of F, Cl, Br, I, -CN, (C₂-C₈)-alkanoyl, (C₂-C₈)-alkoxycarbonyl, formyl, carboxyl, -CF₃, methyl and methoxy;
or
R(17) is -(C₃-C₈)-cycloalkyl or phenyl,
which is not substituted or is substituted by 1 - 3 substituents selected from the group consisting of F and Cl, -CF₃, methyl, hydroxyl, methoxy, -NR(37)R(38), CH₃SO₂- and H₂NO₂S-;
R(37) and R(38) are hydrogen or -CH₃;
R(4) and R(5) are, independently of each other, hydrogen, alkyl having 1, 2, 3 or 4 carbon atoms, F, Cl, -OR(32), -NR(33)R(34) or -CᵣF₂ᵣ₊₁;
R(32), R(33) and R(34) are, independently of each other, hydrogen or alkyl having 1, 2 or 3 carbon atoms;
r is 1, 2, 3 or 4;
and the pharmacologically tolerated salts thereof.

2. A compound of the formula I as claimed in claim 1, wherein:
R(1) is hydrogen, F, Cl,-(C₁-C₄)-alkyl,-(C₂-C₄)-alkenyl, -NR(35)R(36) or R(17)-C_{g}H_{2g}-Zₕ-;
R(35) and R(36) are, independently of each other, hydrogen, methyl or ethyl;
or
R(35) and R(36) are, together, 4 - 5 methylene groups of which one CH₂ group can be replaced by oxygen, -S-, -NH- or -NCH₃;
R(17) is hydrogen, cycloalkyl having 5 or 6 carbon atoms, or CₖF₂ₖ₊₁-;
k is 1, 2 or 3,
g is zero, 1, 2, 3 or 4;
h is zero or 1;
Z is -O-, -CO-, -SOᵥ- , -NR(18)-, -NR(18)-CO-, -NR(18)-CO-NH- or -NR(18)-SO₂-;
R(18) is hydrogen or methyl;
v is zero, 1 or 2;
or, if g and h are zero,
R(17) is pyrrol-1-yl, pyrrol-2-yl or pyrrol-3-yl,
which is not substituted or is substituted by 1 - 2 substituents selected from the group consisting of F, Cl, (C₂-C₅)-alkanoyl, (C₂-C₅)-alkoxycarbonyl, formyl, carboxyl, -CF₃, methyl and methoxy;
or
R(17) is -(C₃-C₈)-cycloalkyl or phenyl, which is not substituted or is substituted by 1 - 2 substituents selected from the group consisting of F⁻ and Cl, -CF₃, methyl, methoxy, -NR(37)R(38), CH₃SO₂- and H₂NO₂S-;
R(37) and R(38) are, independently of each other, hydrogen or -CH₃;
one of the substituents R(2) and R(3) is
R(6)-A-B-D-;
R(6) is -NR(7)R(8), an amidino group R(7)R(8)N-C[=N-R(9)]- or a guanidino group 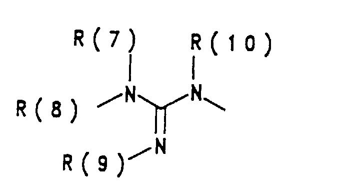
R(7), R(8), R(9) and R(10) are, independently of each other, hydrogen or alkyl having 1, 2, 3 or 4 carbon atoms;
or
R(7) and R(8) are, together, CₐH₂ₐ;
a is 4, 5, 6 or 7; where, when a = 5, 6 or 7, a methylene group of the group CₐH₂ₐ can be replaced by a heteroatom group O, SOₘ or NR(11);
R(11) is hydrogen or methyl;
or
R(8) and R(9) are, together, CₐH₂ₐ;
a is 2, 3, 4 or 5; where, when a = 3, 4 or 5, a methylene group of the group CₐH₂ₐ can be replaced by a heteroatom group O, SOₘ or NR(11);
m is zero, 1 or 2;
or
R(6) is imidazolyl, pyridyl, quinolinyl or isoquinolinyl;
A is C_{b}H_{2b};
b is 1, 2, 3, 4 or 5,
where, in the group C_{b}H_{2b}, one or two methylene groups can be replaced by one of the groupings selected from the group consisting of -O-, -CO-, -CH[OR(20)]-, -SOₘ-, NR(20), -NR(20)-CO-, -NR(20)-CO-NH-, -NR(20)-CO-NH-SO₂-, 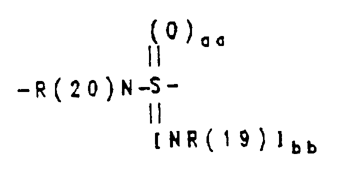 and -SOₐₐ[NR(19)]_{bb}-;
and where, in the group C_{b}H_{2b}, a methylene group can be replaced by -CH-R(99), where R(99), together with
R(7), forms a pyrrolidine or piperidine ring;
aa is 1 or 2;
bb is 0 or 1;
aa + bb = 2;
R(19) is hydrogen or alkyl having 1, 2, 3 or 4 carbon atoms
R(20) is hydrogen or methyl;
B is a phenylene or naphthylene radical 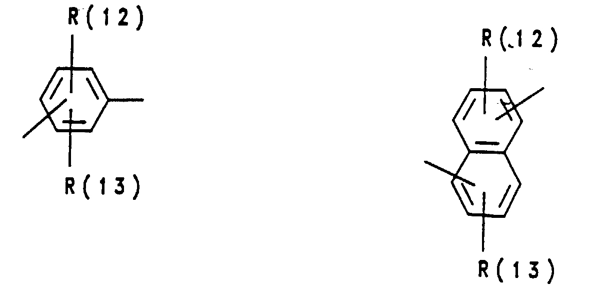
R(12) and R(13) are, independently of each other, hydrogen, methyl, F, Cl, CF₃ or -SO₂-R(14);
R(14) is methyl or NR(15)R(16);
R(15) and R(16) are, independently of each other, hydrogen or alkyl having 1, 2, 3 or 4 carbon atoms;
D is -C_{d}H_{2d}-X_{f}-;
d is zero, 1, 2, 3 or 4;
X is -O-, -CO-, -CH[OR(21)]-, -SOₘ- or -NR(21)-;
f is 1;
R(21) is hydrogen or methyl;
m is zero, 1 or 2;
and in each case the other radical R(2) and R(3) is
hydrogen, alkyl having 1, 2, 3 or 4 carbon atoms, F, Cl or CF₃;
R(4) and R(5) are, independently of each other hydrogen, alkyl having 1, 2 or 3 carbon atoms, F, Cl, or -CF₃.

3. A compound of the formula I as claimed in claim 1 or 2, wherein:
R(1) is hydrogen, F, Cl, alkyl having 1, 2, 3 or 4 carbon atoms, -NR(35)R(36) or R(17)-C_{g}H_{2g}-Zₕ-;
g is zero, 1, 2, 3 or 4;
h is zero or 1;
Z is -O-, -CO-, -SOᵥ-, -NR(18)-, -NR(18)-CO-, -NR(18)-CO-NH- or -NR(18)-SO₂-;
R(18) is hydrogen or methyl;
v is zero, 1 or 2;
R(17) is hydrogen, cycloalkyl having 5 or 6 carbon atoms, or CF₃-;
or, if g and h are zero,
R(17) is pyrrol-1-yl, which is not substituted or is substituted by 1 - 2 substituents selected from the group consisting of F, Cl, (C₂-C₅)-alkanoyl, (C₂-C₅)-alkoxycarbonyl, -CF₃ and methyl;
or
R(17) is -(C₅-C₆)-cycloalkyl or phenyl, which is not substituted or is substituted by a substituent which is selected from the group consisting of F and Cl, -CF₃, methyl, CH₃SO₂- and H₂NO₂S-;
R(35) and R(36) are, independently of each other, hydrogen, methyl or ethyl;
or
R(35) and R(36) are, together, 4 - 5 methylene groups of which one CH₂ group can be replaced by oxygen, -S-, -NH- or -NCH₃;
one of the substituents R(2) and R(3) is
R(6)-A-B-D-;
R(6) is -NR(7)R(8), an amidino group R(7)R(8)N-C[=N-R(9)]- or a guanidino group 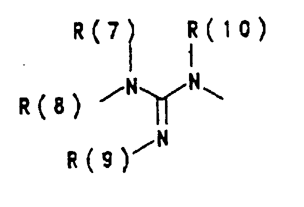
R(7) is hydrogen or alkyl having 1, 2, 3 or 4 carbon atoms;
R(8), R(9) and R(10) are, independently of each other, hydrogen, methyl or ethyl;
or
R(7) and R(8) are, together, CₐH₂ₐ;
a is 4 or 5;
where, when a = 5, a methylene group of the group CₐH₂ₐ can be replaced by NR(11),
R(11) is hydrogen or methyl;
or
R(6) is imidazolyl or pyridyl;
A is C_{b}H_{2b};
b is 1, 2, 3, 4 or 5,
where, in the group C_{b}H_{2b}, one or two methylene groups can be replaced by one of the groupings selected from the group consisting of -CO-, -CH[OR(20)]-, -NR(20)-CO-, 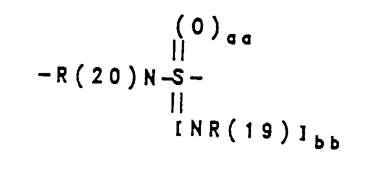 -SOₐₐ[NR(19)]_{bb} and -SO₂-;
and where, in the group C_{2b}H_{2b}, a methylene group can be replaced by -CH-R(99), where R(99), together with R(7), forms a pyrrolidine or piperidine ring;
aa is 1 or 2;
bb is 0 or 1;
aa + bb = 2;
R(19) is hydrogen or alkyl having 1, 2, 3 or 4 carbon atoms
R(20) is hydrogen or methyl;
or, if b is 2, 3, 4 or 5,
a carbon atom in C_{b}H_{2b} can be replaced by a grouping -O-, -S-, -NR(20)-, -NR(20)-CO- or -NR(20) -CO-NH-;
B is a phenylene radical, 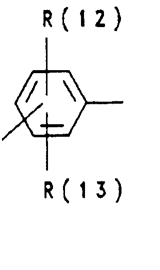
R(12) and R(13) are, independently of each other, hydrogen, methyl, F, Cl, CF₃ or -SO₂R(14);
R(14) is methyl or NH₂;
D is -CH₂-, -O-, -CO-, -SOₘ- or -NR(21)-;
m is zero or 2;
R(21) is hydrogen or methyl;
and in each case the other radical R(2) and R(3) is hydrogen;
R(4) and R(5) are, independently of each other, hydrogen, alkyl having 1, 2 or 3 carbon atoms, F, Cl or -CF₃.

4. A compound of the formula I as claimed in at least one of claims 1 - 3, wherein:
R(1) is hydrogen, F, Cl, alkyl having 1, 2, 3 or 4 carbon atoms, -NR(35)R(36) or R(17)-C_{g}H_{2g}-Zₕ-;
g is zero or 1;
h is zero or 1;
Z is -O-, -CO-, -NR(18)-CO-, -NR(18)- CO-NH- or -NR(18)-SO₂-;
R(18) is hydrogen or methyl;
or, if g is 1;
Z is -SO₂-;
R(17) is hydrogen or CF₃-;
R(35) and R(36) are, independently of each other, hydrogen, methyl or ethyl;
or
R(35) and R(36) are, together, 4 - 5 methylene groups of which one CH₂ group can be replaced by oxygen, -S-, -NH- or -NCH₃;
one of the substituents R(2) and R(3) is
R(6)-A-B-O-;
R(6) is -NR(7)R(8) or a guanidino group 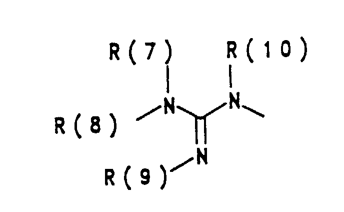
R(7) is hydrogen or alkyl having 1, 2, 3 or 4 carbon atoms;
R(8), R(9) and R(10) are, independently of each other, hydrogen, methyl or ethyl;
or
R(7) and R(8) are, together, CₐH₂ₐ;
a is 4 or 5;
where, when a = 5, a methylene group of the group CₐH₂ₐ can be replaced by -NH- or -NCH₃-,
or
R(6) is imidazolyl;
A is C_{b}H_{2b};
b is 1, 2, 3 or 4;
where, in the group C_{b}H_{2b}, one or two methylene groups can be replaced by one of the groupings selected from the group consisting of -CO-, 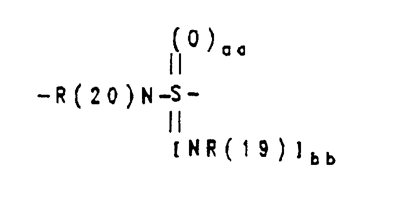 -Soₐₐ[NR(19)]_{bb}- and -SO₂-, and where, in the group C_{b}H_{2b}, a methylene group can be replaced by -CH-R(99), where R(99), together with R(7), can form a pyrrolidine or piperidine ring;
or, if b is 2, 3 or 4,
a methylene group in the group C_{b}H_{2b} can be replaced by a grouping -O- or -S-;
aa is 1 or 2;
bb is 0 or 1;
aa + bb = 2;
R(19) is hydrogen or alkyl having 1, 2, 3 or 4 carbon atoms
R(20) is hydrogen or methyl;
B is a phenylene radical 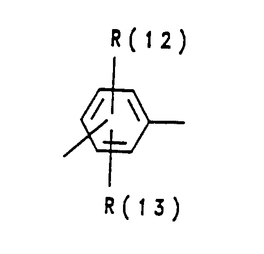
R(12) and R(13) are hydrogen;
and in each case the other radical R(2) and R(3) is hydrogen;
R(4) and R(5) are hydrogen.

5. A compound of the formula I as claimed in claim 1, which is selected from the group consisting of 4-[4-N-(dimethylaminoethyl)methylsulfamoyl]phenoxy-3-trifluoromethylbenzoylguanidine, dihydrochloride; 4-[4-(4-methylpiperazinosulfonyl)phenoxy]-3-trifluoromethylbenzoylguanidine, dihydrochloride; 4-[4-(2-pyrrolidineethylaminosulfonyl)phenoxy]-3-trifluoromethylbenzoylguanidine, dimaleate; 4-[4-(2-piperidineethylaminosulfonyl)phenoxy]-3-trifluoromethylbenzoylguanidine, dimaleate; 4-[4-(N-dimethylamino-n-propyl)sulfamoyl]phenoxy-3-trifluoromethylbenzoylguanidine; 4-[4-(N-dimethylaminoethyl)sulfamoyl]phenoxy-3-trifluoromethylbenzoylguanidine; 4-(4-imidamidosulfonyl)phenoxy-3-trifluoromethylbenzoylguanidine; 3-trifluoromethyl-4-(4-N-methylimidamidosulfonyl)phenoxybenzoylguanidine; 3-methyl-4-(4-(1-methylpiperazin-4-ylsulfonyl)phenoxy)benzoylguanidine; 4-(4-guanidinosulfonyl)phenoxy-3-trifluoromethylbenzoylguanidine; 4-[4-(2-imidazolylthioacetyl)phenoxy]-3-methylsulfonylbenzoylguanidine dihydrochloride; 4-[4-(N,N'-dimethyl-S-isothiuronylacetyl)phenoxy]-3-methylsulfonylbenzoylguanidine dihydrochloride; 4-[4-(2-benzimidazolylthioacetyl)phenoxy]-3-methylsulfonylbenzoylguanidine dihydrochloride; 4-[4-(2-N-imidazolyl-1-hydroxyethyl)phenoxy]-3-methylsulfonylbenzoylguanidine dihydrochloride; 4-[4-(N,N-dimethylglycylamino)phenoxy]-3-methylsulfonylbenzoylguanidine dihydrochloride; 4-[4-(N,N-diethylaminoethyl)aminosulfonylphenoxy]-3-methylsulfonylbenzoylguanidine dihydrochloride; 4-[4-(4-imidazolylethyl)aminosulfonylphenoxy]-3-methylsulfonylbenzoylguanidine dihydrochloride; 4-[4-(3-N-imidazolyl-l-propyl)aminosulfonylphenoxy]-3-methylsulfonylbenzoylguanidine dihydrochloride; 4-[4-(1-methyl-2-pyrrolidinylethyl)aminosulfonylphenoxy]-3-methylsulfonylbenzoylguanidine dihydrochloride; 4-[4-(N-piperidinoethyl)aminosulfonylphenoxy]-3-methylsulfonylbenzoylguanidine dihydrochloride; 4-[4-(2-dimethylaminoethyl)sulfonylmethylphenoxy]-3-methylsulfonylbenzoylguanidine dihydrochloride; 4-[4-(2-dimethylaminoethyl)sulfonylmethylphenoxy]-3-trifluoromethylbenzoylguanidine dihydrochloride.

6. A process for preparing a compound I as claimed in claim 1, which comprises reacting a compound of the formula II 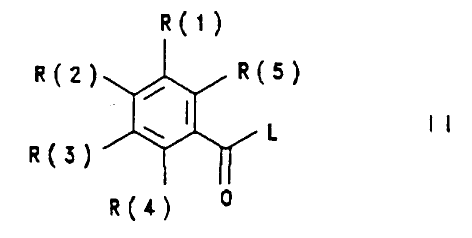 in which R(1) to R(5) have the given meaning and L is a leaving group which can readily be substituted nucleophilically,
with guanidine,
and, where appropriate, converting the product into a pharmacologically tolerated salt.

7. The use of a compound I as claimed in claim 1 for preparing a medicament for treating arrhythmias.

8. The use of a compound I as claimed in claim 1 for preparing a medicament for the treatment or prophylaxis of cardiac infarction.

9. The use of a compound I as claimed in claim 1 for preparing a medicament for the treatment or prophylaxis of angina pectoris.

10. The use of a compound I as claimed in claim 1 for preparing a medicament for the treatment or prophylaxis of ischemic conditions of the heart.

11. The use of a compound I as claimed in claim 1 for preparing a medicament for the treatment or prophylaxis of ischemic conditions of the peripheral and central nervous system and of stroke.

12. The use of a compound I as claimed in claim 1 for preparing a medicament for the treatment or prophylaxis of ischemic conditions of peripheral organs and limbs.

13. The use of a compound I as claimed in claim 1 for preparing a medicament for the treatment of states of shock.

14. The use of a compound I as claimed in claim 1 for preparing a medicament for use in surgical operations and organ transplants.

15. The use of a compound I as claimed in claim 1 for preparing a medicament for the preservation and storage of transplants for surgical procedures.

16. The use of a compound I as claimed in claim 1 for preparing a medicament for the treatment of diseases in which cell proliferation represents a primary or secondary cause.

17. A medicine which contains an effective quantity of a compound I as claimed in one or more of claims 1 to 4.

## Revendications

1. Composés de formule I 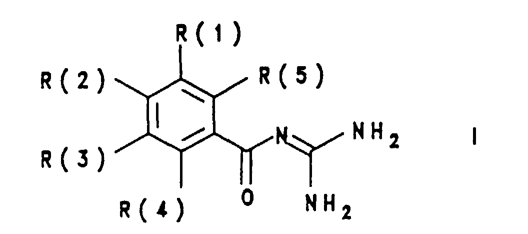 dans laquelle
un des trois substituants R(1), R(2) et R(3) représente
R(6)-A-B-D-,
R(6) représente un radical basique protonable, c'est-à-dire un groupe amino -NR(7)R(8), un groupe amidino R(7)R(8)N-C[=N-R(9)]- ou un groupe guanidino 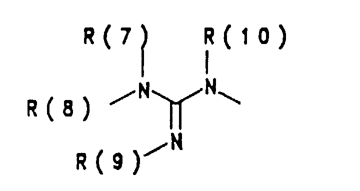
R(7) , R(8), R(9) et R(10) représentent, indépendamment les uns des autres, un atome d'hydrogène ou un groupe alkyle ayant 1, 2, 3 ou 4 atomes de carbone,
ou
R(7) et R(8) forment ensemble un groupe CₐH₂ₐ,
a est égal à 4, 5, 6 ou 7, dans le cas de a = 5, 6 ou 7, un groupe méthylène du groupe CₐH₂ₐ pouvant être remplacé par un hétéroatome O ou par SOₘ ou NR(11),
ou
R(8) et R(9) ou R(9) et R(10) ou R(7) et R(10) forment ensemble un groupe CₐH₂ₐ,
a est égal à 2, 3, 4 ou 5, dans le cas de a = 3, 4 ou 5, un groupe méthylène du groupe CₐH₂ₐ pouvant être remplacé par un hétéroatome O ou par SOₘ ou NR(11),
m étant égal à zéro, 1 ou 2,
R(11) représentant un atome d'hydrogène ou le groupe méthyle,
ou
R(6) représente un système cyclique hétéroaromatique basique ayant de 1 à 9 atomes de carbone,
A représente un groupe C_{b}H_{2b},
b est égal à 1, 2, 3, 4, 5, 6, 7, 8, 9 ou 10, un ou deux groupes méthylène dans le groupe C_{b}H_{2b} pouvant être remplacés par l'un des groupements choisis dans l'ensemble constitué par -O-, -CO-, -CH[OR(20)]-, -SOₘ-, -NR(20)-, -NR(20)-CO-, -NR(20)-CO-NH-, -NR(20) -CO-NH-SO₂-, 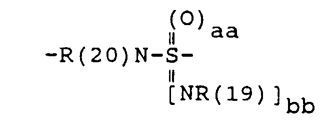 et -SOₐₐ[NR(19)]_{bb}-,
et dans le groupe C_{b}H_{2b} un groupe méthylène pouvant être remplacé par -CH-R(99), R(99) formant conjointement avec R(7) un cycle pyrrolidine ou pipéridine,
aa étant 1 ou 2,
bb étant 0 ou 1,
aa + bb étant égal à 2,
R(19) représentant un atome d'hydrogène ou un groupe alkyle ayant 1, 2, 3 ou 4 atomes de carbone,
R(20) représentant un atome d'hydrogène ou le groupe méthyle,
B représente un radical phénylène ou naphtylène, 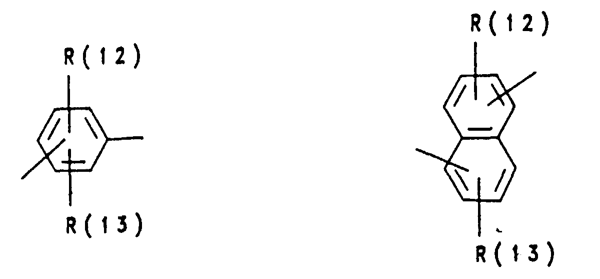
R(12) et R(13) représentent, indépendamment l'un de l'autre, un atome d'hydrogène, le groupe méthyle, F, Cl, Br, I, CF₃ ou -SO_{w}-R(14),
R(14) représente un groupe méthyle ou NR(15)R(16),
R(15) et R(16) représentent, indépendamment l'un de l'autre, un atome d'hydrogène ou un groupe alkyle ayant 1, 2, 3 ou 4 atomes de carbone
w est égal à zéro, 1 ou 2,
D représente C_{d}H_{2d}-X_{f}-,
d est égal à zéro, 1, 2, 3 ou 4,
X représente -O-, -CO-, -CH[OR(21)]-, -SOₘ- ou -NR(21)-,
f est égal à 1,
R(21) représentant un atome d'hydrogène ou le groupe méthyle,
m étant égal à zéro, 1 ou 2,
et les autres substituants R(1) et R(2) et R(3)
représentent, indépendamment les uns des autres, un atome d'hydrogène, F, Cl, Br, I, -CN, un groupe alkyle en C₁-C₈, alcényle en C₂-C₈, -NR(35)R(36) ou R(17)-C_{g}H_{2g}-Zₕ-,
g est égal à zéro, 1, 2, 3 ou 4,
h est égal à zéro ou 1,
R(35) et R(36) représentent, indépendamment l'un de l'autre, un atome d'hydrogène ou un groupe alkyle ayant 1, 2, 3, 4, 5 ou 6 atomes de carbone,
ou bien
R(35) et R(36) représentent ensemble 4 - 7 groupes méthylène, dont un groupe CH₂ peut être remplacé par un atome d'oxygène ou de soufre, ou par un groupe -NH-, -NCH₃ ou -N-benzyle,
Z représente -O-, -CO-, -SOᵥ-, -NR(18)-, -NR(18)-CO-, -NR(18)-CO-NH- ou -NR(18)-SO₂-,
R(18) représentant un atome d'hydrogène ou le groupe méthyle,
v étant zéro, 1 ou 2,
R(17) représente un atome d'hydrogène ou un groupe cycloalkyle ayant 3, 5 ou 6 atomes de carbone ou CₖF₂ₖ₊₁-,
k étant 1, 2 ou 3,
ou
R(17) représente un groupe pyrrol-1-yle, pyrrol-2-yle ou pyrrol-3-yle,
qui n'est pas substitué ou porte 1 - 4 substituants choisis dans l'ensemble constitué par F, Cl, Br, I, -CN, les groupes alcanoyle en C₂-C₈, alcoxycarbonyle en C₂-C₈, formyle, carboxyle, -CF₃, méthyle et méthoxy,
ou
R(17) représente un groupe cycloalkyle en C₃-C₈ ou phényle,
qui n'est pas substitué ou porte 1 - 3 substituants choisis dans l'ensemble constitué par F et Cl, CF₃, les groupes méthyle, hydroxyle, méthoxy, -NR(37)R(38), CH₃SO₂- et H₂NO₂S-,
R(37) et R(38) représentant un atome d'hydrogène ou -CH₃,
R(4) et R(5) représentent, indépendamment l'un de l'autre, un atome d'hydrogène ou un groupe alkyle ayant 1, 2, 3 ou 4 atomes de carbone, F, Cl, -OR(32),
-NR(33)R(34) ou -CᵣF₂ᵣ₊₁,
R(32) , R(33) et R(34) représentant, indépendamment les uns des autres, un atome d'hydrogène ou un groupe alkyle ayant 1, 2 ou 3 atomes de carbone,
r étant égal à 1, 2, 3 ou 4,
ainsi que leurs sels pharmacologiquement acceptables.

2. Composés de formule I selon la revendication 1, caractérisés en ce que, dans ces composés:
R(1) représente un atome d'hydrogène, F, Cl, un groupe alkyle en C₁-C₄, alcényle en C₂-C₄, -NR(35)R(36) ou R(17)C_{g}H_{2g}-Zₕ-,
R(35) et R(36) représentent, indépendamment l'un de l'autre, un atome d'hydrogène ou le groupe méthyle ou éthyle,
ou bien
R(35) et R(36) représentent ensemble 4 - 5 groupes méthylène, dont un groupe CH₂ peut être remplacé par un atome d'oxygène ou de soufre, ou par un groupe -NH- ou -NCH₃,
R(17) représente un atome d'hydrogène ou un groupe cycloalkyle ayant 5 ou 6 atomes de carbone ou CₖF₂ₖ₊₁-,
k étant 1, 2 ou 3,
g est égal à zéro, 1, 2, 3 ou 4,
h est égal à zéro ou 1,
Z représente -O-, -CO-, -SOᵥ-, -NR(18)-, -NR(18)-CO-, -NR(18)-CO-NH- ou -NR(18)-SO₂-,
R(18) représentant un atome d'hydrogène ou le groupe méthyle,
v étant zéro, 1 ou 2,
ou, lorsque g et h valent zéro,
R(17) représente un groupe pyrrol-1-yle, pyrrol-2-yle ou pyrrol-3-yle,
qui n'est pas substitué ou porte 1 - 2 substituants choisis dans l'ensemble constitué par F, Cl, les groupes alcanoyle en C₂-C₅, alcoxycarbonyle en C₂-C₅, formyle, carboxyle, -CF₃, méthyle et méthoxy,
ou
R(17) représente un groupe cycloalkyle en C₃-C₈ ou phényle,
qui n'est pas substitué ou porte 1 - 2 substituants choisis dans l'ensemble constitué par F et Cl, -CF₃, les groupes méthyle, méthoxy, -NR(37)R(38), CH₃SO₂- et H₂NO₂S-,
R(37) et R(38) représentant, indépendamment l'un de l'autre, un atome d'hydrogène ou CH₃,
l'un des substituants R(2) et R(3) représente
R(6)-A-B-D-,
R(6) représente -NR(7)R(8), un groupe amidino R(7)R(8)N-C[=N-R(9)]- ou un groupe guanidino 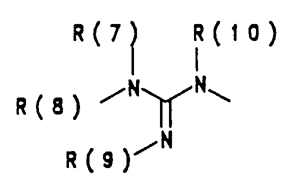
R(7), R(8), R(9) et R(10) représentent, indépendamment les uns des autres, un atome d'hydrogène ou un groupe alkyle ayant 1, 2, 3 ou 4 atomes de carbone,
ou
R(7) et R(8) forment ensemble un groupe CₐH₂ₐ, a est égal à 4, 5, 6 ou 7,
dans le cas de a = 5, 6 ou 7, un groupe méthylène du groupe CₐH₂ₐ pouvant être remplacé par un hétéroatome O ou par SOₘ ou NR(11),
R(11) représentant un atome d'hydrogène ou le groupe méthyle,
ou
R(8) et R(9) forment ensemble un groupe CₐH₂ₐ,
a est égal à 2, 3, 4 ou 5, dans le cas de a = 3, 4 ou 5, un groupe méthylène du groupe CₐH₂ₐ pouvant être remplacé par un hétéroatome O ou par SOₘ ou NR(11),
m étant égal à zéro, 1 ou 2,
ou
R(6) représente un cycle imidazolyle, pyridyle, quinolyle ou isoquinolinyle,
A représente un groupe C_{b}H_{2b},
b est égal à 1, 2, 3, 4 ou 5, un ou deux groupes méthylène dans le groupe C_{b}H_{2b} pouvant être remplacés par l'un des groupements choisis dans l'ensemble constitué par -O-, -CO-, -CH[OR(20)]-, -SOₘ-, -NR(20)-, -NR(20)-CO-, -NR(20)-CO-NH-, -NR(20)-CO-NH-SO₂-, 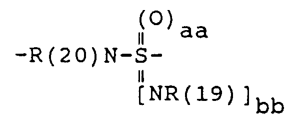 et -SOₐₐ[NR(19)]_{bb}-,
et dans le groupe C_{b}H_{2b} un groupe méthylène pouvant être remplacé par -CH-R(99), R(99) formant conjointement avec R(7) un cycle pyrrolidine ou pipéridine,
aa étant 1 ou 2,
bb étant 0 ou 1,
aa + bb étant égal à 2,
R(19) représentant un atome d'hydrogène ou un groupe alkyle ayant 1, 2, 3 ou 4 atomes de carbone,
R(20) représentant un atome d'hydrogène ou le groupe méthyle,
B représente un radical phénylène ou naphtylène, 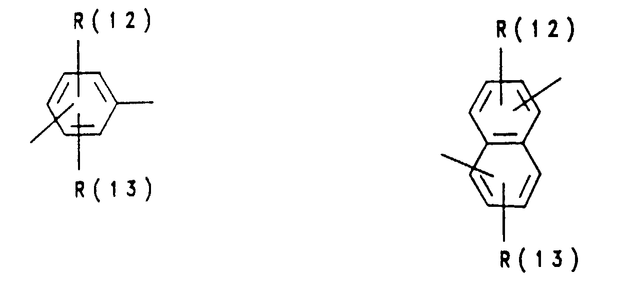
R(12) et R(13) représentent, indépendamment l'un de l'autre, un atome d'hydrogène, le groupe méthyle, F, Cl, CF₃ ou -SO₂-R(14),
R(14) représente un groupe méthyle ou NR(15)R(16),
R(15) et R(16) représentent, indépendamment l'un de l'autre, un atome d'hydrogène ou un groupe alkyle ayant 1, 2, 3 ou 4 atomes de carbone,
D représente -C_{d}H_{2d}-X_{f}-,
d est égal à zéro, 1, 2, 3 ou 4,
X représente -O-, -CO-, -CH[OR(21)]-, -SOₘ- ou -NR(21)-,
f est égal à 1,
R(21) représentant un atome d'hydrogène ou le groupe méthyle,
m étant égal à zéro, 1 ou 2,
et l'autre radical R(2) ou R(3)
représente un atome d'hydrogène ou un groupe alkyle ayant 1, 2, 3 ou 4 atomes de carbone, F, Cl ou CF₃,
R(4) et R(5) représentent, indépendamment l'un de l'autre, un atome d'hydrogène ou un groupe alkyle ayant 1, 2 ou 3 atomes de carbone, F, Cl ou CF₃.

3. Composés de formule I selon la revendication 1 ou 2, caractérisés en ce que, dans ces composés:
R(1) représente un atome d'hydrogène, F, Cl, un groupe alkyle ayant 1, 2, 3 ou 4 atomes de carbone, -NR(35)R(36) ou R(17)-C_{g}H_{2g}-Zₕ-,
g est égal à zéro, 1, 2, 3 ou 4,
h est égal à zéro ou 1,
Z représente -O-, -CO-, -SOᵥ-, -NR(18)-, -NR(18)-CO-, -NR(18)-CO-NH- ou -NR(18)-SO₂-,
R(18) représentant un atome d'hydrogène ou le groupe méthyle,
v étant zéro, 1 ou 2,
R(17) représente un atome d'hydrogène ou un groupe cycloalkyle ayant 5 ou 6 atomes de carbone ou CF₃-,
ou, lorsque g et h valent zéro,
R(17) représente un groupe pyrrol-1-yle, qui n'est pas substitué ou porte 1 - 2 substituants choisis dans l'ensemble constitué par F, Cl, les groupes alcanoyle en C₂-C₅, alcoxycarbonyle en C₂-C₅, -CF₃ et méthyle,
ou
R(17) représente un groupe cycloalkyle en C₅-C₆ ou phényle,
qui n'est pas substitué ou porte un substituant choisi dans l'ensemble constitué par F et Cl, -CF₃, les groupes méthyle, CH₃SO₂- et H₂NO₂S-,
R(35) et R(36) représentent, indépendamment l'un de l'autre, un atome d'hydrogène ou le groupe méthyle ou éthyle,
ou bien
R(35) et R(36) représentent ensemble 4 - 5 groupes méthylène, dont un groupe CH₂ peut être remplacé par un atome d'oxygène ou de soufre, ou par un groupe -NH- ou -NCH₃,
l'un des substituants R(2) et R(3) représente
R(6)-A-B-D-,
R(6) représente -NR(7)R(8), un groupe amidino R(7)R(8)N-C[=N-R(9)]- ou un groupe guanidino 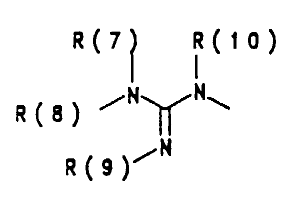
R(7) représente un atome d'hydrogène ou un groupe alkyle ayant 1, 2, 3 ou 4 atomes de carbone,
R(8), R(9) et R(10) représentent, indépendamment les uns des autres, un atome d'hydrogène ou le groupe méthyle ou éthyle,
ou
R(7) et R(8) forment ensemble un groupe CₐH₂ₐ, a est égal à 4 ou 5,
dans le cas de a = 5, un groupe méthylène du groupe CₐH₂ₐ pouvant être remplacé par NR(11),
R(11) représentant un atome d'hydrogène ou le groupe méthyle,
ou
R(6) représente le cycle imidazolyle ou pyridyle,
A représente un groupe C_{b}H_{2b},
b est égal à 1, 2, 3, 4 ou 5,
un ou deux groupes méthylène dans le groupe C_{b}H_{2b} pouvant être remplacés par l'un des groupements choisis dans l'ensemble constitué par -CO-, -CH[OR(20)]-, -NR(20)-CO-, 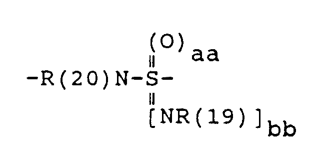 -SOₐₐ[NR(19)]_{bb}- et -SO₂-,
et dans le groupe C_{b}H_{2b} un groupe méthylène pouvant être remplacé par -CH-R(99), R(99) formant conjointement avec R(7) un cycle pyrrolidine ou pipéridine,
aa étant 1 ou 2,
bb étant 0 ou 1,
aa + bb étant égal à 2,
R(19) représentant un atome d'hydrogène ou un groupe alkyle ayant 1, 2, 3 ou 4 atomes de carbone,
R(20) représentant un atome d'hydrogène ou le groupe méthyle,
ou, lorsque b représente 2, 3, 4 ou 5,
un atome de carbone dans C_{b}H_{2b} peut être remplacé par -O-, -S-, -NR(20)-, -NR(20)-CO- ou -NR(20)-CO-NH-,
B représente un radical phénylène, 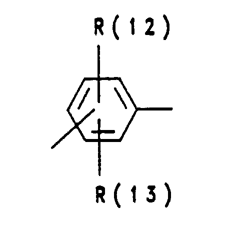
R(12) et R(13) représentent, indépendamment l'un de l'autre, un atome d'hydrogène, le groupe méthyle, F, Cl, CF₃ ou -SO₂-R(14),
R(14) représente un groupe méthyle ou NH₂,
D représente -CH₂-, -O-, -CO-, -SOₘ- ou -NR(21)-,
m étant zéro ou 2,
R(21) représentant un atome d'hydrogène ou le groupe méthyle,
et l'autre radical R(2) ou R(3) représente un atome d'hydrogène,
R(4) et R(5) représentent, indépendamment l'un de l'autre, un atome d'hydrogène ou un groupe alkyle ayant 1, 2 ou 3 atomes de carbone, F, Cl ou CF₃.

4. Composés de formule I selon au moins l'une des revendications 1 à 3, caractérisés en ce que, dans ces composés:
R(1) représente un atome d'hydrogène, F, Cl, un groupe alkyle ayant 1, 2, 3 ou 4 atomes de carbone, -NR(35)R(36) ou R(17)-C_{g}H_{2g}-Zₕ-,
g est égal à zéro ou 1,
h est égal à zéro ou 1,
Z représente -O-, -CO-, -NR(18)-CO-, -NR(18)-CO-NH- ou -NR(18)-SO₂-,
R(18) représentant un atome d'hydrogène ou le groupe méthyle,
ou, lorsque g est égal à 1,
Z représente -SO₂-,
R(17) représente un atome d'hydrogène ou CF₃-,
R(35) et R(36) représentent, indépendamment l'un de l'autre, un atome d'hydrogène ou le groupe méthyle ou éthyle,
ou bien
R(35) et R(36) représentent ensemble 4 - 5 groupes méthylène, dont un groupe CH₂ peut être remplacé par un atome d'oxygène ou de soufre, ou par un groupe -NH- ou -NCH₃,
l'un des substituants R(2) et R(3) représente
R(6)-A-B-O-,
R(6) représente -NR(7)R(8) ou un groupe guanidino 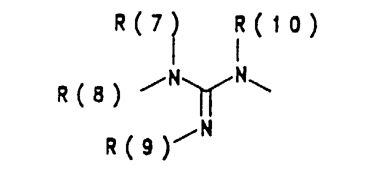
R(7) représente un atome d'hydrogène ou un groupe alkyle ayant 1, 2, 3 ou 4 atomes de carbone,
R(8), R(9) et R(10) représentent, indépendamment les uns des autres, un atome d'hydrogène ou le groupe méthyle ou éthyle,
ou
R(7) et R(8) forment ensemble un groupe CₐH₂ₐ, a est égal à 4 ou 5,
dans le cas de a = 5, un groupe méthylène du groupe CₐH₂ₐ pouvant être remplacé par NH- ou -NCH₃-,
ou
R(6) représente le cycle imidazolyle,
A représente un groupe C_{b}H_{2b},
b est égal à 1, 2, 3 ou 4,
un ou deux groupes méthylène dans le groupe C_{b}H_{2b} pouvant être remplacés par l'un des groupements choisis dans l'ensemble constitué par -CO-, 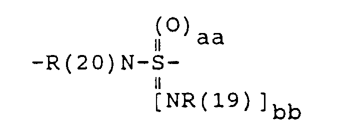 -SOₐₐ[NR(19)]_{bb}- et -SO₂-,
et dans le groupe C_{b}H_{2b} un groupe méthylène pouvant être remplacé par -CH-R(99), R(99) formant conjointement avec R(7) un cycle pyrrolidine ou pipéridine,
ou, lorsque b représente 2, 3 ou 4, un groupe méthylène dans le groupe C_{b}H_{2b} peut être remplacé par -O- ou -S-,
aa étant 1 ou 2,
bb étant 0 ou 1,
aa + bb étant égal à 2,
R(19) représentant un atome d'hydrogène ou un groupe alkyle ayant 1, 2, 3 ou 4 atomes de carbone,
R(20) représentant un atome d'hydrogène ou le groupe méthyle,
B représente un radical phénylène, 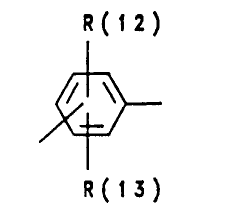
R(12) et R(13) représentent chacun un atome d'hydrogène,
et l'autre radical R(2) ou R(3) représente un atome d'hydrogène,
R(4) et R(5) représentent chacun un atome d'hydrogène.

5. Composé de formule I selon la revendication 1, caractérisé en ce qu'il est choisi dans le groupe constitué par
la 4-[4-N-(diméthylaminoéthyl)méthylsulfamoyl]phénoxy-3-trifluorométhylbenzoylguanidine (dichlorhydrate);
la 4-[4-(4-méthylpipérazinosulfonyl)phénoxy]-3-trifluorométhylbenzoylguanidine (dichlorhydrate);
la 4-[4-(2-pyrrolidine-éthylaminosulfonyl)phénoxy]-3-trifluorométhylbenzoylguanidine (dimaléate);
la 4-[4-(2-pipéridine-éthylaminosulfonyl]phénoxy]-3-trifluorométhylbenzoylguanidine (dimaléate);
la 4-[4-(N-diméthylamino-n-propyl)sulfamoyl]phénoxy-3-trifluorométhylbenzoylguanidine;
la 4-[4-(N-diméthylaminoéthyl)sulfamoyl]phénoxy-3-trifluorométhylbenzoylguanidine;
la 4-(4-imidamidosulfonyl)phénoxy-3-trifluorométhylbenzoylguanidine;
la 3-trifluorométhyl-4-(4-N-méthylimidamidosulfonyl)phénoxy-benzoylguanidine;
la 3-méthyl-4-(4-(1-méthylpipérazine-4-ylsulfonyl)phénoxy)benzoylguanidine;
la 4-(4-guanidinosulfonyl)phénoxy-3-trifluorométhylbenzoylguanidine;
la 4-[4-(2-imidazolylthio-acétyl)phénoxy]-3-méthylsulfonyl-benzoylguanidine (dichlorhydrate);
la 4-[4-(N,N'-diméthyl-S-isothiuronyl-acétyl)phénoxy]-3-méthylsulfonyl-benzoylguanidine (dichlorhydrate);
la 4-[4-(2-benzimidazolylthio-acétyl)phénoxy]-3-méthylsulfonyl-benzoylguanidine (dichlorhydrate);
la 4-[4-(2-N-imidazolyl-1-hydroxyéthyl)phénoxy]-3-méthylsulfonyl-benzoylguanidine (dichlorhydrate);
la 4-[4-(N,N-diméthylglycylamino)phénoxy]-3-méthylsulfonylbenzoylguanidine (dichlorhydrate);
la 4-[4-(N,N-diéthylaminoéthyl)aminosulfonylphénoxy]-3-méthylsulfonylbenzoylguanidine (dichlorhydrate);
la 4-[4-(4-imidazolyléthyl)aminosulfonyl-phénoxy]-3-méthylsulfonyl-benzoylguanidine, (dichlorhydrate);
la 4-[4-(3-N-imidazolyl-1-propyl)aminosulfonyl-phénoxy]-3-méthylsulfonylbenzoylguanidine (dichlorhydrate);
la 4-[4-(1-méthyl-2-pyrrolidinyléthyl)aminosulfonylphénoxy]-3-méthylsulfonylbenzoylguanidine (dichlor- hydrate)
la 4-[4-(N-pipéridinoéthyl)aminosulfonyl-phénoxy]-3-méthylsulfonyl-benzoylguanidine (dichlorhydrate);
la 4-[4-(2-diméthylaminoéthyl)sulfonylméthyl-phénoxy]-3-méthylsulfonylbenzoylguanidine (dichlorhydrate);
la 4-[4-(2-diméthylaminoéthyl)sulfonylméthyl-phénoxy]-3-trifluorométhylbenzoylguanidine (dichlorhydrate).

6. Procédé pour la préparation d'un composé I selon la revendication 1, caractérisé en ce que l'on fait réagir avec de la guanidine un composé de formule II 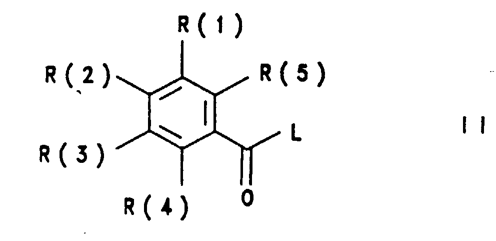 dans laquelle R(1) à R(5) ont les significations indiquées et L représente un groupe partant pouvant être aisément remplacé par substitution nucléophile,
et en ce que, éventuellement, on convertit le produit obtenu en un sel pharmacologiquement acceptable.

7. Utilisation d'un composé I selon la revendication 1, pour la fabrication d'un médicament destiné au traitement d'arythmies.

8. Utilisation d'un composé I selon la revendication 1, pour la fabrication d'un médicament destiné au traitement ou à la prophylaxie de l'infarctus du myocarde.

9. Utilisation d'un composé I selon la revendication 1, pour la fabrication d'un médicament destiné au traitement ou à la prophylaxie de l'angine de poitrine.

10. Utilisation d'un composé I selon la revendication 1, pour la fabrication d'un médicament destiné au traitement ou à la prophylaxie d'états ischémiques du coeur.

11. Utilisation d'un composé I selon la revendication 1, pour la fabrication d'un médicament destiné au traitement ou à la prophylaxie d'états ischémiques du système nerveux périphérique et central et de l'apoplexie cérébrale.

12. Utilisation d'un composé I selon la revendication 1, pour la fabrication d'un médicament destiné au traitement ou à la prophylaxie d'états ischémiques d'organes périphériques et des membres.

13. Utilisation d'un composé I selon la revendication 1, pour la fabrication d'un médicament destiné au traitement d'états de choc.

14. Utilisation d'un composé I selon la revendication 1, pour la fabrication d'un médicament destiné à l'utilisation dans des opérations chirurgicales et des greffes d'organes.

15. Utilisation d'un composé I selon la revendication 1, pour la fabrication d'un médicament destiné à la conservation et au stockage de transplants pour des opérations chirurgicales.

16. Utilisation d'un composé I selon la revendication 1, pour la fabrication d'un médicament destiné au traitement de maladies dans lesquelles la prolifération cellulaire représente une cause primaire ou secondaire.

17. Médicament, contenant une quantité efficace d'un composé de formule I selon une ou plusieurs des revendications 1 à 4.
